# EUROPEAN PATENT SPECIFICATION

(11) **EP 3 924 008 B1**
(45) Date of publication and mention of the grant of the patent: **09.04.2025**
(21) Application number: 20755506.1
(22) Date of filing: 17.02.2020
(51) Int. Cl.: A61L 27/52, A61L 27/54, A61L 31/14, A61L 27/50, A61L 27/38, A61F 2/00, B33Y 80/00

(54) **VASCULARIZING DEVICES AND METHODS FOR IMPLANTED DIAGNOSTICS AND THERAPEUTICS**
VASKULARISIERENDE VORRICHTUNGEN UND VERFAHREN FÜR IMPLANTIERTE DIAGNOSTIKA UND THERAPEUTIKA
DISPOSITIFS ET MÉTHODES DE VASCULARISATION POUR AGENTS DIAGNOSTIQUES ET THÉRAPEUTIQUES IMPLANTÉS

(30) Priority: 15.02.2019 US 201962806496 P
(43) Date of publication of application: 22.12.2021
(73) Proprietor: William Marsh Rice University, Houston, TX 77005 (US)
(72) Inventor: VEISEH, Omid, Houston, TX 77005 (US); GRIGORYAN, Bagrat, Houston, TX 77005 (US); SAZER, Daniel, Warren, Houston, TX 77005 (US); PARKHIDEH, Siavash, Houston, TX 77005 (US); MILLER, Jordan, Houston, TX 77005 (US); MUKHERJEE, Sudip, Houston, TX 77005 (US)
(74) Representative: Potter Clarkson
(86) International application number: PCT/US2020/018511
(87) International publication number: WO 2020/168327

(56) References cited:
- US-A1- 2003 153 972
- US-A1- 2007 254 833
- US-A1- 2012 165 935
- US-A1- 2014 249 362
- US-A1- 2015 352 229
- US-A1- 2017 035 941
- US-A1- 2017 072 074
- US-A1- 2018 002 658
- US-A1- 2018 110 901
- KIM JOSEPH J ET AL: "Vascularization of three-dimensional engineered tissues for regenerative medicine applications", ACTA BIOMATERIALIA, ELSEVIER, AMSTERDAM, NL, vol. 41, 2 June 2016 (2016-06-02), pages 17 - 26, XP029666130, ISSN: 1742-7061, DOI: 10.1016/J.ACTBIO.2016.06.001

## Description

### BACKGROUND

### A. FIELD

This disclosure relates to the fields of biology, medicine, medical devices and immunology. More specifically, it relates to vascularizing devices and methods for their use as implanted diagnostics and therapeutics.

### B. RELATED ART

The field of tissue engineering aims to fulfill the great clinical need for development of technologies and processes to facilitate regeneration of human disease or injury by incorporating a combination of cells, biomaterials, and biochemical and physicochemical factors. Indeed, tremendous strides have been achieved over the past several decades to construct implantable vascularized constructs such as skin, cornea, and bladder. However, obtaining functional, physiologically relevant tissues is still a major challenge in the field due to the necessity of a vasculature system to supply nutrients and remove waste in thick constructs. This challenge can be attributed to diffusion transport limitations which results in necrotic cells due to inadequate access to nutrients. Additionally, lack of the ability to recreate the heterogeneous patterns of cells and matrix to obtain constructs with controlled shape and architectures has hindered progress towards organ replacement.

Several approaches have been investigated to facilitate transport of nutrients into engineered tissues. One of the most common approaches utilizes various material processing steps such as critical point drying, gas foaming and salt leaching, or electrospinning to create macroporous structures that can be perfused *in vitro* for tissue culture. While these processes have yielded templates for the construction of functional tissue with matched mechanical compliance of native tissue and high surface area for delivery of nutrients and oxygen, the precise spatial control of scaffold architecture is not easily achieved with these methodologies given that these constructs contain open, porous void volumes rather than a vessel network. Additionally, most of the processing steps involved during the fabrication of these engineered tissues require reagents or conditions that are cytotoxic, further limiting their use with the presence of living cells.

Additionally, needle-based molding techniques have been utilized to obtain straight, perfusable channels with natural and synthetic materials. However, advanced constructs that closely resemble the heterogeneity and complexity of tissues cannot be obtained with this technique due to use of a straight needle.

Another approach utilizes soft lithography, a suite of techniques adapted from the microprocessor industry, which offers spatial control of scaffold architecture through photopatterning with photolithography to obtain microfluidic networks. However, these techniques require expensive, proprietary equipment to reach micrometer-scale resolution and this mode of fabrication is much too slow to make large models of organ vasculature efficiently. Additionally, lithography is typically done in Cartesian coordinates to yield channels with rectangular cross-sections. However, native vasculature rarely follows straight x-, y-, or z-vectors, and blood vessels have circular cross-sections. Strategies combining photolithographic processes with layer-by-layer lamination methods have been pursued to achieve multilayered engineered tissues. Unfortunately, the harsh fabrication process and the difficulties in aligning successive layers with high precision and presence of edge-to-edge artifacts limit use of this approach to obtain complex, three-dimensional (3D) interconnected microfluidic networks.

To circumvent challenges associated with manual alignment of layers, multilayered 3D structures have been either manually or automatically fabricated in a layer-by-layer fashion using either 3D projection stereolithography, resulting in the fabrication of an entire layer under a single light exposure (Lin *et al.,* 2013), or deposition techniques, resulting in fabrication of heterogeneous 3D tissue constructs (Kolesky *et al.,* 2014). While these efforts have yielded engineered tissues with spatially patterned cells (Gauvin *et al.,* 2012), they do not resemble complex, physiologically relevant vasculature. Additionally, only hydrogels with a few layers have been fabricated thus far.

Although living vascular systems consist of fluidic networks that span from a few centimeters, such as the aorta, to several microns, such as capillaries, the currently available technologies cannot fully capture the whole range of the transport network that is critical for multicellular life. Additionally, most of the current *in vitro* models contain 3D constructs with a single vascular network containing one inlet and one outlet. However, a key feature of complex biological systems, the presence of interpenetrating vascular networks, is lacking in current literature but central to mammalian physiology for sufficient transport of nutrients. For example, the alveolus must be located in close proximity to the pulmonary capillary network to ensure oxygenation of red blood cells due to the passive transport nature of oxygen.

Accordingly, a fundamental barrier to successful cell therapy is the lack of biocompatible transplantation devices. Implanted biomaterials lead to foreign body responses (FBRs), which are cascades of inflammatory events and wound-healing processes that lead to fibrosis (walling off) and subsequent implant failure (Harding and Reynolds, 2014; Langer, 2009; Williams, 2008). This response has been described for many materials, from naturally occurring polymers to synthetic materials (Ratner, 2002, Ward, 2008, Zhang *et al.,* 2013). Thus, a critical medical need exists to develop biomaterials that overcome this key challenge of eliminating the FBR associated with biomaterial grafting. The inventors have resolved this challenge through identification of immunomodulatory small molecules by combinatorial chemistry and high throughput screening (Vegas *et al.,* 2016b) and validated the long-term effectiveness of these small molecules in the prevention of fibrosis and long-term cell survival in non-human primates.

The most commonly investigated method for islet encapsulation therapy is the formulation of isolated islets into alginate microspheres (Lum *et al.,* 1991; Scharp and Marchetti, 2014; Schneider *et al.,* 2005). Alginate is a versatile biomaterial that forms hydrogels in di-cationic (Ca²⁺, Ba²⁺) aqueous solutions and has been used for several biomedical applications such as drug delivery, tissue regeneration, implantable sensors, and encapsulation (Kearney and Mooney, 2013; Lee and Mooney, 2012). Its popularity for coating and encapsulation technologies lies in its low cost, low toxicity, mild gelation that is harmless to cells, and tenability. Unfortunately, however, immune recognition of even empty alginate microspheres elicits an FBR that can be further exacerbated by the presence of encapsulated allo- or xenogeneic donor tissue (de Vos *et al.,* 2006; Robitaille *et al.,* 2005; King *et al.,* 2001). This fibrotic response to alginate has been observed in experimental C57BL/6 mouse and non-human primate (NHP) studies (Jacobs-Tulleneers-Thevissen *et al.,* 2013; Scharp and Marchetti, 2014; King *et al.,* 2001), as well as human clinical trials.

In addition, the requirement for placing allogeneic islet cells near the bloodstream to rapidly respond to blood glucose levels as described above, coupled with the high oxygen requirements of pancreatic islet cells, especially during insulin secretion (Sato *et al.,* 2011), adds additional challenges to recapitulating normal pancreatic function at the scale of the whole organ. Although these vascularization challenges are clearly defined, biomaterials technologies which could be used to develop vascularized devices have remained elusive.

US 2015/352229 A1 discloses a substantially non-degradable three-dimensional scaffold having surfaces to which the cells are attached, and a hydrogel, which is attached to the cells. The scaffold, the cells, and the hydrogel are arranged such that the cells are sandwiched in spaces between the hydrogel and the surfaces of the scaffold, thereby allowing mobility and proliferation of the cells in the spaces between the hydrogel and the surfaces of the scaffold, and preventing the mobility and the proliferation of the cells to locations outside of the spaces between the hydrogel and the surfaces of the scaffold.

### SUMMARY

The invention herein is as defined by the claims.

Briefly, the present disclosure concerns vascularizing devices and methods for implanted diagnostics and therapeutics. The inventors have applied the latest in 3D bioprinting technology to direct vascularization of immunoisolated therapeutic cell constructs for long-term viability and functionality, and to permit engraftment of higher densities of therapeutic cells.

In light of the requirements for an effective vascular network, the design criteria for fabricated tissues which mimic blood vessel geometry include, but are not limited to, a plurality of branched vessels ranging in size from tens to hundreds of micrometers in diameter and substantially smooth inner walls that may minimize frictional drag and turbulence during fluid flow.

More specifically, there is provided an implantable device comprising a degradable outer housing comprising a degradable polymer, and an inner non-degradable portion comprising a degradable polymer. The degradable outer housing comprises a patterned architecture that promotes vascularization of said implantable device, *i.e.,* a vascularization bed, and optionally the non-degradable portion may comprise a natural or synthetic hydrogel. The synthetic hydrogel may be poly(ethylene glycol) diacrylate (PEDGA). The device may further comprise gelatin methacrylate (GelMA), natural and chemically modified hyaluronic acid, and natural and chemically modified alginate.

The hydrogel may comprise more than 80% water. The hydrogel may comprise about 10% GelMA and about 3% PEDGA. The hydrogel may comprise monomers of acrylate-(PEG-protein)ₙ-PEG-acrylate, optionally wherein PEG is 2-35 kDa, protein is greater than 100 Da, and total MW is at least 500 kDa or n = 2-500 repeat units. The hydrogel may comprise acrylamide, vinyl-sulfone, norbornene replace the acrylate groups, alginate, silk, dextran, chondroitin sulfate, hyaluronic acid, cellulose, heparin, poly(caprolactone) or multi-component versions thereof.

The inner non-degradable portion may be loaded with drugs, *i.e.,* small molecule therapeutics or biological therapeutics (peptides, antibodies, *etc*.), crystals, cells, such as cells from xenotissue, cells from a cadaver, stem cells, cells derived from stem cells, cells from a cell line, primary cells, reprogrammed cells, reprogrammed stem cells, cells derived from reprogrammed stem cells, genetically engineered cells, or combinations thereof. The cells may be human cells and/or insulin-producing cells and/or pancreatic islet cells. The inner nondegradable portion may be patterned. The device may comprises multiple layers of hydrogels.

The degradable outer housing or inner non-degradable portion may be loaded with drugs, *i.e.,* small molecule therapeutics or biological therapeutics (peptides, antibodies, *etc*.), crystals, or oxygen-releasing compounds, such as CaO₂. The degradable outer housing or inner non-degradable portion is loaded with non-crystallized, crystallized, and/or encapsulated proangiogenic factors, such as VEGF, or pro-angiogenic cells such as endothelial cells, mesenchymal stem cells, endothelial progenitors, proangiogenic macrophages. The inner nondegradable portion may be loaded with a biosensor, such as a biosensor that can measure blood oxygen, blood glucose, tumor antigens, cholesterol, creatinine, hemoglobin A1C, insulin, glucagon, hormone levels, levels of small molecule drugs, DNA, RNA, cytokines, circulating tumor cells, among other possible diagnostic indicators. The non-degradable outer housing and/or the inner non-degradable portion may be patterned, such as where the inner nondegradable portion is patterned to permit vascularization once implanted, wherein the patterned architecture may comprise blood vessel geometries, such as a plurality of branched vessels ranging in size from tens to hundreds of micrometers in diameter and having substantially smooth inner walls to minimize frictional drag and turbulence during fluid flow.

Also described but not claimed are a method of monitoring a state or condition in a subject comprising implanting into said subject a device as described herein, wherein said device comprises a sensor or biosensor, a method of treating a medical condition or disease in a subject comprising implanting into said subject a device as described herein, wherein device comprises a therapeutic agent, a method of creating a vascularized device in a subject comprising implanting into said subject a device as described herein, a method of creating a vascularized tissue around an implant or prosthetic in a subject comprising implanting into said subject a device as described herein, such as wherein said implant or prosthetic is a neural interface device, and a method of creating a volumized tissue around an implant or prosthetic in a subject comprising implanting into said subject a device as described herein, such as wherein said implant or prosthetic is a breast implant or tissue filler.

Any embodiment of any of the present devices, methods, compositions, kits, and systems may consist of or consist essentially of, rather than comprise/include/contain/have, the described steps and/or features. Thus, in any of the claims, the term "consisting of" or "consisting essentially of" may be substituted for any of the open-ended linking verbs recited above, in order to change the scope of a given claim from what it would otherwise be using the open-ended linking verb.

The use of the term "or" in the claims is used to mean "and/or" unless explicitly indicated to refer to alternatives only or the alternatives are mutually exclusive, although the disclosure supports a definition that refers to only alternatives and "and/or." Throughout this application, the term "about" or "approximately" is used to indicate that a value includes the standard deviation of error for the device or method being employed to determine the value. Following long-standing patent law, the words "a" and "an," when used in conjunction with the word "comprising" in the claims or specification, denotes one or more, unless specifically noted.

Other objects, features and advantages of the present invention will become apparent from the following detailed description.

### BRIEF DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein. The patent or application file may contain at least one drawing executed in color. Copies of this patent or patent application publication with color drawing(s) will be provided by the Office upon request and payment of the necessary fee.

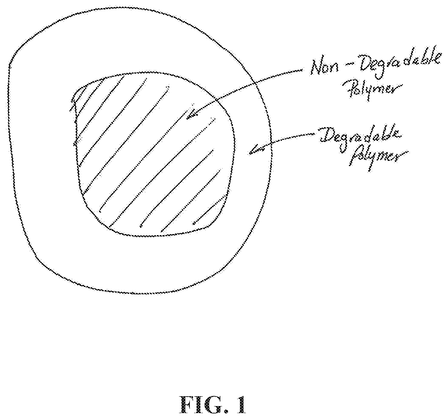

**FIG. 1** illustrates a schematic of an embodiment comprising a non-degradable portion encased by a degradable polymer.

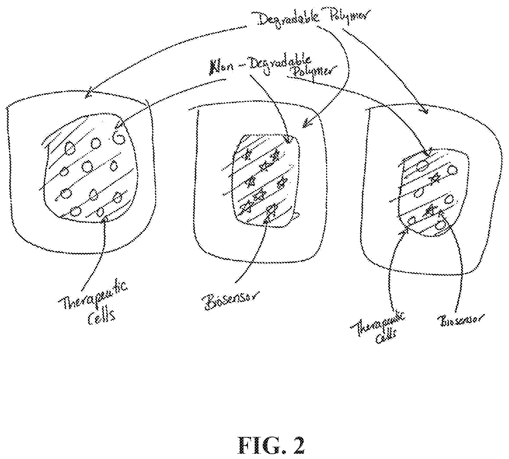

**FIG. 2** illustrates a schematic of an embodiment comprising therapeutic cells, biosensors, or other implants loaded into a non-degradable portion of the device.

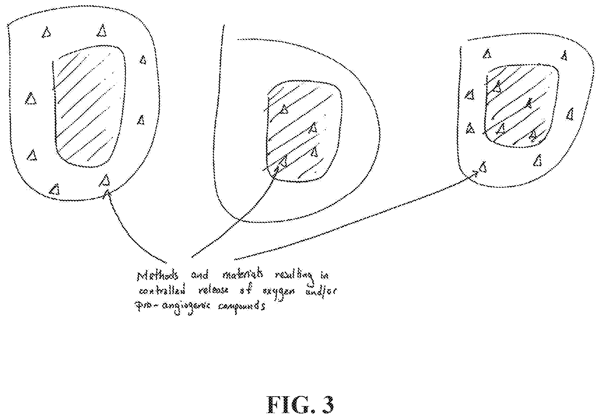

**FIG. 3** illustrates a schematic of embodiments comprising methods and materials resulting in controlled release of oxygen, pro-angiogenic substance or other relevant compounds can be loaded into the degradable and/or non-degradable portions of the device.

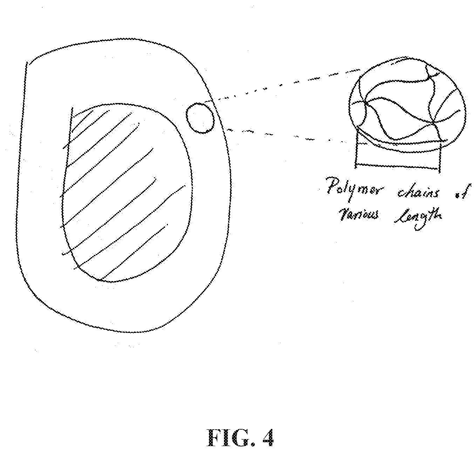

**FIG. 4** illustrates a schematic of an embodiment comprising a degradable portion that can be modified in concentration, stiffness, and anti-fibrotic property, using polymer chains of various lengths.

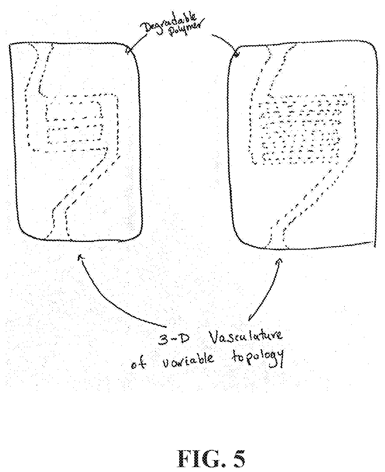

**FIG. 5** illustrates a schematic of embodiments comprising vasculature with many possible topologies.

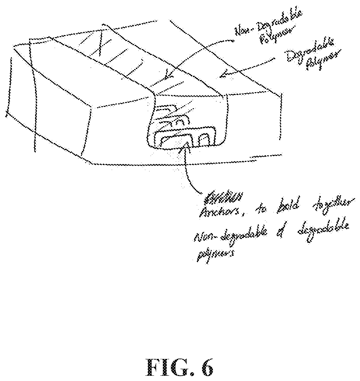

**FIG. 6** illustrates a schematic of an embodiment comprising anchors that can hold together non-degradable and degradable portions of the device.

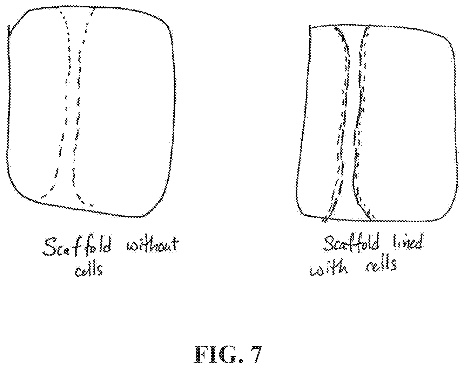

**FIG. 7** illustrates a schematic of embodiments with and without endothelial cells lining the vascular scaffold.

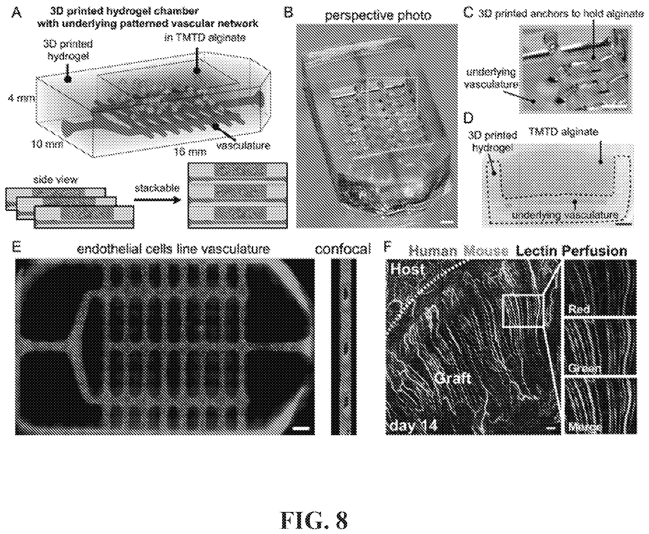

**FIG. 8** illustrates hydrogel for rapid vascularization of therapeutic cell constructs.

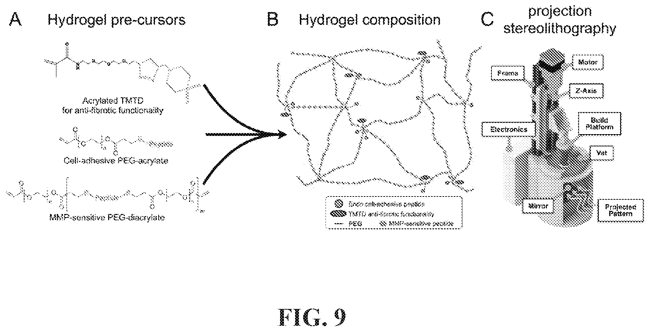

**FIG. 9** illustrates development of an optimized shell chemistry for macrodevice development.

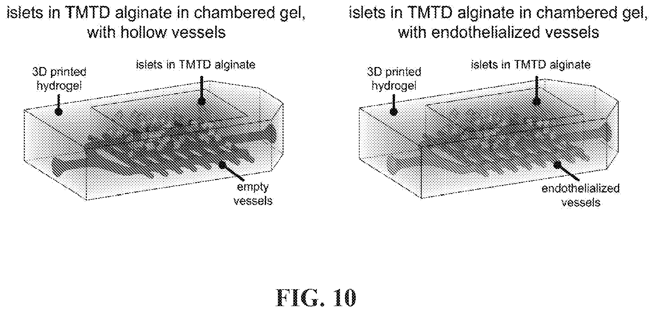

**FIG. 10** illustrates two embodiments of devices tested *in vivo*: a device with hollow vessels (left), and a device with endothelialized vessels (right).

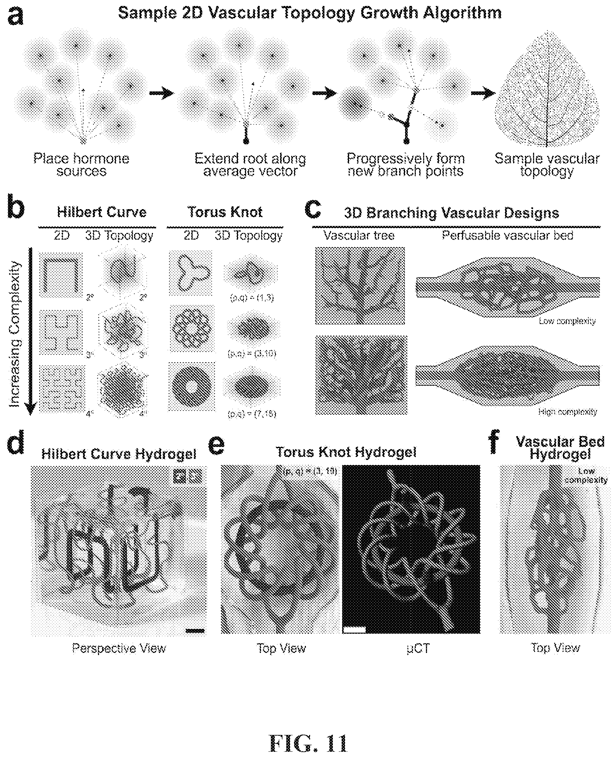

**FIG. 11** illustrates vascular designs to explore vascular efficiencies in perfusable hydrogels.

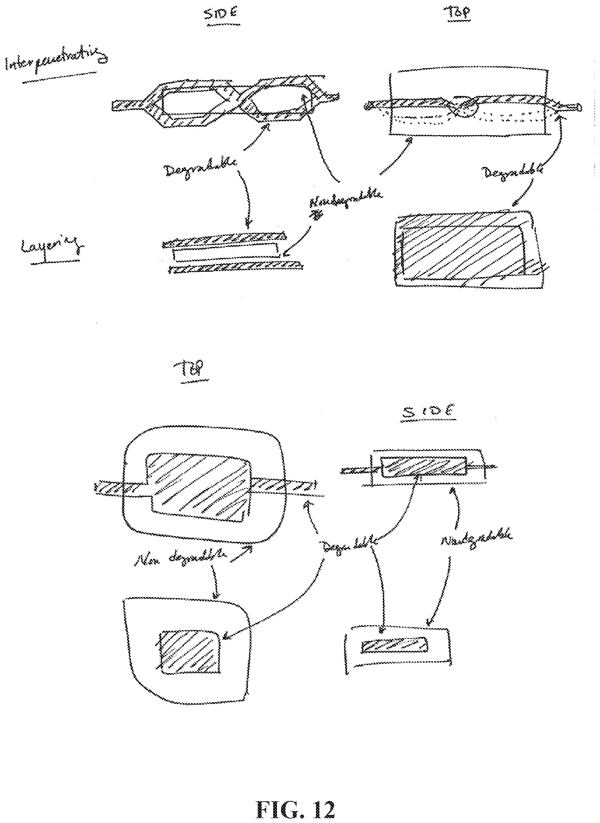

**FIG. 12** illustrates devices with various geometries for both the inner and outer housing.

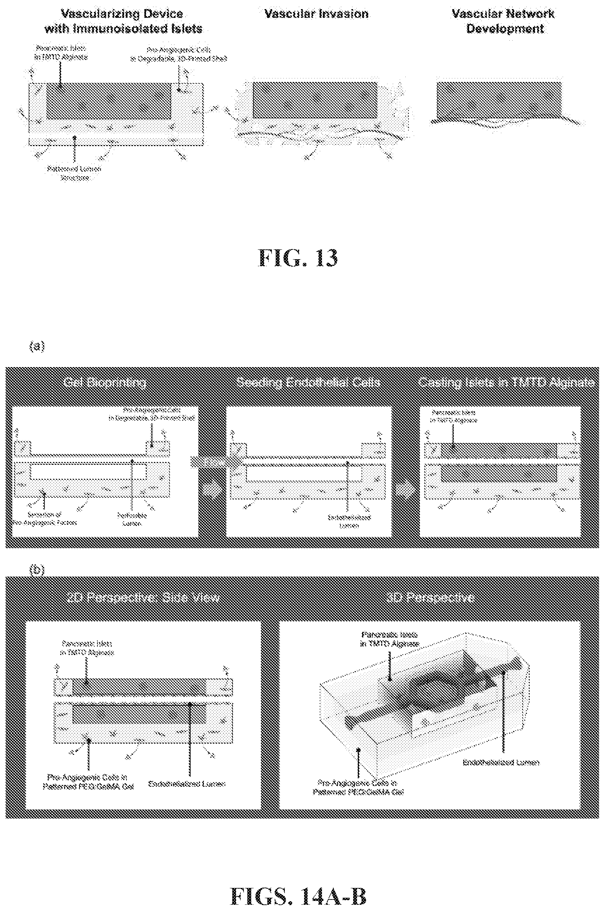

**FIG. 13****. Vascularizing Device for Transplantation of Immuno-isolated Islets.** Proangiogenic cells such as endothelial cells and human mesenchymal stem cells are encapsulated in a 3D-printed, degradable shell, and pancreatic islets are cast in a non-degradable core of TMTD alginate. Encapsulated pro-angiogenic cells recruit vascular invasion, and 3D-printed vascular channels help guide vascular invasion. Over time, the vascular network remains, supplying nutrients to immunoisolated islets.
**FIGS. 14A-B.** **Gel Preparation and Design.** Gels are prepared as follows: light sensitive pre-hydrogel solution is combined with desired cell types to encapsulate cells in a hydrogel patterned with complex architectures and designs. Endothelial cells can be seeded into the lumens of the bioprinted gels, allowing for perfusion culture to boost vasculogenic phenotype of endothelial cells. Finally, therapeutic cells, in this case, pancreatic islets, can be cast into a chamber of the 3D-printed device (FIG. 14A). This results in a core-shell vascularizing device, with therapeutic cells located in one chamber and provascularizing cells located in the bulk gel. In this example, pancreatic islets are encapsulated in TMTD alginate (non-degradable), with HUVECs and hMSCs encapsulated in the bulk PEG/GelMA gel, with endothelial cells lining the lumen (FIG. 14B).

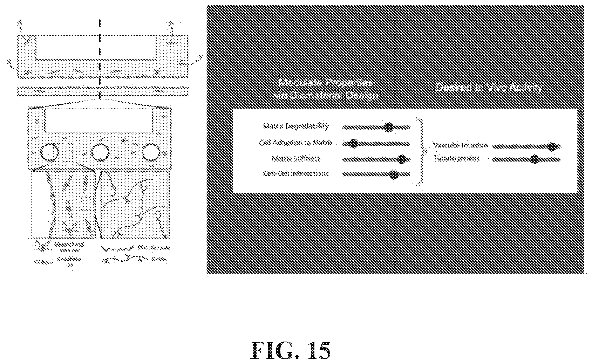

**FIG. 15****. Biomaterial composition of vascularizing gels.** Gels are prepared by stereolithography of monomers mixed with bioactive cells, in order to create bulk encapsulation of bioactive cells in a complex gel architecture. The lumens of the gel can also be seeded with endothelial cells, which can be maintained in perfusion culture to boost vasculogenic potential of these cells. In this example, PEG diacrylate and GelMA are utilized to create the gel structure, and MSCs and endothelial cells are encapsulated in the bulk, to secrete vascularizing factors and aid in gel degradation. Endothelial cells are also seeded into the lumens to aid vessel formation. Modulation of biomaterial properties and cell compositions allows us to finely tune biomaterial properties to target desired *in vivo* responses.

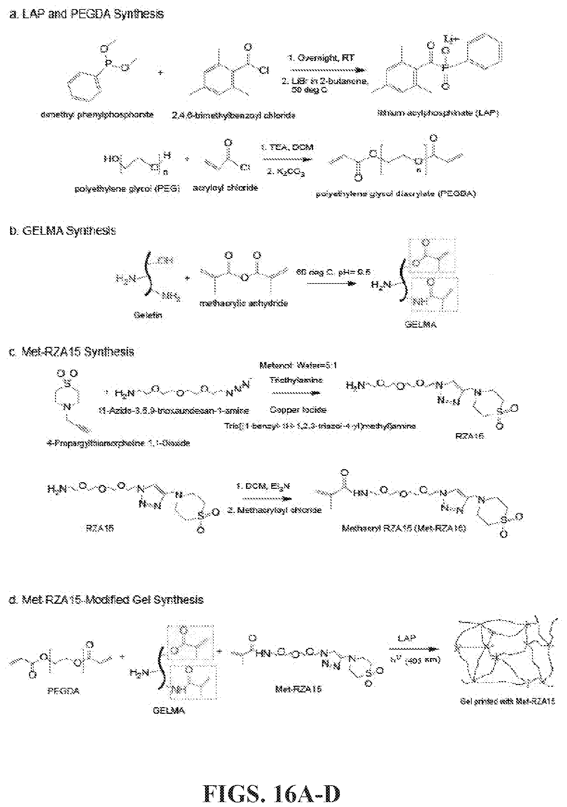

**FIGS. 16A-D.** **Reaction schemes for the synthesis of photoinitiator (LAP), photoreactive polymer (PEGDA and GelMA), and small molecule modified 3D printed gel.** (FIG. 16A) Synthesis of LAP and PEGDA. (FIG. 16B) Synthesis of GelMA. (FIG. 16C) Synthesis of Met-RZA15, an immunomodulatory small molecule. (FIG. 16D) Photocrosslinking of PEGDA and GelMA with Met-RZA15 to form a hydrogel network with pendant Met-RZA15.

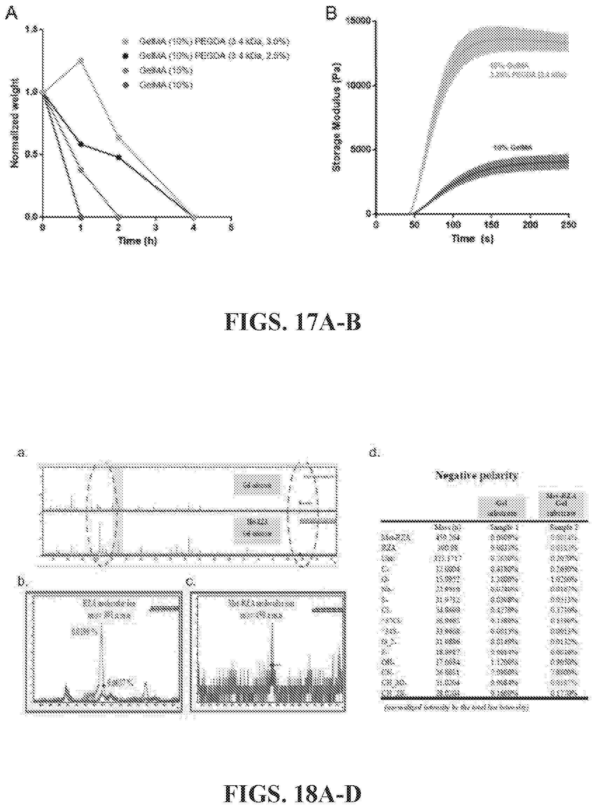

**FIGS. 17A-B.** **Hydrogel structures exhibit an inverse relationship between collagenase degradation rate and shear stiffness.** (FIG. 17A) Degradation kinetics of various hydrogel formulations in 0.2 mg/mL collagenase. The inventors observe a general trend that degradation rates decrease as polymer concentrations increase. Furthermore, the addition of PEGDA appears to have a particularly strong attenuation effect, due to its lack of enzymatically cleavable peptide sequences. (FIG. 17B) The addition of PEGDA dramatically increases shear stiffness when compared to pure GelMA gels.
**FIGS. 18A-D.** **TOF-SIMS surface analysis of the printed gel verifies conjugation with the Met-RZA small molecule.** (FIG. 18A) TOF-SIMS measurement of blank gel substrate (top trace) compared with gel conjugated with Met-RZA (bottom trace). (FIG. 18B) Overlay of the blank gel trace and Met-RZA gel trace from part (a) in the mass range of RZA15. (FIG. 18C) Overlay of the blank gel trace and Met-RZA gel trace from part (a) in the mass range of Met-RZA. (FIG. 18D) Quantification of the TOF-SIMS data for both the unmodified gel and the small molecule-modified gel.

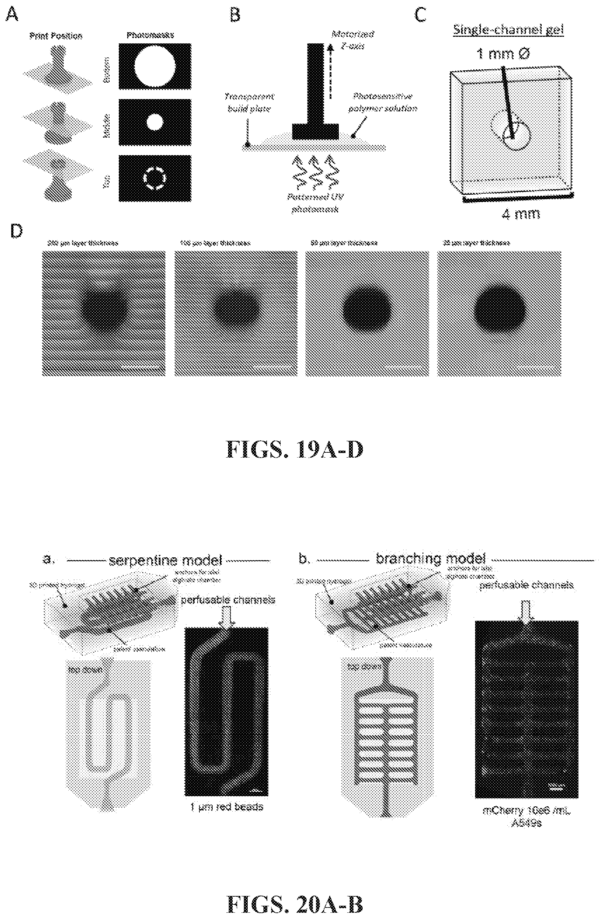

**FIGS. 19A-D.** **Hollow vascular channels can be printed within biocompatible 3D printed hydrogels.** (FIG. 19A) 3D models are first sliced into a series of 2D patterns. (FIG. 19A) 2D patterns are sequentially projected as UV photomasks onto a photosensitive polymer solution, yielding solid hydrogel structures with patterned architecture. (FIG. 19C) Schematized geometry of a single-channel gel, the smallest unit of any vascular network. (FIG. 19D) Hollow vascular channels of increasing circularity are achieved by decreasing print layer height (bar = 1 mm). Gels are comprised of 20 wt% 6 kDa PEGDA with 1 mg/mL FITC-dextran (150 kDa) to enable fluorescent visualization. 3D printing of single-channel hydrogels remains a non-trivial challenge in the biofabrication field. Due to the overhanging nature of circular features, material can inadvertently clog hollow channels in both extrusion and lightbased printing methods.
**FIGS. 20A-B.** **Fully perfusable architecture models are faithfully fabricated with projection stereolithography:** The inventors designed (FIG. 20A) serpentine and (FIG. 20B) branched vascular architectures with an open-source 3D computer graphics software, Blender (Blender Foundation, Amsterdam, Netherlands). The inventors generated these designed vascular architectures in a PEGDA:GelMA (3.25 wt% 3.4 kDa PEGDA/10 wt% GelMA) hydrogel featuring totally perfusable hollow lumens. They verify lumen patency by perfusing (FIG. 20A) 1 µm red fluorescent beads (1:800 in PBS + 10% glycerol) and (FIG. 20B) adenocarcinoma human alveolar basal epithelial cells (A549) expressing H2B-mVenus (not shown) and mCherry which flow through the entirety of the inventors' designed vascular architecture.

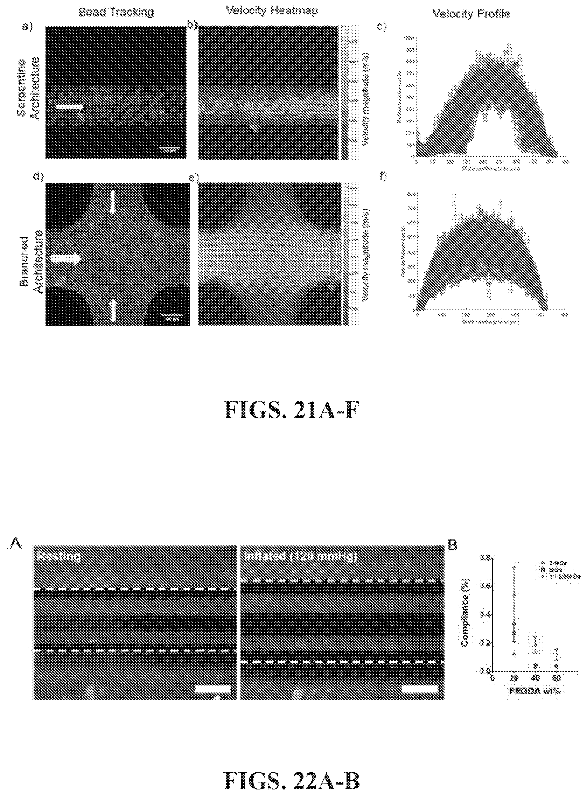

**FIGS. 21A-F.** **Printed hydrogels demonstrate desirable flow patterns.** (FIG. 21A) 1 µm diameter fluorescent beads in a 1:800 ratio of beads to PBS + 10% glycerol were perfused through a serpentine architecture hydrogel. Video data was captured using a Nikon Eclipse Ti epifluorescence microscope (24.6 ms frame rate). The inlet flow rate was set to 2.0 µL/min and the direction of flow is indicated by the white arrow. Particle image velocimetry (PIV) data for the motion of fluorescent beads was analyzed using PIVlab, an open-source application for MATLAB (Theilicke and Stamhuis, 2014). (FIG. 21B) following analysis in PIVlab, a heatmap of velocity vectors was generated. The lighter regions in FIGS. 21A and 21D represent areas of the hydrogel containing no moving particles, which were manually masked prior to image processing, and arrows represent velocity vectors. Velocity vectors were examined over a line perpendicular to the flow direction (arrows in FIGS. 21B and 21E) to determine the flow characteristics within this architecture. (FIG. 21C) Velocity profile data for each point along the line. Individual data points (circles) were compiled for all video frames and plotted on the same graph. For each distance along the profile line, velocities were averaged among all of the frames. The average velocities associated with each distance were used to generate a parabolic best-fit curve, shown in orange (r2 = 0.855). (FIGS. 21D-F) Experiments were repeated for a branched architecture hydrogel. For this design, the fluorescent bead concentration was 1:200 of beads:PB S and the inlet flow rate was set to 5 µL/min. For the best-fit curve of this velocity profile, r2 = 0.966.
**FIGS. 22A-B.** **3D printed vessels are highly compliant under dynamic fluid cycles between systolic and diastolic pressures.** (FIG. 22A) Vessel distension was measured upon application of pneumatic air pressure (hydrogel composition: 20 wt% 6 kDa PEGDA; channel diameter: 1.5 mm; channel length: 20 mm). White dashed lines represent walls of the imaged channel (bar = 1 mm). (FIG. 22B) Compliance was found to be highly tunable within the polymer parameter space, directly correlating with molecular weight but inversely correlating with polymer concentration (n ≥ 4). Printed gels were connected to an open-source pneumatic pump (MillerLab Pneumatic System, 2016), providing dynamic fluid cycling between diastolic (80 mmHg) and systolic (120 mmHg) pressures. Diameter changes were captured on an optical stereo microscope and sCMOS camera. Images were captured at > 50 frames per second, allowing high spatial resolution to determine maximum changes in diameter. Compliance was measured as previously reported for tissue-engineered blood vessels (L'Heureux *et al.,* 2006).

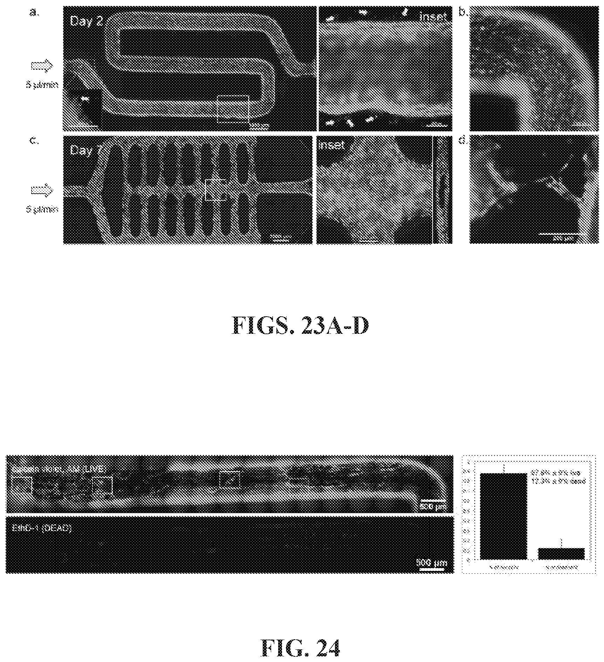

**FIGS. 23A-D.** **Fully endothelialized vascular networks coat the channel walls and maintain lumen patency.** The inventors' optimized hydrogel formulation (3.25 wt% 3.4 kDa PEGDA, 10 wt% GelMA) supports human endothelial cell attachment. The inventors inject 10-30 × 10⁶ cells/mL of constituently expressing GFP human umbilical vein endothelial cells (GFP-HUVECs, Angioproteomie, Boston, MA) into the vessel inlet and allow for cells to adhere to the lumenal wall. Following initial cell injection, the inventors rotate the gel about the vascular axis by 90 degree increments every 15 minutes for a total of 2 hours. The gels are then cultured with Vasculife media (Lifeline Technologies, LL-0003, Frederick, MD) by fluidically connecting tubing through a peristaltic pump into the inlet of the vascular network to precisely control a flow rate of 5 µl/min. Images were acquired on a Nikon Eclipse Ti epifluorescence (FIGS. 23A, 23B and 23D) microscope or on a Zeiss LSM 780 confocal microscope (FIG. 23C).
**FIG. 24****. Over 85% of HUVECs remain viable in vascular networks after 7 days of perfusion:** An endothelialized serpentine channel, perfused at 5 µl/min for 7 days was stained for cell viability. The inventors prepared the calcein violet, AM solution (ThermoFisher Scientific) at 1 µM and 1 µl/mL of the DEAD (Ethidium Homodimer-1) component of the LIVE/DEAD imaging kit (Invitrogen, R37601) in PBS. The staining solution was injected into the channel and incubated for 30 minutes at 37 °C before imaging (Nikon Eclipse Ti epifluorescence microscope). Four regions of interest were randomly selected to manually count live and dead cells for a cell viability output.

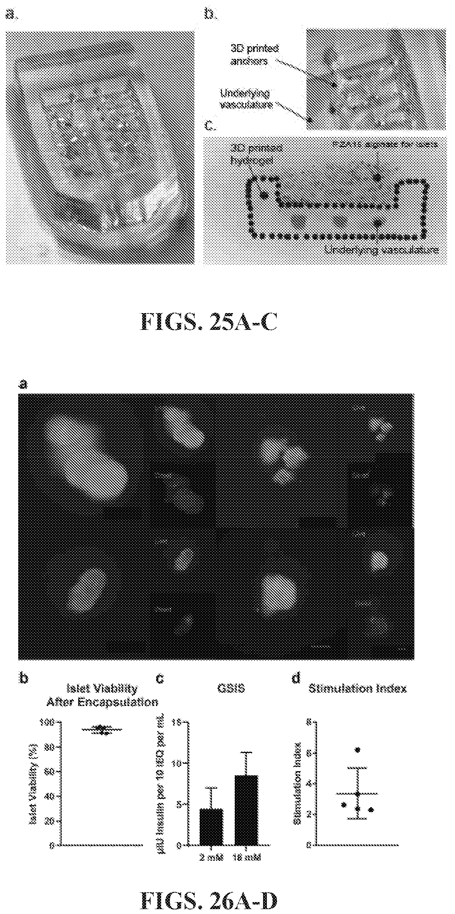

**FIGS. 25A-C.** **TMTD alginate blocks can be cast into the 3D printed gel.** (FIG. 25A) Perspective photo of the printed gel. (FIG. 25B) Zoom-in to highlight the underlying vasculature and 3D printed anchors to hold the alginate block. (FIG. 25C) Cross section of printed gel.
**FIGS. 26A-D.** **Islets demonstrate >90% viability and stimulation index >2 after casting into vascular scaffolds.** (FIG. 26A) Islets in TMTD alginate were cast into 3D-printed vascular scaffolds. After casting, gels were incubated with complete CMRL-1066 containing 1:200 Calcein AM and 1:200 Ethidium Homodimer-1 for 30 min, then fixed and imaged. Scale bar = 100 µm. (FIG. 26B) Islet cell viability was quantified for 5 islets after encapsulation. (FIG. 26C) Gels were subjected to GSIS assay, demonstrating a mean stimulation index of 3.35, with n = 5 independent samples. (FIG. 26D) Stimulation index from GSIS assay > 2. Mean +/- S.D. depicted for all plots.

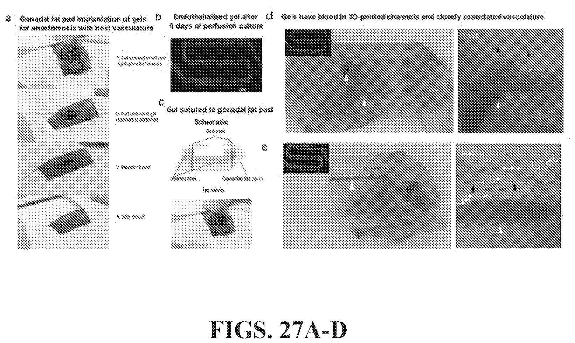

**FIGS. 27A-E. *In vivo* gonadal fat pad implantation demonstrates blood in 3D printed channels and closely associated host vasculature.** (FIG. 27A) Surgical method for fat pad implantation of 3D printed devices. (FIG. 27B) Endothelialized gel after 6 days in perfusion culture, demonstrating lining with adherent GFP-HUVECs. Scale Bar = 1 mm. (FIG. 27C) Gel sutured to the fat pad. (FIGS. 27D-E) Explanted gels demonstrate blood in channels (white arrowheads) and closely associated host vasculature (black arrowheads). Endothelialized gels 4 (FIG. 27D) and 5 (FIG. 27E) with serpentine channel before implantation (left inset). Left inset, scale bar = 1 mm.

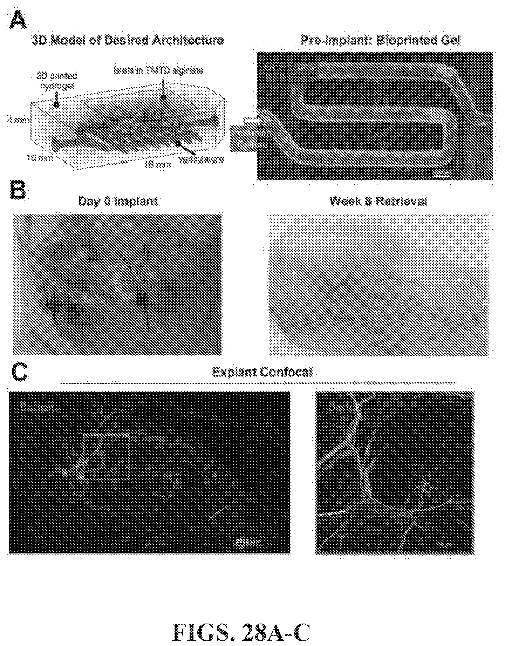

**FIGS. 28A-C.** **Patterned Biomaterials Lead to Vascular Invasion.** 3D-model of vascularizing devices for immune-isolated islets (FIG. 28A, left). Bioprinted gel containing perfusion-primed GFP-endothelial cells in the s-shaped vascular channel and red endothelial cells in the bulk of the gel (FIG. 28A, right). The inventors note the cells have achieved a monolayer in the serpentine structure, a sign of endothelial maturity. Gels are sutured to the epididymal fat pads of male SCID-beige mice (Charles River Labs) (FIG. 28B, left) and retrieved from that same site (FIG. 28B, right). Intravenous injected fluorescent dextran illuminates all vasculature that has invaded the gel and can be visualized with confocal microscopy (FIG. 28C).

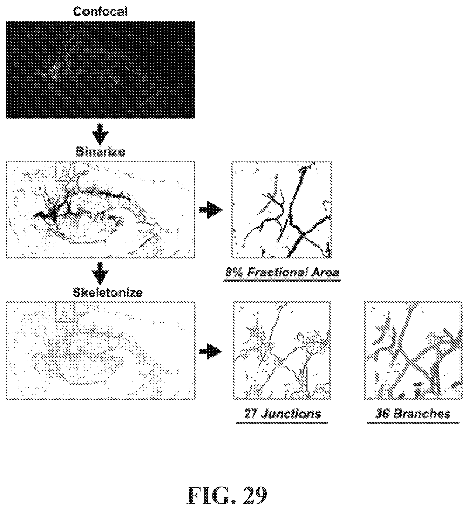

**FIG. 29****. Image Analysis Pipeline to Identify Key Vascular Parameters.** The inventors can leverage image analysis methods to identify key vascular parameters in their hydrogels. Confocal imaging of intravenous fluorescent dextran illuminates vascular networks in the hydrogels (Confocal). This image with 16-bit color depth is first binarized with ImageJ (Binarize). The binary image indicates location of vasculature (black pixels) compared to the background (white pixels). This binary image is quantified to identify the fraction of blood vessels (black pixels) to total pixels in the image. This results in quantification of the fractional area of the gel occupied by blood. Then, binary image is skeletonized with ImageJ. Using the ImageJ Skeletonize plugin, the number of junctions, number of branches, and total branch length (not shown here) can be quantified. Junctions and Branches in sample ROIs are shown below.

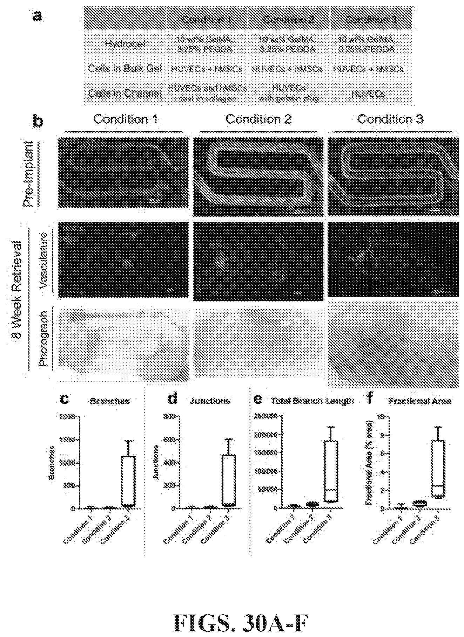

**FIGS. 30A-F.** **Biomaterial Design to Achieve *In vivo* Vascularization.** 3D-printed vascularizing devices can be made with various hydrogel chemistries, cells in bulk gel, and cells in channel. 3 such formulations were chosen and evaluated *in vivo,* via implantation and suturing to the epididymal fat pads of SCID-beige mice (FIG. 30A). Hydrogels were prepared *in vitro* and imaged via epifluorescence microscopy, demonstrating presence of RFP-HUVECs in the bulk of all gels, and contracted collagen cords (GFP HUVECs in Condition 1, Pre-Implant) and confluent endothelial monolayer (GFP-HUVECs in Conditions 2 and 3, Pre-Implant) (see FIG. 30B). Prior to retrieval, CF633-conjugated dextran (Biotium) was injected via tail vein and allowed to circulate for 15 min. Mice were euthanized and gels were fixed. Key parameters of vascular invasion were quantified (FIGS. 30C-F; n = 4 - 6 replicates per group).

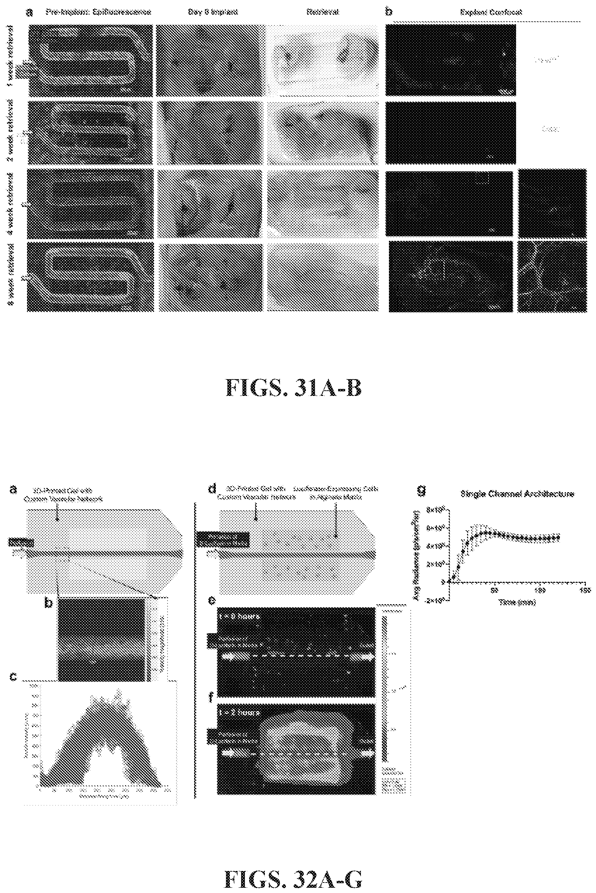

**FIGS. 31A-B.** **Kinetic Study of Vascular Development.** A key factor in vascularization for islet transplantation is the kinetics of vascular development. The inventors probed the kinetics by explanting gels at various time points. Gels were prepared *in vitro* and assayed for proper endothelial morphology prior to implantation (FIG. 31A, Pre-Implant Epifluorescence). Gels were implanted and retrieved at various time points. The inventors note vascular invasion occurring between 4 and 8 weeks (FIG. 31B). Images are representative of n = 4-6 replicates per group.
**FIGS. 32A-G.** **Evaluation of Fluid Flow in Patterned Vascular Network and Initial Testing of Single Channel Vascular Network *in vitro.*** Gels with various vascular architectures can be prepared *in vitro* (FIG. 32A). Velocity magnitude can be quantified via fluorescent bead tracking (Nikon Ti Epifluorescence Microscope; see FIG. 32B). Velocity profile data for each point along the line. Individual data points (blue circles) were compiled for all video frames and plotted on the same graph. For each distance along the profile line, velocities were averaged among all of the frames. The average velocities associated with each distance were used to generate a parabolic best-fit curve, shown in orange (r2 = 0.855; FIG. 32C). In order to study effect of vascular architecture on cell functionality and nutrient delivery, the inventors performed an *in vitro* assay utilizing encapsulated luciferase-expressing cells and perfusion of D-luciferin in complete medium. In the presence of ATP, oxygen, and luciferin, the luciferase enzyme will illuminate, allowing quantification of nutrient delivery and cell functionality in complex hydrogels. Gels with various vascular structures were cast with luciferase-expressing cells in an alginate matrix in order to study nutrient delivery and cell activity (FIG. 32D). These gels were perfused with D-Luciferin in complete medium (0.15 mg/mL final concentration; PerkinElmer). Data is shown for a representative gel at 0 hours (FIG. 32E) and at 2 hours of perfusion (FIG. 32F) via intravital imaging (PerkinElmer IVIS Kinetic III). Kinetics of cell luminescence were quantified via Living Image software and plotted in Prism 8 (FIG. 32G; n = 4 +/- s.d.).

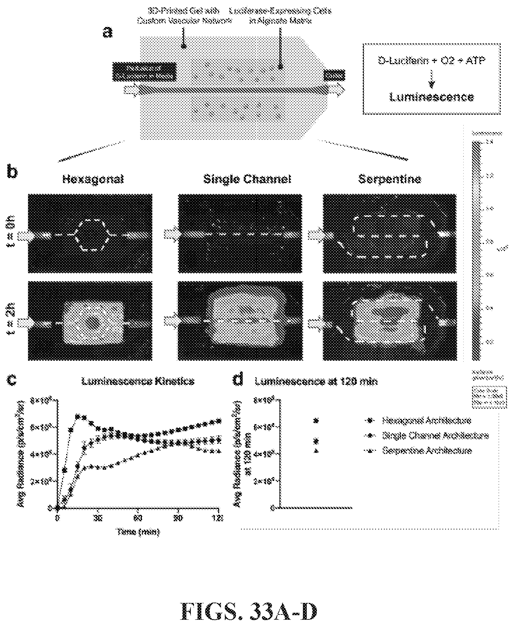

**FIGS. 33A-D.** **Screening Vascular Architectures for Nutrient Delivery.** Gels with various vascular architectures were printed and the space around the vascular networks were cast with alginate containing luciferase-expressing HEK293 cells (25 million cells per mL of alginate) (FIG. 33A). These hydrogels are then perfused with D-Luciferin containing medium through their vascular lumens, delivering luciferin and oxygen through the bulk gel. Via intravital imaging (IVIS Kinetic III), the inventors can quantify luminescence, which results from nutrient delivery and cell functionality in engineered hydrogels. Three different vascular architectures were prepared and analyzed for nutrient delivery (FIG. 33B). Luminescence was quantified every 5 minutes for 120 minutes (FIG. 33C; hexagonal and serpentine architecture, n = 1; single channel architecture, n = 9; mean +/- s.e.m). In their studies this far, the inventors note hexagonal architecture had more effective nutrient delivery compared to the single channel or serpentine architecture through the kinetic analysis (FIG. 33C) and at the endpoint of the study (FIG. 33D).

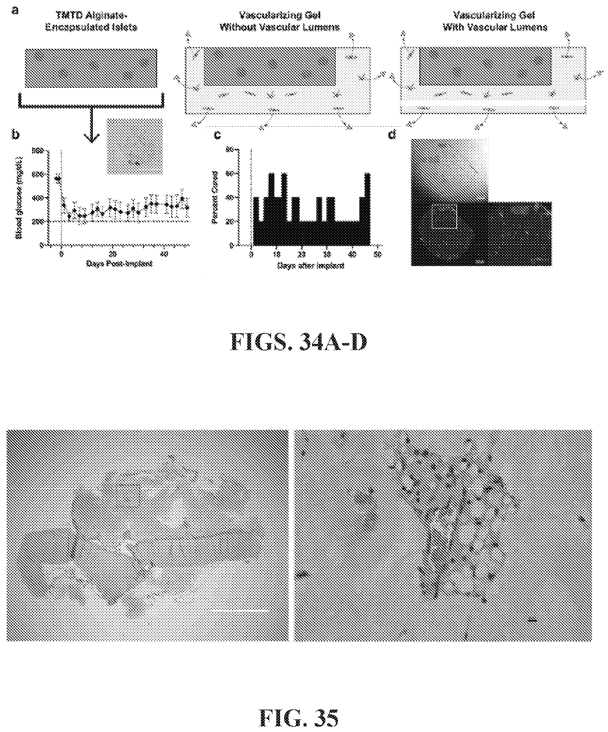

**FIGS. 34A-D.** **Evaluation of High-Density Encapsulated Islets and Future Diabetic Studies.** Diabetic studies will compare TMTD-alginate encapsulated islets with an experimental group; a hydrogel with optimized biomaterial formulation and with optimized vascular architecture for nutrient delivery (FIG. 34A). The inventors also plan to perform a cell-type control, to control for the effect of islet co-transplantation with pro-angiogenic cells (FIG. 34A). Alginate blocks were cast with 750 IEQ of fresh rat islets and transplanted in 5 STZ-induced C57Bl6 mice (Jackson Labs; FIG. 34B). Mice below 200 mg/dL were considered cured. Percent of mice cured varied between 0% and 60% over the 7-week study, demonstrating unstable glycemic control (FIG. 34C). Upon explant, alginate blocks were stained for islet viability (Live/Dead Assay, Thermo Fisher). Islets were found to be dead (FIG. 34D). This demonstrates that without vasculature, high-density encapsulated islets do not adequately restore normoglycemia. This study serves as a baseline for comparison with vascularizing gels.
**FIG. 35****. Histology Demonstrates Formation of Blood Vessels in Gels.** To verify that vascular invasion has occurred in the bioengineered gels, samples were submitted for histology. 3D-printed PEG/GelMA gels, patterned with collagen cords in the lumens and with hMSCs and HUVECs in the bulk of the gel, were implanted in immunocompromised mice for an 8-week period. Gels were then retrieved and submitted for histological analysis. Histology was performed via formalin fixation, paraffin embedding (FFPE). Gels were sectioned at 6-micron thickness, and H&E staining was performed. The overall gel was imaged (left; EVOS XL Color CCD 2X magnification, scale = 2000 µm), as well as an inset (right; Nikon A1 RSI 40X water immersion Di-F3 color camera, scale = 10 µm). Red blood cells and capillary-like structures are visible in multiple regions of the inset (right).

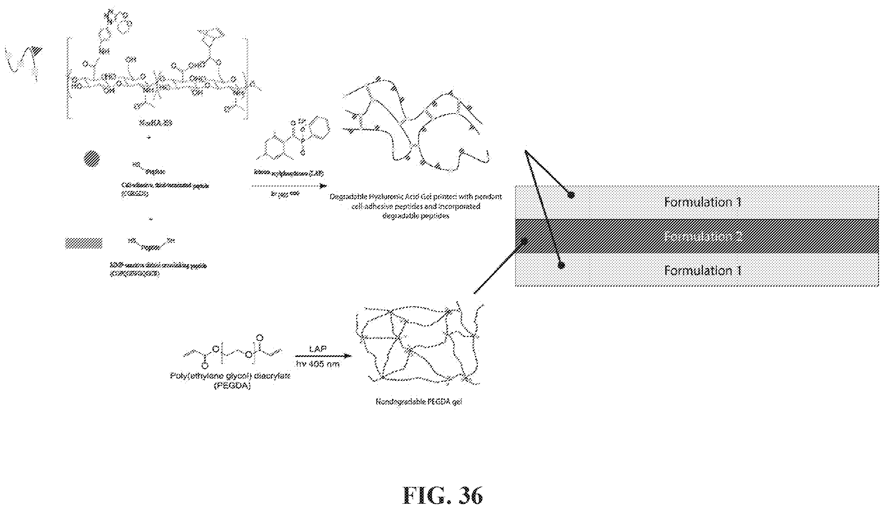

**FIG. 36****. Printing of Multi-Layered Hydrogel Constructs with Various Biomaterial Properties.** In this example, a stereolithography 3D-printing system can be utilized as follows: Printing may be performed with one pre-hydrogel solution, here denoted as Formulation 1, composed of norbornene-modified hyaluronic acid (NorHA), thiolterminated cell adhesive peptide CGRGDS (SEQ ID NO: 2), and di-thiol terminated degradable peptide CGPQGIWGQGCR (SEQ ID NO: 1). This results in a component of the device being printed as a degradable biomaterial formulation. The print is then paused, the printing solution is exchanged for one with different properties, for example, PEG-diacrylate, which is nondegradable. This is photopolymerized in the presence of LAP to create a second component of the device, denoted as Formulation 2. Finally, this print solution can be exchanged for another one, such as the degradable Formulation 1, to print the final component of the device. This ultimately results in a 3D printed device with various biomaterial properties that are spatially separated.

### DETAILED DESCRIPTION OF THE INVENTION

Transport and diffusion limitations remain a major impediment in the development of highly cellularized constructs. Although the challenges are clearly defined, technologies to develop vascularized devices have remained elusive. A critical medical need exists to develop biomaterials that overcome the challenge of eliminating the FBR associated with biomaterial grafting. The inventors have resolved this challenge through identification of immunomodulatory small molecules by combinatorial chemistry and high throughput screening (Vegas *et al.* 2016b) and validated the long-term effectiveness of these small molecules in the prevention of fibrosis and long-term cell survival in non-human primates.

In one aspect, the inventors have developed biomaterials chemistries and novel fabrication strategies, such as advanced three-dimensional printing, explicitly to address the vascularization challenge in engineered tissues and implantable devices (Miller, 2014). They have developed a high-throughput pipeline for the synthesis and evaluation of hundreds of materials and prototype devices and have explored new approaches to both cell encapsulation and device manufacture. With regard to alginate-based materials, novel lead compounds that exhibit low levels of inflammation have been identified but require further development (see below). To improve safety and efficacy, the inventors are investigating the use of semipermeable, low fibrosis and vascularized device in combination of alginate sheets as containment units that will facilitate the complete removal of all transplanted cells in the event of teratoma formation. This work lays important groundwork for functional and scalable allogenic replacement tissues which can restore glycemic control in large diabetic animal models and ultimately in humans.

These and other aspects of the disclosure are set out in detail below.

### I. 3D Printable Devices

### A. Device Components

The inventors have previously described a 3D printing process that can fabricate 3D engineered tissues with biologically-inspired design criteria including, but not limited to, conforming to Murray's Law, multiscale branched vessels from tens to hundreds of micrometers in diameter, smooth inner walls, circular cross sections, and multiple inlet/outlets. Indeed, with printing parameter optimization, the limit to what can be fabricated depends on what one can model. In addition, more complex designs that contain heterogenous properties in a single layer or in multiple layers can be fabricated. For instance, grayscale photomasks with predefined gradients can be incorporated to obtain a layer with varied stiffness or for controlled immobilization of biomolecules and cells while still using the same vat and solution. Additionally, utilization of fractal space-filling models to computationally grow vascular networks around and through pre-existing vascular networks or following the architecture of native tissues can be achieved by computer growth models for even more complex and physiologically relevant 3D models. These mathematical fractal, space-filling models can be derived from, for example, knot theory, the Hilbert curve, and the L-system. Such mathematical fractal space-filling models to predict idealized vascular networks include, but are not limited to knot theory, Plumber's Nightmare, Peano curve, Hilbert curve, Pythagoras tree, and Brownian tree models. As an example, the Plumber's Nightmare model essentially comprises two Vascular Ladder models that are connected to each other by straight vertical cylinders. Multiple Plumber's Nightmare models can be intercalated such that they are interpenetrating. The Vascular Ladder models are comprised of 1 inlet and 1 outlet with two horizontal cylinders that are connected by diagonal cylinders, resulting in interchannel junctions.

Photopolymerizable hydrogel materials such as poly(ethylene glycol) diacrylate (PEGDA) can be crosslinked using a photoinitiator system such as lithium acylphosphinate (LAP) (Fairbanks *et al.* 2009) which absorbs in the UV to visible light wavelength range. By adding, for example, low concentrations of carbon black (which can absorb light across all UV-visible light spectrum), or low concentrations of tartrazine (which has a peak light absorption near 500 nm), the inventors can limit the depth of penetration of light. To quantify this process, the inventors developed a photorheology assay to monitor hydrogels polymerization and stiffness evolution as a function of light dosage and sample thickness. Indeed, the addition of additive materials, such as tartrazine, control the extent of gelation of the prepolymerization mixture by impacting the gelation kinetics and final gel rheological properties. Other materials include -ene modified natural and synthetic materials that can be photopolymerized such as alginate, silk, dextran, chondroitin sulfate, hyaluronic acid, cellulose, heparin, and poly(caprolactone) and multi-component versions of these.

To achieve complex patterning of multilayered hydrogels, on the order of several centimeters, with high pattern fidelity, light exposure during the printing process is controlled so that the light projected onto the build platform interacts mainly with the layer that undergoes gelation for either partial or complete gelation. Radical mediated photopolymerization of hydrogels utilizes a photoinitiator - a molecule sensitive to a particular wavelength range that, upon light absorption, the molecule decays and release free radicals which can catalyze hydrogel polymerization. To this end, it is imperative to quantify the wavelength sensitivity of the photoinitiator. High concentrations of photoinitiator will absorb more light and provide higher z-resolution by limiting penetration depth of the incident light. However, high photoinitiator concentrations disrupt the photopolymerization reaction (more free radicals have a higher chance of annihilating each other), and photoinitiators at high concentrations are cytotoxic. In addition, with high x-y resolution from the projector, a complication is that light shines through the z-direction of the previously printed layers, potentially limiting the ability to form complex overhang structures (such as found in vasculature), and also may cause phototoxicity to entrapped cells.

Thus, to achieve high resolution printing, the present disclosure provides a photochemical means to provide, for the first time, high z-resolution in bioprinted tissues while maintaining high cell viability. To address the concerns outlined above, the inventors have identified a general strategy whereby biocompatible materials or chemicals are added to the prepolymerization solution to provide higher z-resolution. The additive material is selected based on three criteria: 1) ability to absorb light wavelengths which fully encompass the photosensitive wavelength range of the photoinitiator, 2) limited participation or limited inhibition of photopolymerization reactions, and 3) biocompatibility at the concentrations desired. This additive material is referred to herein as a biocompatible, light-absorbing additive material suitable to control light penetration. Multiple molecules have been screened that absorb light, limiting the penetration depth of light into already formed layers. Suitable molecules absorb in the same region as the photoinitiator used in the prepolymerization solution. Examples of molecules capable of controlling light penetration and therefore suitable for use as the biocompatible, light-absorbing additive material include carbon black, yellow food coloring, tartrazine, nanoparticles, microparticles, gold nanoparticles, riboflavin, phenol red, Beta-carotene, curcumin, saffron, and turmeric. Proteins may also act as suitable biocompatible, light-absorbing additive materials provided that their peak absorption overlaps with the peak absorption of the photoinitiator and matched to the incident light source. Additionally, the inventors recognize that cells that are transfected or transduced with proteins that absorb in the same region as the photoinitiator, such as cyan fluorescent protein (CFP) or green fluorescent protein (GFP), can be used at high concentrations, with reduced or no additives, to result in reduced lateral overcuring due to the light absorbing molecules present inside cells. Additionally, the inventors' methodology allows us to print hydrogels with both horizontal and vertical channels due to stringent control of the penetration of the projected light.

To address potential cell viability concerns associated with long print times for fabrication of engineered tissues with this method, the cell viability can be maintained or enhanced in multiple ways. One method involves lowering the metabolic activity of the cells by printing in hypothermic conditions. Similar to hypothermic preservation of solid organs for extended preservation of transplant organs (typically done at 4 °C), decreasing the temperature in which cells are printed in will significantly decrease cellular metabolism. For example, the inventors have demonstrated that a cold room (4 °C) can be utilized to achieve hypothermic bioprinting. Additionally, incorporation of vitamins, growth factors, or serum in the prepolymerization solution, for readily accessible supply of nutrients, can be done to maintain or enhance cell viability. Importantly, photopolymerization is highly tolerant of decreased temperatures with only a modest increase in required exposure time. Further, decreasing the temperature of the entire 3D printing apparatus and reagents may help to quench heat generated from incident light or the photopolymerization process. In addition, molecules such as PEG and glycerol have been widely used as cryoprotectants in cell culture. Thus, use of a polymer such as PEG during the hypothermic printing process may enhance cell survivability.

Branching multi-scale transport systems are found in all multicellular life. Similar to the highly complex branching structure of vascular networks, the respiratory tree is also composed of a complex branching structure for sufficient supply of air in the distal regions of the lung. It has been indicated that endothelial cells may aid in lung epithelial branching. However, current manufacturing techniques do not allow for structures that mimic the anatomical complexity of native lung tissue. By using 3D printing, it should be possible to produce structures that mimic the anatomical complexity of native lung tissue and vasculature. The proposed approach can allow the printing of such structures and for embedding endothelial and epithelial cell types in channel lumens to mimic vascular and respiratory networks. The circular cross-sections attainable permit the development of confluent cell layers along the channel lumens. Given the higher z-resolution under the proposed approach, the channels can more closely mimic vascular and respiratory networks. The disclosed methods and materials can enable the fine control of the geometry and architecture of multiple networks. By using fractal, space-filling models akin to physiological vascular networks, the technology permits the design and fabrication of relevant 3D constructs with interpenetrating channels.

Additionally, the proposed approach can be combined with other scaffold fabrication techniques, such as porogen leaching or surface coating, to result in physiologically relevant complex constructs with modified internal microarchitecture or surface properties. Additionally, the proposed approach can be used for fabrication of microfluidic devices for organ-on-a-chip or human-on-a-chip applications. Additionally, the printer can be modified to include specific sensors for ensuring printing of more precise layer thickness.

In certain embodiments, a prepolymerization solution is provided. The prepolymerization solution comprises a photosensitive polymer having a molecular weight greater than 2,000 Daltons, a photoinitiator, and a biocompatible, light-absorbing additive material suitable to control light penetration. In some embodiments, the prepolymerization solution can also include one or more living cells (referred to herein simply as a cell). In some aspects, the prepolymerization solution can include a cryoprotectant such as low molecular weight PEG, glycerol, ethylene glycol, sucrose, propylene glycol, trehalose, raffinose, guar gum, xanthan gum, and D-mannitol. Cryoprotectants can be permeating or non-permeating. Each of these components is described in further detail herein. The prepolymerization solution can also include a water content of 10 wt % to about 99.5 wt %. The prepolymerization solution can include a water content of 80 wt % to about 90 wt %. In some aspects, the prepolymerization can further comprise DMEM media, serum, proteins, growth factors, thickening agents and/or anti-clumping components. Such components can provide nutrition for and/or neutral buoyancy for cells in the prepolymerization solution.

In certain embodiments, a photosensitive polymer having a molecular weight greater than 2,000 Daltons can be used. Photosensitive polymers can include at least two vinyl groups per molecule of polymer. Such vinyl groups can include acrylate, acrylamide and methacrylate. Photosensitive polymers which can be used include, by example but not limitation, poly(ethylene glycol) diacrylate (PEDGA), cell-adhesive poly(ethylene glycol), MMP-sensitive poly(ethylene glycol), poly(ethylene glycol) dimethacrylate (PEGDMA), poly(ethylene glycol) diacrylamide (PEGDAAm), gelatin methacrylate (GelMA), methacrylated hyaluronic acid (MeHA), and PEGylated fibrinogen. The photosensitive polymers can also be modified by the conjugation of cell-adhesive peptides such as CGRGDS (SEQ ID NO: 2).

In certain embodiments, a photoinitiator can be used. The photoinitiator is a molecule sensitive to a particular wavelength range that, upon light absorption, the molecule decays and releases free radicals which can catalyze hydrogel polymerization. Such photoinitiators can include, by way of example but not limitation, lithium acylphosphinate, Irgacure 2959, the Eosin Y system (consisting of eosin Y, 1-vinyl-2-pyrrolidinone (NVP), and triethanolamine (TEA)), tris(triphenlphosphine)ruthenium(II), and camphorquinone which is typically used with either ethyl 4-N,N-dimethylaminobenzoate or TEA and the photosensitizer isopropyl thioxanthone. High concentrations of photoinitiators can be used to achieve increased z-resolution by limiting the penetration depth of incident light, however, these high concentrations can disrupt the photopolymerization reaction and are cytotoxic. In addition, because light can shine through previously printed layers, use of high concentrations of photoinitiators can limit the ability to print complex overhang structures and cause phototoxicity to entrapped cells.

In certain embodiments a biocompatible, light-absorbing additive material can be used. The biocompatible, light-absorbing additive material is selected based on three criteria: 1) ability to absorb light wavelengths which fully encompass the photosensitive wavelength range of the photoinitiator, 2) limited participation or limited inhibition of photopolymerization reactions, and 3) biocompatibility at the concentrations desired. The biocompatible, light-absorbing additive material can be organic. The biocompatible, light-absorbing additive material can include, by way of example, but not limitation carbon black, yellow food coloring, tartrazine, nanoparticles, microparticles, gold nanoparticles, riboflavin, phenol red, Beta-carotene, curcumin, saffron, and turmeric. Additionally, cells that are transfected or transduced with proteins that absorb in the same region as the photoinitiator, such as cyan fluorescent protein (CFP) or green fluorescent protein (GFP), can be used at high concentrations, with reduced or no additives, to result in reduced lateral overcuring due to the light absorbing molecules present inside cells. The biocompatible, light-absorbing additive material enables printing of hydrogels with both horizontal and vertical channels due to stringent control of the penetration of the projected light which allows for high z-resolution as the penetration of light into already printed layers is reduced.

In some embodiments, a hydrogel matrix is provided. The hydrogel matrix can include a first tubular channel and a second tubular channel. The hydrogel matrix can be porous. The hydrogel matrix can also include a first cell type and a second cell type embedded therein. In certain aspects, the hydrogel matrix can include a first cell type embedded therein.

The hydrogel matrix can be produced in one or more layers and in certain embodiments, will include more than 1,000 layers, from about 10 layers to about 2,000 layers, from about 10 layers to about 1,000 layers, from about 10 to about 500 layers, from about 10 to about 100 layers, from about 100 to about 2,000 layers, from about 100 to about 1,000 layers, from about 100 layers to 500 layers, from about 100 layers to about 300 layers, from about 500 layers to about 1 ,000 layers, from about 500 layers to about 2,000 layers, from about 1 ,000 layers to about 2,000 layers, and any range therebetween of the above. In certain embodiments, each layer can have a thickness of from about 25 microns to about 100 microns, from about 50 microns to about 100 microns, from about 25 microns to about 50 microns, and any range therebetween. In certain other aspects, each layer can have a thickness of 50 microns. In still other aspects, each layer can have a thickness of less than 50 microns. In some embodiments, each layer can have a thickness of 25 microns. In certain aspects, each layer can have a thickness of 100 microns. In certain aspects, the one or more layers of the hydrogel matrix can include a first cell type wherein one or more other layers of the hydrogel matrix include a second cell type, but not the first cell type.

The one or more layers of the hydrogel matrix can have cells embedded therein. In certain aspects, the one or more layers of the hydrogel matrix adjacent to the one or more layers of the hydrogel matrix with embedded cells comprises an extracellular matrix protein.

The one or more layers of the hydrogel matrix can be formed from a photosensitive polymer. In certain aspects, the one or more layers of the hydrogel matrix can be formed from a second photosensitive polymer. The one or more layers of the hydrogel matrix can each include a first portion and second portion. In certain aspects, the first portion is formed from the photosensitive polymer and the second portion is formed from a second photosensitive polymer having a molecular weight of greater than 2,000 Daltons. In certain aspects, the first portion can include a first cell type embedded therein and the second portion can include a second cell type embedded therein, wherein the first cell type is different from the second cell type. In certain aspects, the first portion can include a first fluorophore and the second portion can include a second fluorophore, wherein the first fluorophore is different from the second fluorophore.

In some embodiments, a hydrogel can include a first tubular channel and a second tubular channel. In certain aspects, the first and second tubular channel each can include a horizontal segment that intersects more than one layer of the bulk hydrogel matrix. The second tubular channel can interpenetrate the first channel where interpenetrating is defined as the spatial relationship between two channels wherein one channel intersects at least once, a plane between two separate portions of the other channel. The tubular channels can also be branched. For example, the tubular channels may branch, as observed in the torus knot model, wherein the tubular channels reconverge at another point within the hydrogel. However, branched structures can also include channels which extend from the first tubular channel and/or the second tubular channel and terminate within the hydrogel. For, example, tree-like structures can be designed and produced using the present approach. In certain embodiments, the tubular channels have a diameter of 300 to 500 microns, 500 microns or less, 400 microns or less, or 300 microns or less. The tubular channel can also be perfusable. In addition, the tubular channels can also be expandable in response to increases in pressure therein. Tubular channels can be lined with cells, including epithelial and endothelial cells. In certain aspects, the first tubular channel is lined with endothelial cells. In certain aspects, the second tubular channel is lined with epithelial cells.

The first tubular channel can also include a first tubular inlet and a first tubular outlet on the surface of the hydrogel matrix. The second tubular channel can also include a second tubular inlet and a second tubular outlet on the surface of the hydrogel matrix. The first tubular channel can include a valve or other positive feature. Tubular channels can also include spikes that extend therefrom into the hydrogel matrix. Tubular channels can be filled with any appropriate fluid or gas. Such fluids or gases can include, by way of example but not limitation, bodily fluids and oxygen. In certain aspects, the first tubular channel can be filled with a fluid.

In certain other aspects, the first tubular channel can be filled with culture media, red blood cells, blood, urine, bile and/or gases such as nitrogen and/or oxygen. In certain aspects, the second tubular channel can be filled with culture media, red blood cells, blood, urine, bile and/or gases such as nitrogen and/or oxygen. Tubular channels can also be filled with one or more different fluids and/or gases.

Hydrogels of the present disclosure can include more than two tubular channels. For example, a hydrogel can include a third tubular channel and a fourth tubular channel. A tubular channel can interpenetrate more than one other tubular channel. For instance, a third tubular channel can interpenetrate a fourth tubular channel. Similarly, a second tubular channel can interpenetrate a first tubular channel and a third tubular channel. Tubular channel networks comprising multiple tubular channels may also interpenetrate at least one tubular channel or at least one other tubular channel network. For example, a third tubular channel may interpenetrate a first tubular channel that is also interpenetrated by a second tubular channel. As another example, a third tubular channel and fourth tubular channel can be interpenetrating and interpenetrate a first tubular channel or an interpenetrating network comprising a first tubular channel and a second tubular channel. In this manner, complex models can be constructed which permit complex interactions between tubular channels and tubular channel networks. The foregoing examples of multiple tubular channels are for exemplary purposes only and not intended to limit this disclosure.

It should be noted that list of polymers for various features of the present devices may overlap, as potential modifications to any given polymer can render it useful for the degradable or non-degradable portion. The degradable portion serves many purposes, including containing pro-angiogenic compounds, oxygen-releasing compounds, immune-modulating compounds, or other biologically active compounds, as well as cells, including endothelial cells. The degradable can affect the local site where it is implanted, including but not limited to vascularization, immunomodulation, and controlled release of other compounds, and/or confer useful advantages to the nondegradable portion of the device through these and other related means.

### B. Process for Manufacturing a Multi-Layer Hydrogel Matrix Construct

In certain embodiments not forming part of the invention, a process for manufacturing a multi-layer hydrogel matrix construct is provided. First, a 3D model of the multi-layer hydrogel matrix construct is created using a design software, wherein the 3D model of the multi-layer hydrogel matrix construct comprises a first computational algorithm that yields a first elongated void in the multilayer hydrogel matrix construct providing a first tubular channel, and a second computational algorithm that yields a second elongated void in the multilayer hydrogel matrix construct providing a second tubular channel, wherein the second computational algorithm results in the second tubular channel interpenetrating the first tubular channel. The 3D model is then converted to a format suitable for use in a 3D printing software to yield a formatted 3D model. The formatted 3D model is then imported into the 3D printing software, wherein the 3D printing software is programmed to slice the 3D model into multiple two-dimensional (2D) xy images. A prepolymerization solution is supplied to a vat associated with a build platform of a 3D printer, wherein the vat is transparent, and wherein the prepolymerization solution comprises a photosensitive polymer having a molecular weight of greater than 2,000 Daltons and at least two vinyl groups per molecule of polymer, a light-absorbing additive material to control light penetration, and a photoinitiator. The vat can also include a coating to which the hydrogel will not adhere such as a hydrophobic coating. For example, the coating can be polydimethylsiloxane (PDMS). This allows the hydrogel to separate from the vat without sticking. A mobile Z-axis stage of the 3D printer is positioned at a distance from the vat, wherein the Z-axis stage includes a surface sufficient for gelled material to adhere thereto, wherein the distance between the surface and an inner bottom surface of the vat is equivalent to a desired layer thickness of the tissue construct. A pattern is then projected on the inner bottom surface of the vat. For example, a light source may be projected through an optical configuration such as a DLP system, to produce the pattern. A layer of the multilayer hydrogel matrix construct is then polymerized. The steps of supplying a prepolymerization solution, positioning the mobile Z-axis stage, projecting the light source and polymerizing a layer can be repeated one or more times, wherein the mobile Z-axis stage is moved so that the distance moved is equivalent to the desired thickness of each subsequent layer, and wherein the same or a different pattern is displayed for each subsequent layer. In certain aspects, at least the steps of supplying a prepolymerization solution to a vat, positioning a mobile Z-axis stage, projecting a light source through an optical configuration, and polymerizing a layer of the multi-layer tissue construct are performed under hypothermic conditions. The hypothermic conditions can include a temperature of 4°C. The optical configuration can include a collimator, a condenser, filters, and a DMD array. For example, the optical configuration can be part of a digital light processing system.

In certain aspects, the distance is from 50 microns to 100 microns. In certain other aspects, the distance is 50 microns. The optical configuration can include a mirror, wherein the mirror is positioned to provide the pattern on the inner bottom surface of the vat. The mirror can be slid back and forth to center the projection and provide for alignment of the layers of the multi-layer hydrogel matrix construct.

In some embodiments not forming part of the invention, the repeating step is performed at least once to produce a second layer, wherein the prepolymerization solution used for the second layer comprises a second photosensitive polymer that is different from the photosensitive polymer used to fabricate the first layer. In certain aspects, the second photosensitive polymer can have a different molecular weight from the photosensitive polymer. The repeating step can be performed a number of times sufficient to yield the multi-layered hydrogel matrix construct having the first tubular channel and the second tubular channel.

In certain embodiments not forming part of the invention, the first computational algorithm can be derived from knot theory. In certain aspects, the first computational algorithm and/or the second computational algorithm can be a Hilbert curve. The first computational algorithm and second computational algorithm can conform to Murray's Law. In certain aspects, the first computational algorithm can result in the first tubular channel being branched. The first computational algorithm and the second computational algorithm can include a mathematical space-filling model. The mathematical space-filling model can include the Plumber's Nightmare, Peano curve, Hilbert curve, Pythagoras tree, and Brownian tree models.

The assembled 3D printer is an automated, computer-aided 3D prototyping device which utilizes additive manufacturing to selectively pattern photosensitive biomaterials one layer at a time, yielding a 3D tissue engineered construct. The printer contains a mobile Z-axis stage that is lowered onto the build platform which contains a vat with the prepolymerization solution containing photosensitive polymers and a photoinitiator. Attached to the Z-axis stage is a surface onto which the gelled material adheres to. Additionally, the base of the printer houses a 45 ° mirror which reflects a horizontal projection onto the inner bottom surface of the transparent vat. The assembled printer also houses microelectronics that automate the printing process.

In order to print structures, a 3D model is created in computer aided design (CAD) software and then exported to stereolithography (.stl) format. The .stl file is imported into software in which printing parameters are input. Then, the software computationally slices the 3D model into two-dimensional (2D) xy images, which act as dynamic photomasks, and uses the inputted print parameters to create commands for controlled automated printing. Once the transparent vat is filled with prepolymerization mixture, the Z-axis stage is lowered onto the vat so that the distance between the surface and the inner bottom surface of the vat is the desired layer thickness. Then, a light source projects light through an optical setup containing a combination of collimator, condenser, filters such as dichroic or bandpass to select specific wavelengths of interest, and a DMD array, such as a commercially available projector or a pico-projector, resulting in a pattern (the first layer of the 3D model), on the inner bottom surface of the vat, yielding a specific 3D patterned layer. After the material undergoes gelation, the Z-axis stage automatically moves up to the next layer height and the process is automatically continues with successive, automated projection/Z-axis stage movement until the final 3D construct is obtained.

Additionally, the power output from projector devices may not be homogenous. Thus, the inventors will homogenize power output, or flat-field, so that the projected pattern does not have unintentional heterogenous properties. To this end, they projected a blank exposure onto a film of phosphors and then obtained a luminescent image. This image contains a 2D matrix of intensity values that are essentially normalized and inverted in numerical computing programs, such as MATLAB. The inverted image essentially acts as a filter, resulting in a more homogenous power output to ensure that the gelation of materials is homogenous throughout the whole projected layer.

Modifications to this assembly can involve addition of a syringe setup to automatically dispense more prepolymerization solution during the printing process, as necessary. To achieve more heterogenous constructs with different materials, a modification to the assembly involves modifying the build platform, vat, and/or the Z-axis stage so that multiple materials can be automatically printed with this technique. For example, the build platform can be designed in such a way as to house multiple vats with different materials for printing 3D hydrogels with multiple materials.

Multiscale, branched vascular network with an interpenetrated airway network can be fabricated by using the bioprinter in its basic configuration. This model is prepared in CAD software and then exported into an ".stl" format. The exported file is uploaded to a software and the print parameters are entered. Once the prepolymerization solution is prepared, it is transferred onto the vat housed on the build platform. Then, the Z-axis stage is lowered to obtain the desired layer thickness. A series of automated projections of the 3D model and Z-stage translations ultimately results in the final 3D printed hydrogel. The 3D bioprinted hydrogel can then be removed from the Z-platform. The vascular channel of the 3D printed model can be endothelialized by perfusion of endothelial cells while the airway channel can be epithelialized by perfusion of epithelial cells. The vascular network serves as the blood supply, delivering oxygen and nutrients to the cells in the bulk of the hydrogel while the airway network provides a means to supply oxygen to the vascular network.

However, once the pressure of the inlet is less than that of the outlet, the valve closes, blocking the backflow of fluid through the leaflets. Negative features, such as spikes which have sharp tips can be exploited to control the spatial sprouting of seeded endothelial cells into the bulk hydrogel region.

Multi-layered vascular tubes consisting of an outer layer of fibroblasts, an inner layer of smooth muscle cells, and endothelialized lumen can be fabricated by using the bioprinter in a configuration that allows printing with multiple materials. Fibroblasts, smooth muscle cells, and endothelial cells are cultured separately with appropriate culture conditions. The fibroblast cells and smooth muscle cells are individually mixed at desired densities with the prepolymerization solution containing photopolymer and photoinitiator. Then, the prepolymerization solution, containing cells, is deposited in vat(s) on the build platform.

Additionally, the build platform may contain a vat with an acellular prepolymerization mixture that will be used to print the base of the 3D model. The Z-axis stage is lowered into the acellular prepolymerization mixture at the desired layer thickness, and the printing process is initiated. Once sufficient base layers are printed, the Z-axis stage retracts from the base vat and is lowered into the position of the second material, containing the prepolymerization mixture with smooth muscle cells, at the next layer height. After gelation of that layer, the Z-axis stage moves out of the second vat, is rinsed with an acellular prepolymerization mixture to reduce contamination, and is lowered into the third vat, containing the prepolymerization mixture with fibroblast cells, at the same layer height as the previous projection. After this layer is gelled, the resultant layer consists of a pattern with 2 different cell types. The Z-axis stage moves out of the third vat, is rinsed, and moves back to the second vat and the process is repeated until the final multilayered, multi-material 3D hydrogel is bioprinted. The 3D bioprinted hydrogel is then removed, and endothelialized by perfusing the channel with endothelial cells.

### II. Biological Materials

The disclosed devices can include one or more biologic materials. Materials are any compound, composition, conjugate, or construct that can be used to diagnose or treat a disease, disorder, condition, symptom, *etc.* Examples of such materials agents include cells, tissues, cell products, tissue products, proteins, antibodies, vaccines, vaccine components, antigens, epitopes, drugs, salts, nutrients, buffers, acids, and bases.

Biological material for inclusion in the disclosed devices can be any biological substance. For example, the biological material can be tissue, cells, biological micromolecules, or biological macromolecules. Examples of biological macromolecules include nucleotides, amino acids, cofactors, and hormones. Examples of biological macromolecules include nucleic acids, polypeptides, proteins, and polysaccharides.

Examples of proteins include enzymes, receptors, secretory proteins, structural proteins, signaling proteins, hormones, and ligands. Any class, type, form, or particular biological material can be used together with any other classes, types, forms, or particular biological materials.

### A. Cells

In some embodiments, the device can include a cell or tissue, *e.g.,* a living cell or tissue, which in some embodiments is encapsulated in, or coated with, a polymer. In such embodiments, the surface of the polymer encapsulation or coating is modified with moieties or compounds disclosed herein. In some embodiments, the cell can include an exogenous nucleic acid that encodes a therapeutic or diagnostic polypeptide.

The cell type chosen for inclusion in the disclosed devices depends on the desired therapeutic effect. The cells may be from the patient (autologous cells), from another donor of the same species (allogeneic cells), or from another species (xenogeneic). Xenogeneic cells are easily accessible, but the potential for rejection and the danger of possible transmission of viruses to the patient restricts their clinical application. Any of these types of cells can be from natural sources, stem cells, derived cells, or genetically engineered cell.

In some embodiments, the cell is a genetically engineered cell that secretes a therapeutic agent, such as a protein or hormone for treating a disease or other condition. In some embodiments, the cell is a genetically engineered cell that secretes a diagnostic agent. In some embodiments, the cell is a stem cell, *e.g.,* an embryonic stem cell, mesenchymal stem cell, hepatic stem cell, or bone marrow stem cell.

Types of cells for inclusion in the disclosed devices include cells from natural sources, such as cells from xenotissue, cells from a cadaver, and primary cells; stem cells, such as embryonic stem cells, mesenchymal stem cells, and induced pluripotent stem cells; derived cells, such as cells derived from stem cells, cells from a cell line, reprogrammed cells, reprogrammed stem cells, and cells derived from reprogrammed stem cells; and genetically engineered cells, such as cells genetically engineered to express a protein or nucleic acid, cells genetically engineered to produce a metabolic product, and cells genetically engineered to metabolize toxic substances.

Types of cells for inclusion in the disclosed devices include liver cells *(e.g.,* hepatoblasts liver stellate cells, biliary cells, or hepatocytes), insulin producing cells (*e.g.,* pancreatic islet cells, isolated pancreatic beta cells, or insulinoma cells), kidney cells, epidermal cells, epithelial cells, neural cells, including neurons and glial cells (*e.g*., astrocytes), ganglion cells, retinal epithelial cells, adrenal medulla cells, lung cells, cardiac muscle cells, osteoblast cells, osteoclast cells, bone marrow cells, spleen cells, thymus cells, glandular cells, blood cells (*e.g.,* T cells, B cells, macrophage lineage cells, lymphocytes, or monocytes), endocrine hormone-producing cells (*e.g.*, parathyroid, thyroid, or adrenal cells), cells of intestinal origin and other cells acting primarily to synthesize and secret or to metabolize materials, endothelial cells (*e.g.,* capillary endothelial cells), fibroblasts (*e.g.,* dermal fibroblasts), myogenic cells, keratinocytes, smooth muscle cells, progenitor cells (*e.g.,* bone marrow progenitor cells, adipose progenitor cells, hepatic precursor cells, endothelia progenitor cells, peripheral blood progenitor cells, or progenitor cells from muscle, skin ) marrow stromal cells cell lines (*e.g.,* CHO cells, MDCK cells and PC12 cells).

A particular cell type is a pancreatic islet cell or other insulin-producing cell. Hormone-producing cells can produce one or more hormones, such as insulin, parathyroid hormone, antidiuretic hormone, oxytocin, growth hormone, prolactin, thyroid stimulating hormone, adrenocorticotropic hormone, follicle-stimulating hormone, lutenizing hormone, thyroxine, calcitonin, aldosterone, Cortisol, epinephrine, glucagon, estrogen, progesterone, and testosterone. Genetically engineered cells are also suitable for inclusion in the disclosed devices. In some embodiments, the cells are engineered to produce one or more hormones, such as insulin, parathyroid hormone, antidiuretic hormone, oxytocin, growth hormone, prolactin, thyroid stimulating hormone, adrenocorticotropic hormone, follicle-stimulating hormone, lutenizing hormone, thyroxine, calcitonin, aldosterone, Cortisol, epinephrine, glucagon, estrogen, progesterone, and testosterone. In some embodiments, the cells are engineered to secrete blood clotting factors *(e.g.,* for hemophilia treatment) or to secrete growth hormones. In some embodiments, the cells are contained in natural or bioengineered tissue. For example, the cells for inclusion in the disclosed devices are in some embodiments a bioartificial renal glomerulus. In some embodiments, the cells are suitable for transplantation into the central nervous system for treatment of neurodegenerative disease.

In certain embodiments, a cell may be included in the prepolymerization solution and/or hydrogel. Such cells can include endothelial and epithelial cell types. Such cells can be human mesenchymal stem cells (hMSCs). By way of example but not limitation, epithelial cells can include human bronchial epithelial cells HBECs), columnar ciliated epithelial cells, mucous cells, serous cells, basal cells, Clara cells, neureoendocrme cells, type I and type II alveolar cells, and A549 adenocarcinomic human alveolar basal epithelial cells. By way of example but not limitation, epithelial cells can be characterized based on staining for E-cadherin, surfactant proteins A, C and D, basal marker keratin 14, and TTF-1 Similarly, by way of example but not limitation, endothelial cells can include human umbilical vein endothelial cells HUVECs), human pulmonary microvascular endothelial cells, and induced pluripotent endothelial cells. By way of example but not limitation, endothelial cells can be characterized based on statining for platelet endothelial cell adhesion molecular (PECAM/CD31) and vascular endothelial (VE)-cadherin.

Cells can be obtained directly from a donor, from cell culture of cells from a donor, or from established cell culture lines. In particular embodiments, cells are obtained directly from a donor, washed and implanted directly in combination with the polymeric material. The cells are cultured using techniques known to those skilled in the art of tissue culture.

Cell viability can be assessed using standard techniques, such as histology and fluorescent microscopy. The function of the implanted cells can be determined using a combination of these techniques and functional assays. For example, in the case of hepatocytes, *in vivo* liver function studies can be performed by placing a cannula into the recipient's common bile duct. Bile can then be collected in increments. Bile pigments can be analyzed by high pressure liquid chromatography looking for underivatized tetrapyrroles or by thin layer chromatography after being converted to azodipyrroles by reaction with diazotized azodipyrroles ethylanthranilate either with or without treatment with P-glucuronidase. Diconjugated and monoconjugated bilirubin can also be determined by thin layer chromatography after alkalinemethanolysis of conjugated bile pigments. In general, as the number of functioning transplanted hepatocytes increases, the levels of conjugated bilirubin will increase. Simple liver function tests can also be done on blood samples, such as albumin production. Analogous organ function studies can be conducted using techniques known to those skilled in the art, as required to determine the extent of cell function after implantation. For example, pancreatic islet cells and other insulin-producing cells can be implanted to achieve glucose regulation by appropriate secretion of insulin. Other endocrine tissues and cells can also be implanted.

The site, or sites, where cells are to be implanted is determined based on individual need, as is the requisite number of cells. For cells replacing or supplementing organ or gland function (for example, hepatocytes or islet cells), the mixture can be injected into the mesentery, subcutaneous tissue, retroperitoneum, preperitoneal space, and intramuscular space.

The amount and density of cells included in the disclosed devices will vary depending on the choice of cell and site of implantation. In some embodiments, the single cells are present in the hydrogel capsule at a concentration of 0.1 × 10⁶ to 4 × 10⁶ cells/ml, more particularly 0.5 × 10⁶ to 2 × 10⁶ cells/ml. In other embodiments, the cells are present as cell aggregates. For example, islet cell aggregates (or whole islets) preferably contain about 1500-2000 cells for each aggregate of 150µm diameter, which is defined as one islet equivalent (IEQ). Therefore, in some embodiments, islet cells are present at a concentration of 100-10000 IE/ml, particularly 200-3,000 IE/ml, more particularly 500- 1500 IEQ/ml.

### 1. Islet Cells and Other Insulin-Producing Cells

In particular embodiments, the disclosed compositions contain islet cells or other insulin-producing cells. Methods of isolating pancreatic islet cells are known in the art. Field et al., Transplantation 61: 1554 (1996); Linetsky et al., Diabetes 46: 1 120 (1997). Fresh pancreatic tissue can be divided by mincing, teasing, comminution and/or collagenase digestion. The islets can then be isolated from contaminating cells and materials by washing, filtering, centrifuging or picking procedures. Methods and apparatus for isolating and purifying islet cells are described in U.S. Patent Nos. 5,447,863, 5,322,790, 5,273,904, and 4,868,121. The isolated pancreatic cells may optionally be cultured prior to inclusion in the hydrogel capsule using any suitable method of culturing islet cells as is known in the art. See *e.g.,* U.S. Patent No. 5,821,121. Isolated cells may be cultured in a medium under conditions that helps to eliminate antigenic components. Insulin- producing cells can also be derived from stem cells and cell lines and can be cells genetically engineered to produce insulin.

### 2. Genetically Engineered Cells

In some embodiments, the disclosed compositions contain cells genetically engineered to produce a protein or nucleic acid *(e.g.,* a therapeutic protein or nucleic acid). In these embodiments, the cell can be, for example, a stem cell *(e.g.,* pluripotent), a progenitor cell *(e.g.,* multipotent or oligopotent), or a terminally differentiated cell (i.e., unipotent). Any of the disclosed cell types can be genetically engineered. The cell can be engineered, for example, to contain a nucleic acid encoding, for example, a polynucleotide such miRNA or RNAi or a polynucleotide encoding a protein. The nucleic acid can be, for example, integrated into the cells genomic DNA for stable expression or can be, for example, in an expression vector *(e.g.,* plasmid DNA). The polynucleotide or protein can be selected based on the disease to be treated (or effect to be achieved) and the site of transplantation or implantation. In some embodiments, the polynucleotide or protein is anti-neoplastic. In other embodiments, the polynucleotide or protein is a hormone, growth factor, or enzyme.

### B. Non-Cellular Materials

In some embodiments, the cells secrete a therapeutically effective substance, such as a protein or nucleic acid. In some embodiments, the cells produce a metabolic product. In some embodiments, the cells metabolize toxic substances. In some embodiments, the cells form structural tissues, such as skin, bone, cartilage, blood vessels, or muscle. In some embodiments, the cells are natural, such as islet cells that naturally secrete insulin, or hepatocytes that naturally detoxify. In some embodiments, the cells are genetically engineered to express a heterologous protein or nucleic acid and/or overexpress an endogenous protein or nucleic acid. In some embodiments, the cells are genetically engineered to produce a new or different product, which can be an expression product of the engineered gene(s) or another product, such as a metabolite, produced because of the engineered gene(s).

Therapeutic agents that can be included in the device, or engineered into cells included in the device include, for example, thyroid stimulating hormone; beneficial lipoproteins such as Apol; prostacyclin and other vasoactive substances, anti-oxidants and free radical scavengers; soluble cytokine receptors, for example soluble transforming growth factor (TGF) receptor, or cytokine receptor antagonists, for example ILlra; soluble adhesion molecules, for example ICAM-1 ; soluble receptors for viruses, *e.g*., CD4, CXCR4, CCR5 for HIV; cytokines; elastase inhibitors; bone morphogenetic proteins (BMP) and BMP receptors 1 and 2; endoglin; serotonin receptors; tissue inhibiting metaloproteinases; potassium channels or potassium channel modulators; anti-inflammatory factors; angiogenic factors including vascular endothelial growth factor (VEGF), transforming growth factor (TGF), hepatic growth factor, and hypoxia inducible factor (HIF); polypeptides with neurotrophic and/or anti-angiogenic activity including ciliary neurotrophic factor (CNTF), glial-derived neurotrophic factor (GDNF), nerve growth factor (NGF), brain-derived neurotrophic factor (BDNF), neurotrophin-3, nurturin, fibroblast growth factors (FGFs), endostatin, ATF, fragments of thrombospondin, variants thereof and the like. More particular polypeptides are FGFs, such as acidic FGF (aFGF), basic FGF (bFGF), FGF-1 and FGF-2 and endostatin.

In some embodiments, the active agent is a protein or peptide. Examples of protein active agents include, but are not limited to, cytokines and their receptors, as well as chimeric proteins including cytokines or their receptors, including, for example tumor necrosis factor alpha and beta, their receptors and their derivatives; renin; lipoproteins; colchicine; prolactin; corticotrophin; vasopressin; somatostatin; lypressin; pancreozymin; leuprolide; alpha-1-antitrypsin; clotting factors such as factor VIIIC, factor IX, tissue factor, and von Willebrands factor; anti-clotting factors such as Protein C; atrial natriuretic factor; lung surfactant; a plasminogen activator other than a tissue-type plasminogen activator (t-PA), for example a urokinase; bombesin; thrombin; hemopoietic growth factor; enkephalinase; RANTES (regulated on activation normally T-cell expressed and secreted); human macrophage inflammatory protein (MIP-1 -alpha); a serum albumin such as human serum albumin; mullerian-inhibiting substance; relaxin A-chain; relaxin B-chain; prorelaxin; mouse gonadotropin-associated peptide; chorionic gonadotropin; a microbial protein, such as betalactamase; DNase; inhibin; activin; receptors for hormones or growth factors; integrin; protein A or D; rheumatoid factors; platelet-derived growth factor (PDGF); epidermal growth factor (EGF); transforming growth factor (TGF) such as TGF-a and TGF-β, including TGF-βI, TGF-2, TGF-3, TGF-4, or TGF-5; insulin-like growth factor-I and -II (IGF-I and IGF-II); des(l-3)-IGF-I (brain IGF-I), insulin-like growth factor binding proteins; CD proteins such as CD- 3, CD-4, CD-8, and CD- 19; erythropoietin; osteoinductive factors; immunotoxins; an interferon such as interferon-alpha (*e.g*., interferon alpha 2A), -beta, -gamma, -lambda and consensus interferon; colony stimulating factors (CSFs), *e.g.,* M-CSF, GM-CSF, and G-CSF; interleukins (ILs), *e.g.*, IL-1 to IL-10; superoxide dismutase; T-cell receptors; surface membrane proteins; decay accelerating factor; transport proteins; homing receptors; addressins; fertility inhibitors such as the prostaglandins; fertility promoters; regulatory proteins; antibodies (including fragments thereof) and chimeric proteins, such as immunoadhesins; precursors, derivatives, prodrugs and analogues of these compounds, and pharmaceutically acceptable salts of these compounds, or their precursors, derivatives, prodrugs and analogues. Suitable proteins or peptides may be native or recombinant and include, *e.g.*, fusion proteins.

Examples of protein active agents also include CCL1, CCL2 (MCP-1), CCL3 (MIP-Iα), CCL4 (MIP-Iβ), CCL5 (RANTES), CCL6, CCL7, CCL8, CCL9 (CCL10), CCL11, CCL12, CCL13, CCL14, CCL15, CCL16, CCL17, CCL18, CCL19, CCL20, CCL21, CCL22, CCL23, CCL24, CCL25, CCL26, CCL27, CCL28, CXCL1 (KC), CXCL2 (SDFla), CXCL3, CXCL4, CXCL5, CXCL6, CXCL7, CXCL8 (IL8), CXCL9, CXCL10, CXCL11, CXCL12, CXCL13, CXCL14, CXCL15, CXCL16, CXCL17, CX3CL1, XCL1, XCL2, TNFA, TNFB (LTA), TNFC (LTB), TNFSF4, TNFSF5 (CD40LG), TNFSF6, TNFSF7, TNFSF8, TNFSF9, TNFSF10, TNFSF11, TNFSF13B, EDA, IL2, IL15, IL4, IL13, IL7, IL9, IL21, IL3, IL5, IL6, ILl l, IL27, IL30, IL31, OSM, LIF, CNTF, CTFl, IL12a, IL12b, IL23, IL27, IL35, IL14, IL16, IL32, IL34, IL10, IL22, IL19, IL20, IL24, IL26, IL29, IFNL1, IFNL2, IFNL3, IL28, IFNA1, IFNA2, IFNA4, IFNA5, IFNA6, IFNA7, IFNA8, IFNA10, IFNA13, IFNA14, IFNA16, IFNA17, IFNA21, IFNB1, IFNK, IFNW1, IFNG, ILIA (IL1F1), IL1B (IL1F2), ILIRa (IL1F3), IL1F5 (IL36RN), IL1F6 (IL36A), IL1F7 (IL37), IL1F8 (IL36B), IL1F9 (IL36G), ILIFIO (IL38), IL33 (IL1F11), IL18 (IL1G), IL17, KITLG, IL25 (IL17E), CSF1 (M-CSF), CSF2 (GM-CSF), CSF3 (G-CSF), SPP1, TGFB1, TGFB2, TGFB3, CCL3L1, CCL3L2, CCL3L3, CCL4L1, CCL4L2, IL17B, IL17C, IL17D, IL17F, AIMP1 (SCYE1), MIF, Areg, BC096441, Bmpl, BmplO, Bmpl5, Bmp2, Bmp3, Bmp4, Bmp5, Bmp6, Bmp7, Bmp8a, Bmp8b, Clqtnf4, Ccl21a, Ccl27a, Cd70, Cerl, Cklf, Clcfl, Cmtm2a, Cmtm2b, Cmtm3, Cmtm4, Cmtm5, Cmtm6, Cmtm7, Cmtm8, Crlfl, Ctf2, Ebi3, Ednl, Fam3b, Fasl, Fgf2, Flt31, GdflO, Gdfl 1, Gdfl5, Gdf2, Gdf3, Gdf5, Gdf6, Gdf7, Gdf9, Gml2597, Gml3271, Gml3275, Gml3276, Gml3280, Gml3283, Gm2564, Gpil, Greml, Grem2, Grn, Hmgbl, Ifnal 1, Ifnal2, Ima9, Ifnab, Ifne, 1117a, 1123a, 1125, 1131, Iltifb,Inhba, Lefty1, Lefty 2, Mstn, Nampt, Ndp, Nodal, Pf4, Pgly 1, Prl7dl, Scg2, Scgb3al, Slurpl, Sppl, Thpo, TnfsflO, Tnfsfl l, Tnfsfl2, Tnfsfl3, Tnfsfl3b, Tnfsfl4, Tnfsfl5, Tnfsfl8, Tnfsf4, Tnfsf8, Tnfsf9, Tslp, Vegfa, Wntl, Wnt2, Wnt5a, Wnt7a, Xcll, Epinephrine, Melatonin, Triiodothyronine, Thyroxine, Prostaglandins, Leukotrienes, Prostacyclin, Thromboxane, Islet Amyloid Polypeptide, Miillerian inhibiting factor or hormone, Adiponectin, Corticotropin, Angiotensin, vasopressin, arginine vasopressin, atriopeptin, Brain natriuretic peptide, Calcitonin, Cholecystokinin, Cortistatin, Enkephalin, Endothelin, Erythropoietin, Follicle-stimulating hormone, Galanin, Gastric inhibitory polypeptide, Gastrin, Ghrelin, Glucagon, Glucagon-like peptide-1, Gonadotropin- releasing hormone, Growth hormone-releasing hormone, Hepcidin, Human chorionic gonadotropin, Human placental lactogen, Growth hormone, Inhibin, Insulin, Somatomedin, Leptin, Lipotropin, Luteinizing hormone, Melanocyte stimulating hormone, Motilin, Orexin, Oxytocin, Pancreatic polypeptide, Parathyroid hormone, Pituitary adenylate cyclase-activating peptide, Prolactin, Prolactin releasing hormone, Relaxin, Renin, Secretin, Somatostatin, Thrombopoietin, Thyrotropin, Thyrotropin- releasing hormone, Vasoactive intestinal peptide, Androgen, Androgen, acid maltase (alpha-glucosidase), glycogen phosphorylase, glycogen debrancher enzyme, Phosphofructokinase, Phosphogly cerate kinase, Phosphogly cerate mutase, Lactate dehydrogenase, Carnitine palymityl transferase, Carnitine, and Myoadenylate deaminase.

Hormones to be included in the disclosed devices or to be produced from cells included in the devices can be any hormone of interest. Examples of endocrine hormones include Antidiuretic Hormone (ADH), which is produced by the posterior pituitary, targets the kidneys, and affects water balance and blood pressure; Oxytocin, which is produced by the posterior pituitary, targets the uterus, breasts, and stimulates uterine contractions and milk secretion; Growth Hormone (GH), which is produced by the anterior pituitary, targets the body cells, bones, muscles, and affects growth and development; Prolactin, which is produced by the anterior pituitary, targets the breasts, and maintains milk secretions; Growth Hormone-Releasing Hormone (GHRH), which is a releasing hormone of GH and is produced in the arcuate nucleas of the hypothalamus; Thyroid Stimulating Hormone (TSH), which is produced by the anterior pituitary, targets the thyroid, and regulates thyroid hormones; Thyrotropin-Release Hormone (TRH), which is produced by the hypothalamus and stimulates the release of TSH and prolactin from the anterior pituitary; Adrenocorticotropic Hormone (ACTH), which is produced by the anterior pituitary, targets the adrenal cortex, and regulates adrenal cortex hormones; Follicle-Stimulating Hormone (FSH), which is produced by the anterior pituitary, targets the ovaries/testes, and stimulates egg and sperm production; Lutenizing Hormone (LH), which is produced by the anterior pituitary, targets the ovaries/testes, and stimulates ovulation and sex hormone release; Luteinizing Hormone-Releasing Hormone (LHRH), also known as Gonadotropin- Releasing Hormone (GnRH), which is synthesized and released from GnRH neurons within the hypothalamus and is a trophic peptide hormone responsible for the release of FSH and LH; Thyroxine, which is produced by the thyroid, targets the body cells, and regulates metabolism; Calcitonin, which is produced by the thyroid, targets the adrenal cortex, and lowers blood calcium; Parathyroid Hormone, which is produced by the parathyroid, targets the bone matrix, and raises blood calcium; Aldosterone, which is produced by the adrenal cortex, targets the kidney, and regulates water balance; Cortisol, which is produced by the adrenal cortex, targets the body cells, and weakens immune system and stress responses; Epinephrine, which is produced by the adrenal medulla, targets the heart, lungs, liver, and body cells, and affects primary "fight or flight" responses; Glucagon, which is produced by the pancreas, targets the liver body, and raises blood glucose level; Insulin, which is produced by the pancreas, targets body cells, and lowers blood glucose level; Estrogen, which is produced by the ovaries, targets the reproductive system, and affects puberty, menstrual, and development of gonads; Progesterone, which is produced by the ovaries, targets the reproductive system, and affects puberty, menstrual cycle, and development of gonads; and Testosterone, which is produced by the adrenal gland, testes, targets the reproductive system, and affects puberty, development of gonads, and sperm.

In some embodiments, the protein is a growth hormone, such as human growth hormone (hGH), recombinant human growth hormone (rhGH), bovine growth hormone, methionehuman growth hormone, des-phenylalanine human growth hormone, and porcine growth hormone; insulin, insulin A-chain, insulin B-chain, and proinsulin; or a growth factor, such as vascular endothelial growth factor (VEGF), nerve growth factor (NGF), platelet-derived growth factor (PDGF), fibroblast growth factor (FGF), epidermal growth factor (EGF), transforming growth factor (TGF), and insulin-like growth factor-I and -II (IGF-I and IGF-II).

### III. Immunoisolating Alginates

A fundamental barrier to successful cell therapy, including in the context of implantable devices, is the lack of biocompatible transplantation devices. Implanted biomaterials lead to foreign body responses (FBRs), which are cascades of inflammatory events and wound-healing processes that lead to fibrosis (walling off) and subsequent implant failure (12-14). This response has been described for many materials, from naturally occurring polymers to synthetic materials (15-17). Thus, a critical medical need exists to develop biomaterials that overcome this key challenge of eliminating the FBR.

To address this issue, the inventors contemplate the use of immunoisolating alginates to create protective coatings for cells contained in the devices of the present application. The have identified immunomodulatory small molecules using combinatorial chemistry and high throughput screening (Vegas *et al.,* 2016b) and validated the long-term effectiveness of these small molecules in the prevention of fibrosis and long-term cell survival in non-human primates.

The present disclosure provides, in certain embodiments not forming part of the invention, a device containing a degradable shell and a nondegradable core. In certain embodiments, the nondegradable core can include encapsulated therapeutic cells and/or biosensors, wherein the degradable shell serves as a scaffold for blood vessel growth, resulting in enhanced blood flow to the cells and/or biosensors. The inventors have applied the latest in 3D bioprinting technology to direct vascularization of immunoisolated therapeutic cell constructs for long-term viability and functionality, and to permit engraftment of higher densities of therapeutic cells.

One embodiment not forming part of the invention provides for the use of modified alginate polymers in the construction of cell containing devices. Modified alginate polymers can be of any desired molecular weight. The weight average molecular weight of the alginates is preferably between 1,000 and 1,000,000 Daltons, more preferably between 10,000 and 500,000 Daltons as determined by gel permeation chromatography.

Modified alginate polymers can contain any ratio of mannuronate monomers, guluronate monomers, and covalently modified monomers. In some embodiments, greater than 2.5%, 5%, 7.5%, 10%, 12%, 14%, 15%, 16%, 18%, 20%, 22%, 24%, 25%, 26%, 28%, 30%, 32.5%, 35%, 37.5%, 40%, 45%, 50%, 55%, or 60% of the monomers in the modified alginate polymer are covalently modified monomers. Greater than 10%, greater than 20%, or greater than 30% of the monomers in the modified alginate polymer are covalently modified monomers.

Modified alginate polymers can be produced incorporating covalently modified monomers possessing a range of different hydrogen bonding potentials, hydrophobicities/hydrophilicities, and charge states. The inclusion of covalently modified monomers into an alginate polymer alters the physiochemical properties of alginate polymer. Accordingly, the physiochemical properties of alginates can be tuned for desired applications by the selective incorporation of covalently modified monomers.

For example, the glass transition temperature (T_{g}), can be varied by the incorporation of covalently modified monomers. In some embodiments, the modified alginate polymer powder possess a T_{g}, as measured by differential scanning calorimetry (DSC), of greater than 50°C, 60°C, 65°C, 70°C, 75°C, 80°C, 85°C, 90°C, 95°C, 100°C, 105°C, 110°C, 115°C, 120°C, 125°C, 130°C, 135°C, 140°C, 145°C, 150°C, 160°C, 175°C, 190°C, or 200°C.

The hydrophobicity/hydrophilicity of alginates can be varied by the incorporation of hydrophobic and/or hydrophilic covalently modified monomers. In particular embodiments, the modified alginate polymer contains one or more hydrophobic covalently modified monomers. The relative hydrophobicity/hydrophilicity of modified alginates can be quantitatively assessed by measuring the contact angle of a water droplet on a film of the modified alginate polymer using a goniometer. In some embodiments, the modified alginate has a contact angle of less than 90° (i.e. it is hydrophilic). In particular embodiments, the modified alginate has a contact angle of more than 90° (*i.e.,* it is hydrophobic). In some embodiments, the modified alginate has a contact angle of more than 95°, 100°, 105°, 110°, 115°, or 120°.

In embodiments used for cell encapsulation, the modified alginate polymer can be ionically crosslinked by a polyvalent cation such as Ca²⁺, Sr²⁺, or Ba²⁺ to form hydrogels.

In some embodiments, the modified alginate polymer forms hydrogels such that the fluorescence intensity measured using the high throughput hydrogel formation assay described herein is greater than 10,000, 15,000, 20,000, 25,000, 30,000, 35,000, 40,000, 45,000, 50,000, or 55,000. In particular embodiments, the modified alginate polymer forms hydrogels such that the fluorescence intensity measured using the high throughput hydrogel formation assay described herein is greater than 15,000. In particular embodiments, the modified alginate polymer forms hydrogels such that the fluorescence intensity measured using the high throughput hydrogel formation assay described herein is between 15,000 and 55,000, preferably between 20,000 and 55,000, more preferably between 25,000 and 55,000.

In embodiments used for cell encapsulation, the modified alginate polymer forms a hydrogel with sufficient porosity to permit nutrients, waste, and the hormones and/or proteins secreted from encapsulated cells to diffuse freely into and out of the capsules, while simultaneously preventing the incursion of immune cells into the gel matrix. The porosity and surface area of modified alginate hydrogels can be measured using BET analysis. Prior to BET analysis, solvent and volatile impurities are removed by prolonged heating of the modified alginate gel under vacuum. Subsequently, the hydrogel samples are cooled under vacuum, for example by liquid nitrogen, and analyzed by measuring the volume of gas (typically N₂, Kr, CO₂, or Ar gas) adsorbed to the hydrogel at specific pressures. Analysis of the physisorption of the gas at variable pressures is used to characterize the total surface area and porosity of gels formed by the modified alginate polymers. A particular method of determining hydrogel porosity is BET analysis.

In particular embodiments, the modified alginate forms a hydrogel with sufficient porosity to permit nutrients, waste, and the hormones and/or proteins secreted from encapsulated cells to diffuse freely into and out of the capsules, while simultaneously preventing the incursion of immune cells into the gel matrix. In some embodiments, the porosity of the hydrogel formed by the modified alginate polymer is increased by 5%, 10%, 15%, or 20% relative to the porosity of a hydrogel formed from the unmodified alginate polymer. In alternative embodiments, the porosity of the hydrogel formed by the modified alginate polymer is decreased by 5%, 10%, 15%, or 20% relative to the porosity of a hydrogel formed from the unmodified alginate polymer.

In particular embodiments used for cell encapsulation, the modified alginate is biocompatible. The biocompatibility of modified alginates can be quantitatively determined using the fluorescence-based *in vivo* biocompatibility assay described in Example 5. In this assay, cathepsin activity was measured using an *in vivo* fluorescence assay to quantify the foreign body response to the modified alginate.

In some embodiments, the modified alginate polymer is biocompatible such that the fluorescence response normalized to unmodified alginate measured using the *in vivo* biocompatibility assay described herein is less than 100%, 95%, 90%, 85%, 80%, 75%, 70%, 65%, 60%, 55%, 50%, 45%, or 40%. In particular embodiments, the modified alginate polymer induces a lower foreign body response than unmodified alginate. This is indicated by fluorescence response normalized to unmodified alginate of less than 100%. In some embodiments, the modified alginate polymer is biocompatible such that the fluorescence response normalized to unmodified alginate measured using the *in vivo* biocompatibility assay described herein is less than 75%, more preferably less than 65%, and most preferably less than 50%.

The modified alginates can be chemically modified as described herein to any desired density of modifications. The density of modifications is the average number of modifications (that is, attached compounds) per a given weight, volume, or area of the surface of a capsule or product that includes he modified alginate. Generally, a density at or above a threshold density can provide a beneficial effect, such as lower foreign body response. In some embodiments, a high density is not required. Without being bound to any particular theory of operation, it is believed that the chemical modifications signal to, indicate to, or are identified by, one or more immune system or other body components to result in a beneficial effect, such as a lower foreign body response. In some embodiments, a lower density of modifications can be effective for this purpose.

Useful densities include densities of at least, of less than, of about, or of 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, 100, 110, 120, 130, 140, 150, 160, 170, 180, 190, 200, 210, 220, 230, 240, 250, 260, 270, 280, 290, 300, 320, 340, 360, 380, 400, 420, 440, 460, 480, 500, 550, 600, 650, 700, 750, 800, 850, 900, and 1000 modifications per square µm, per µg, or per cubic µm. All ranges defined by any pair of these densities are also specifically contemplated and disclosed.

In some embodiments, the density of the modifications on a surface, surfaces, or portions of a surface(s) of a capsule or product that, when the product is administered to (e.g., implanted in the body of) a subject, would be in contact with fluid(s), cell(s), tissue(s), other component(s), or a combination thereof of the subject's body is greater than the density of the modifications on other surfaces of the product.

Density can also be expressed in terms of the concentration of the surface modifications as measured by X-ray photoelectron spectroscopy (XPS). XPS is a surface-sensitive quantitative spectroscopic technique that measures the elemental composition at the parts per thousand range of the elements that exist within a material.

XPS spectra are obtained by irradiating a material with a beam of X-rays while simultaneously measuring the kinetic energy and number of electrons that escape from the top 0 to 10 nm of the material being analyzed. By measuring all elements present on the surface, the percentage of the elements that come from the surface modifications can be calculated. This can be accomplished by, for example, taking the percentage of nitrogen (and/or other elements in the surface modifications) in the total elemental signal measured. Nitrogen is a useful indicator for the surface modification because many substrated and materials forming the capsule or product contain little nitrogen. For convenience, the percent of the element(s) used to indicate the surface modifications can be stated as the percent surface modifications. Also for convenience, the percent surface modifications can be referred to as the concentration of surface modifications.

Useful percent surface modifications include concentrations of about 0.1 , 0.2, 0.3, 0.4, 0.5, 0.6, 0.7, 0.8, 0.9, 1 , 2, 3, 4, 5, 6, 7, 8, 9, 10, 1 1, 12, 13, 14, 15, 16, 17, 18, 19, 20, 25, 30, 35, 40, 45, 50, 55, 60, 65, 70, 75, 80, 85, 90, 95, and 100 percent surface modifications. All ranges defined by any pair of these concentrations are also specifically contemplated and disclosed.

Useful percent surface modifications also include concentrations of less than 0.1, 0.2, 0.3, 0.4, 0.5, 0.6, 0.7, 0.8, 0.9, 1, 2, 3, 4, 5, 6, 7, 8, 9, 10, 11 , 12, 13, 14, 15, 16, 17, 18, 19, 20, 25, 30, 35, 40, 45, 50, 55, 60, 65, 70, 75, 80, 85, 90, 95, and 100 percent surface modifications. All ranges defined by any pair of these concentrations are also specifically contemplated and disclosed.

Useful percent surface modifications also include concentrations of 0.1, 0.2, 0.3, 0.4, 0.5, 0.6, 0.7, 0.8, 0.9, 1 , 2, 3, 4, 5, 6, 7, 8, 9, 10, 1 1, 12, 13, 14, 15, 16, 17, 18, 19, 20, 25, 30, 35, 40, 45, 50, 55, 60, 65, 70, 75, 80, 85, 90, 95, and 100 percent surface modifications. All ranges defined by any pair of these concentrations are also specifically contemplated and disclosed.

In one aspect not forming part of the invention, the alginates may be used to form a capsule. Capsules are particles having a mean diameter of about 150 µm to about 5 cm. The disclosed capsules can be formed of cross-linked hydrogel. Other than the encapsulated material, the capsules, for example, can be formed solely of cross-linked hydrogel, can have a cross-linked hydrogel core that is surrounded by one or more polymeric shells, can have one or more cross-linked hydrogel layers, can have a cross- linked hydrogel coating, or a combination thereof. The capsule may have any shape suitable for, for example, cell encapsulation. The capsule may contain one or more cells dispersed in the cross-linked hydrogel, thereby "encapsulating" the cells. Particular capsules are formed of or include one or more of the disclosed modified alginates.

Capsules can have a mean diameter of about 150 µm about 8 mm. Ccapsules can have any mean diameter from about 150 µM to about 5 cm. In particular, the capsules have a mean diameter that is greater than 1 mm, more particularly 1.5 mm or greater. In some embodiments, the capsules can be as large as about 8 mm in diameter. For example, the capsule can be in a size range of about 1 mm to 8 mm, 1 mm to 6 mm, 1 mm to 5 mm, 1 mm to 4 mm, 1 mm to 3 mm, 1 mm to 2 mm, 1 mm to 1.5 mm, 1.5 mm to 8 mm, 1.5 mm to 6 mm, 1.5 mm to 5 mm, 1.5 mm to 4 mm, 1.5 mm to 3 mm, or 1.5 mm to 2 mm.

The rate of molecules entering the capsule necessary for cell viability and the rate of therapeutic products and waste material exiting the capsule membrane can be selected by modulating capsule permeability. Capsule permeability can also be modified to limit entry of immune cells, antibodies, and cytokines into the capsule. Generally, as shown by the examples, known methods of forming hydrogel capsules can produce capsules the permeability of which limit entry of immune cells, antibodies, and cytokines into the capsule. Since different cell types have different metabolic requirements, the permeability of the membrane can be optimized based on the cell type encapsulated in the hydrogel. The diameter of the capsules is an important factor that influences both the immune response towards the cell capsules as well as the mass transport across the capsule membrane.

The growing recognition of the parameters driving fibrosis *in vivo* has been applied to the analysis of the performance of modified alginates. Intraperitoneal (IP) implantation of modified alginate capsules revealed that modified alginates may result in abnormally shaped capsules when crosslinked using conditions defined for unmodified alginates. These abnormally shaped capsules can complicate implementation and interpretation of modified alginate capsules implanted IP. In an effort to improve the capsule morphology, formulation methods for use with modified alginate microparticles were developed where modified alginates were blended with a small amount of high molecular weight alginate. Particles prepared from this mixture yielded particles with improved morphology and stability.

The unmodified alginate typically has a weight average molecular weight of about 50,000 Daltons to about 500,000 Daltons; however, unmodified alginates having molecular weights can also be used. In some embodiments, the weight average molecular weight is from about 50,000 to about 250,000 Daltons, more preferably from about 50,000 to about 150,000 Daltons. In some embodiments, the weight average molecular weight is about 100,000 Daltons.

In other embodiments, one or more additional hydrogel-forming polymers are used in combination with unmodified alginate or in place of unmodified alginate. Such polymers are known in the art. Examples include, but are not limited to, PEG, chitosan, dextran, hyaluronic acid, silk, fibrin, poly(vinyl alcohol) and poly(hydroxyl ethyl methacrylate).

In some embodiments, the alginate is made up of β-D-mannuronic acid (M) and a-L-guluronic acid (G) linked together. In some embodiments, alginate is a high guluronic acid (G) alginate. In some embodiments, the alginate is a high mannuronic acid (M) alginate. In some embodiments, the ratio of M:G is about 1. In some embodiments, the ratio of M:G is less than 1. In some embodiments, the ratio of M:G is greater than 1.

The particles prepared from a mixture of modified alginate and unmodified alginate produced more homogenous microparticle populations in terms of shape and size as evaluated by scanning electron microscopy (SEM).

In some embodiments, the hydrogel capsules can have any suitable shape. Useful shapes include spheres, sphere-like shapes, spheroids, spheroid-like shapes, ellipsoids, ellipsoid-like shapes, stadiumoids, stadiumoid-like shapes, disks, disk-like shapes, cylinders, cylinder-like shapes, rods, rod-like shapes, cubes, cube-like shapes, cuboids, cuboid-like shapes, toruses, torus-like shapes, and flat and curved surfaces. Products, devices, and surfaces that have been or will be coated can have any of these shapes or any shape suitable for the product or device.

Spheres, spheroids, and ellipsoids are shapes with curved surfaces that can be defined by rotation of circles, ellipses, or a combination around each of the three perpendicular axes, a, b, and c. For a sphere, the three axes are the same length. For oblate spheroids (also referred to as oblate ellipsoids of rotation), the length of the axes are a = b > c. For prolate spheroids (also referred to as prolate ellipsoids of rotation), the length of the axes are a = b < c. For triaxial ellipsoids (also referred to as scalene ellipsoids), the length of the axes are a > b > c. Stadiumoids are rotational shapes of stadiums. Cylinders are rotational shapes of rectangles rotated on the long axis. Disks are squashed cylinders where the diameter is greater than the height. Rods are elongated cylinders where the long axis is ten or more times the diameter.

"Sphere-like shape," "spheroid-like shape," "ellipsoid-like shape," "stadiumoid- like shape," "cylinder-like shape," "rod-like shape," "cube-like shape," "cuboid-like shape," and "torus-like shape" refers to an object having a surface that roughly forms a sphere, spheroid, ellipsoid, stadiumoid, cylinder, rod, cube, cuboid, or torus, respectively. Beyond a perfect or classical form of the shape, a sphere-like shape, spheroid-like shape, ellipsoid-like shape, stadiumoid-like shape, cylinder-like shape, rod-like shape, cube-like shape, cuboid-like shape, and torus-like shape can have waves and undulations.

Generally, a sphere-like shape is an ellipsoid (for its averaged surface) with semi-principal axes within 10% of each other. The diameter of a sphere or sphere-like shape is the average diameter, such as the average of the semi-principal axes. Generally, a spheroid-like shape is an ellipsoid (for its averaged surface) with semi-principal axes within 100% of each other. The diameter of a spheroid or spheroid-like shape is the average diameter, such as the average of the semi-principal axes. Generally, an ellipsoid-like shape is an ellipsoid (for its averaged surface) with semi-principal axes within 100% of each other. The diameter of an ellipsoid or ellipsoid-like shape is the average diameter, such as the average of the semi-principal axes. Generally, a stadiumoid-like shape is a stadiumoid (for its averaged surface) with semi-principal axes of the ends within 20% of each other. The diameter of a stadiumoid or stadiumoid-like shape is the average diameter, such as the average of the semi-principal axes. Alternatively, the size of a stadiumoid or stadiumoid-like shape can be given as the average of the long axis. Generally, a cylinder-like shape is a cylinder (for its averaged surface) with semi- principal axes within 20% of each other. The diameter of a cylinder or cylinder-like shape is the average diameter, such as the average of the semi-principal axes.

Alternatively, the size of a cylinder or cylinder-like shape can be given as the average of the long axis. Generally, a rod-like shape is a rod (for its averaged surface) with semi-principal axes within 10% of each other. The diameter of a rod or rod-like shape is the average diameter, such as the average of the semi-principal axes. Alternatively, the size of a rod or rod-like shape can be given as the average of the long axis. Generally, a cubelike shape is a cube (for its averaged surface) with sides within 10% of each other. The diameter of a cube or cubelike shape is the average side length. Generally, a cuboid-like shape is a cuboid (for its averaged surface) with matching sides within 10% of each other. The diameter of a cuboid or cuboid-like shape is the average side length.

Generally, a torus-like shape is a torus (for its averaged surface) with semi-principal axes within 10% of each other. The diameter of a torus or torus-like shape is the average diameter, such as the average of the semi-principal axes. Alternatively, the size of a torus or torus-like shape can be given as the diameter across the ring.

"Flat side" refers to a contiguous area of more than 5% of a surface that has a curvature of 0. "Sharp angle" refers to a location on a surface across which the tangent to the surface changes by more than 10% over a distance of 2% or less of the circumference of the surface. Edges, corners, grooves, and ridges in a surface are all forms of sharp angles.

Particular capsules can be made of biocompatible materials, have a diameter of at least 1 mm and less than 10 mm, has a spheroid-like shape, and have one or more of the additional characteristics: surface pores of the capsules greater than 0 nm and less than 10 µm; surface of the capsules neutral or hydrophilic; curvature of the surface of the capsules at least 0.2 and is not greater than 2 on all points of the surface; and surface of the capsules lacking flat sides, sharp angles, grooves, or ridges. Generally, the capsules elicit less of a fibrotic reaction after implantation than the same capsules lacking one or more of these characteristics that are present on the capsules.

In some embodiments not forming part of the invention, the capsule s are provided as a preparation and at least 30%, 35%, 40%, 45%, 50%, 55%, 60%, 65%, 70%, 75%, 80%, 85%, 90%, 95% or 100% of the capsules in the preparation have a shape characteristic described herein, e.g., have a spheroid-like shape, or have a curvature of the surface of at least 0.2 to 2.0 on all points of the surface.

In some embodiments, the hydrogel capsules have a mean diameter that is greater than 1 mm, particularly 1.5 mm or greater. In some embodiments, the hydrogel capsules can be as large as 8 mm in diameter. For example, the hydrogel capsules is in a size range of 1 mm to 8 mm, 1 mm to 6 mm, 1 mm to 5 mm, 1 mm to 4 mm, 1 mm to 3 mm, 1 mm to 2 mm, 1 mm to 1.5 mm, 1.5 mm to 8 mm, 1.5 mm to 6 mm, 1.5 mm to 5 mm, 1.5 mm to 4 mm, 1.5 mm to 3 mm, 1.5 mm to 2 mm, 2 mm to 8 mm, 2 mm to 7 mm, 2 mm to 6 mm, 2 mm to 5 mm, 2 mm to 4 mm, 2 mm to 3 mm, 2.5 mm to 8 mm, 2.5 mm to 7 mm, 2.5 mm to 6 mm, 2.5 mm to 5 mm, 2.5 mm to 4 mm, 2.5 mm to 3 mm, 3 mm to 8 mm, 3 mm to 7 mm, 3 mm to 6 mm, 3 mm to 5 mm, 3 mm to 4 mm, 3.5 mm to 8 mm, 3.5 mm to 7 mm, 3.5 mm to 6 mm, 3.5 mm to 5 mm, 3.5 mm to 4 mm, 4 mm to 8 mm, 4 mm to 7 mm, 4 mm to 6 mm, 4 mm to 5 mm, 4.5 mm to 8 mm, 4.5 mm to 7 mm, 4.5 mm to 6 mm, 4.5 mm to 5 mm, 5 mm to 8 mm, 5 mm to 7 mm, 5 mm to 6 mm, 5.5 mm to 8 mm, 5.5 mm to 7 mm, 5.5 mm to 6 mm, 6 mm to 8 mm, 6 mm to 7 mm, 6.5 mm to 8 mm, 6.5 mm to 7 mm, 7 mm to 8 mm, or 7.5 mm to 8 mm. In some embodiments, the capsule has a mean diameter or size between 1 mm to 8 mm. In some embodiments, the capsule has a mean diameter or size between 1 mm to 4 mm. In some embodiments, the capsule has a mean diameter or size between 1 mm to 2 mm. In some embodiments, the capsules are provided as a preparation and at least 30%, 35%, 40%, 45%, 50%, 55%, 60%, 65%, 70%, 75%, 80%, 85%, 90%, 95% or 100% of the hydrogel capsules in the preparation have a diameter in a size range described herein.

Suitable alginates and modified alginates are described in US20160030360A1, US20170239397A1, WO2012167223, and WO2017075631.

### IV. Therapeutic Applications

Therapeutic applications include treatments of cancer, such as where immunomodulatory proteins can be produced by therapeutic cells encapsulated by the microdevice. Therapeutic production of blood factors, such as FVIII or FIX, can be used to mitigate hemophilia. These proteins can be produced by therapeutic cells encapsulated in the microdevice.

A particular embodiment of the present disclosure not forming part of the invention may involve treating diabetes, a global epidemic afflicting more than 285 million people worldwide (Veiseh *et al.,* 2015). While the current standard of care for those suffering from type I diabetes (TID) consists of a rigorous regimen of blood glucose monitoring together with daily administrations of exogenous insulin and diabetic diet, these individuals still are challenged with untoward side effects due to complications of the disease in part the result of daily compliance issues (Veiseh *et al.,* 2015; Pickup, 2012). Additionally, the process by which the beta cells of the pancreatic islets of Langerhans secrete insulin in response to blood glucose fluctuations is imperfectly simulated by periodic insulin injections (Veiseh *et al.,* 2015; Robertson, 2004). The 'holy grail' of diabetes treatment research thus is achieving insulin independence for TID patients through transplantation of exogenous donor islet tissue or cells. A corollary of this goal is to develop methods that obviate the need for life-long immunosuppression, required as the result of allo- or xenogeneic transplantation and itself writhe with complications, in order to fully implement this approach in clinical practice (Hirshberg, 2007; Pagliuca *et al.,* 2014).

Recently, the *in vitro* differentiation of human pluripotent stem cells (hPSCs) into functional pancreatic beta cells was reported, providing a path to produce an unlimited supply of human insulin-producing tissue for the first time (Pagliuca *et al.,* 2014; Vegas *et al.,* 2016a). Methods that relieve the need for lifelong immunosuppression are essential to enabling the broad clinical implementation of this new tissue source (Hirshberg, 2007; Shapiro, 2012; Vogel, 2014). Cell or tissue encapsulation can in theory overcome the need for immunosuppression by protecting transplants from host immune detection and subsequent graft rejection (Dolgin, 2014; Jacobs-Tulleneers-Thevissen, 2013).

### V. Biosensors

Another aspect of this disclosure not forming part of the invention relates to biosensor. Cells, both eukaryotic and prokaryotic, have biological systems for binding and recognition of many analytes. Membrane proteins relay information through the cell and engineered cellular biosensors can provide feedback or analytical response, detectable through medical imaging and/or in the blood.

A variety of non-cellular biosensors, including continuous glucose monitors, rely on consistent access to the bloodstream. Vascularization of biosensors can yield higher temporal resolution and higher signal levels for analytes. Long-term acceptance of biosensors, via anti-fibrotic materials, can provide long-term effectiveness.

### VI. Exemplary Device Architectures, Components and Variations Thereof

Embodiments of the present invention include a modular device based on advanced 3D bioprinting for vascularization and immunoisolation of therapeutic cells, biosensors, and other implants that may benefit from vascularization. Referring now to FIG. 1, an exemplary embodiment illustrates a degradable polymer encasing a non-degradable polymer. The inventors have developed a simple planar vascular architecture within a 3D printed hydrogel that has an empty cavity to seed therapeutic cells encapsulated in immunoisolating alginate hydrogels. This approach allows independent control over vascular architecture, immunoisolation, and immunomodulation (prevention of fibrotic response). The inventors' 3D printed chamber is biocompatible and can be tuned for biodegradation through discrete optimization of the hydrogel formulation (combined poly (ethylene glycol) diacryate and MMP degradable peptide-methacrylate). The biodegradable chamber is a provisional matrix that promotes patterned anastomosis and host integration, while also supporting the immunoisolated therapeutic cells within their non-degradable alginate during the early stages of implantation and remodeling *in vivo.* This scalable design, which could be stacked or designed into vascular multilayers, could eventually allow for the restoration of pancreatic function, provision of clotting factors or enzymes in patients who suffer from genetic disorders, and effective vascularization of biosensors.

### A. Advanced 3D Bioprinting of Biocompatible Hydrogels

Though previous work in bioengineering has sought to provide mass transport to engineered tissues by incorporating various cell types (Finkenzeller *et al.,* 2006; Lovett *et al.,* 2009; Wenger *et al.,* 2005), growth factors, or microfluidic networks (Andrae *et al.,* 2008; Bauer *et al.,* 2005), most of these techniques lack the capacity to generate vascularized tissues with complex hierarchical vascular structure, while also providing quantitative non-invasive metrics over the cellular environment. Three-dimensional (3D) printing provides significant promise to the field of 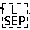 tissue engineering because the 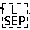 technology provides researchers with control over every (x, y, z) coordinate within the print volume.

Here, the inventors introduce a new approach in the fabrication of biocompatible hydrogels: the automated fabrication of vascularized hydrogels with a low- cost stereolithography system the inventors have developed. The inventors demonstrate, for the first time, the ability to generate highly defined vascularized structures (50 µm x, y, z feature resolution) for the studies described in this proposal, typically with vascular channels on the order of 0.5-1 mm in diameter and the facile generation of tens of scaffolds per day. Here, the inventors seek to engineer living tissue and maintain control over the position of therapeutic cells and the composition of the surrounding extracellular matrix (ECM). The inventors will utilize photopolymerizable synthetic hydrogels with more than 80% water content based on poly(ethylene glycol) diacrylate (PEGDA) and MMP sensitive peptides for the studies described here. PEGDA is a hydrophilic and biocompatible material which exhibits ease of functionalization with acrylate-TMTD small molecule to modulate host immune responses (39, 40).

### B. Advanced Vascular Topologies to Support Therapeutic Cells

Perfusable vessel architectures will stabilize therapeutic cell homeostasis and function in 3D gels, and that convective flow will permit high density therapeutic cell culture to maximize the therapeutic potential of such engineered tissues. The inventors' hydrogel materials are synthesized in-house and fabricated using a custom 3D bioprinting by stereolithography system, and the inventors can now generate a diversity of 3D vascular networks within *ex vivo* hydrogels.

To improve convection and diffusion to entrapped therapeutic cells the inventors will further develop this hydrogel chamber with entrapped therapeutic cells arranged precisely next to the vasculature. The inventors' 3D printing stereolithography system enables the construction of empty cavities for seeding of a secondary alginate hydrogel with entrapped therapeutic cells, and importantly allows for the fabrication of an underlying perfusable vasculature which the inventors hypothesize will boost nutrient and oxygen delivery to therapeutic cells and thereby improve their function *in vivo.*

Hydrogel chambers can also be fabricated with mixtures of PEGDA:PeptideMA that support the attachment of human endothelial cells to the vessel walls, with an optimal 10 wt% peptideMA, 3 wt% PEGDA (3.4kDa) formulation (FIG. 8E) while maintaining vascular patency. Endothelial cells are injected into the vascular network at 25e6 cells/mL in media and the gels are rotated about the vascular axis in 180° increments every 15 minutes over 4 hours to allow endothelial cells to attach to all vascular surfaces. Gels will be cultured overnight and up to 7 days to allow for stable endothelial monolayers to form.

The endothelial monolayer can be characterized for healthy cobblestone morphology using confocal microscopy with stains for VE-cadherin, ZO-1, and CD31. Barrier function of the endothelium can be assessed with histamine and thrombin as previously described (Chrobak *et al.,* 2006). The inventors have previously shown that patterned endothelial networks can direct anastomosis with host vasculature and form hybrid human/mouse vessels. Here the inventors will further characterize the degradation rate of chambers (with 0.4 mg/mL collagenase) made from mixtures of PEGDA:peptideMA which the inventors hypothesize will promote vascular development around the alginate-encapsulated primary therapeutic cells.

### C. Integration of Therapeutic Cells Encapsulated within TMTD Alginate Gels into Vascularizing Devices

A typical cell encapsulation entails the following steps: therapeutic cell clusters are washed with Ca-free Krebs-Henseleit buffer (4.7 mM KCl, 25 mM HEPES, 1.2 mM KH₂PO₄, 1.2 mM MgSO^{¬4}×7H2O, 135 mM NaCl, pH≈7.4, ≈290 mOsm). After washing, therapeutic cells are centrifuged again and all supernatant is aspirated. The therapeutic cell pellet is then re-suspended in TMTD alginate solutions which will be synthesized as previously described (Vegas *et al.,* 2016a) at various cluster densities. The solution is poured into the chamber of the degradable 3D printed hydrogel described above and gels are crosslinked using BaCl^{¬2} gelling solution. Any divalent cations can be used for crosslinking, including Ca²⁺, Sr²⁺ solutions. Immediately after crosslinking, the devices are incubated in cell culture media until just prior to transplantation.

### D. In Vivo Performance

The inventors have previously shown that patterned endothelial cells can anastomose with host vasculature and form hybrid human/mouse vessels after 14 days (Baranski *et al.,* 2013). Based on the inventors' prior work the inventors anticipate that anastomosis of devices should occur in endothelialized devices by two weeks and after 4 weeks the inventors expect complete degradation of PEGDA:PeptideMA gel networks.

### VII. Alternative Applications

### A. Use of Devices for Therapeutic Cells and/or Biosensors

Biosensors, including continuous glucose monitors, as well as therapeutic cells, would benefit from a strong vascular supply. Referring now to FIG. 2, therapeutic cells, biosensors, or other implants can be loaded into the non-degradable portion of the device. This device can be used to improve vascularization for any type of implant, resulting in better data acquisition and greater long-term performance for biosensors, and superior viability and functionality for therapeutic cells.

### B. Sustained Release of Compounds

Sustained release of oxygen may improve long term therapeutic cell viability. Referring now to FIG. 3, methods and materials resulting in controlled release of oxygen and/or pro-angiogenic compounds can be loaded into the degradable and/or non-degradable portions of the device. Oxygen-releasing compounds, such as CaO₂, and pro-angiogenic compounds like VEGF can promote vascularization of large, complex scaffolds in the degradable portions of the device. CaO^{¬2} is an oxygen-generating compound. Sustained release of pro-angiogenic compounds may improve vascularization of the device. Crystallized VEGF and cells constitutively secreting VEGF are possible methods to provide sustained release of these compounds. Methods and materials for sustained release of compounds can be incorporated into the degradable and/or non-degradable portions of the device.

### C. Modifying Degradation Profile and Stiffness of Degradable Portion

Gel degradation profile and stiffness can be controlled by the following parameters: PEGDA of different molecular weight *(e.g.,* 6 kDa), can also be used to modify degradation profile. Referring now to FIG. 4, the degradable portion can be modified, using polymer chains of various lengths. This will allow for different biodegradation profiles. Larger or more complex structures may need finely tuned degradation profiles for successful *in vivo* effect. Modifying the amounts of PEGDA, GelMA, photoinitiator, and photoabsorber (FIG. 9). Slower degradation may be needed for devices with very large, complex vascular scaffolds. Intensity and duration of UV crosslinking can affect gel degradation and stiffness.

### D. Anchors of Various Shapes to Link Degradable and Non-Degradable Portions of the Device

With increasingly complex structures, anchors provide a linkage between the degradable and non-degradable portions. This permits surgical manipulation and implantation without structural failure of the device. Referring now to FIG. 6, anchors can hold together non-degradable and degradable portions of the device. Complex structures and large nondegradable portions may need physical linkages to maintain their integrity for surgical manipulation and implantation. Anchors successfully link both segments together.

### E. Stackable Nature of Device

The device has a stackable nature, allowing for layered vasculature. Larger therapeutic constructs can be developed simply by stacking multiple devices. Referring now to FIGS. 8A-F, 3D printed PEGDA hydrogel chambers are illustrated for rapid vascularization of therapeutic cell constructs. FIG. 8A shows the printed chamber accommodates primary islets (green) suspended in alginate (blue) and has an underlying patterned vascular network (red). Chambers are stackable for scale-up considerations. FIG. 8B illustrates 3D printed chamber demonstrate perfusable vasculature (red), and hydrogel anchors that will physically trap the islet-containing alginate hydrogel. Scale bar = 1 mm. (unpublished results). FIG. 8C shows magnified view of highlighted region from FIG. 8B showing hydrogel anchors and underlying perfusable vasculature (red). Scale Bar = 1 mm. (unpublished results). FIG. 8(D) shows a 250 µm cross-section view demonstrating the alginate seeding volume (blue) and underlying vasculature (red). Scale Bar = 1 mm. (unpublished results). FIG. 8E illustrates endothelial cells injected into the vasculature quickly line the vessels as seen in top-view (left) and form hollow lumens (confocal virtual cross-section, right). (unpublished results). In FIG. 8F, the inventors have previously shown that patterned endothelial cells can anastomose with host vasculature and form hybrid human/mouse vessels after 14 days (Baranski *et al.,* 2013).

### F. Modifying Immunomodulatory Properties and Inducible Degradation of the Degradable Portion

Hydrogel precursors can be varied, as shown in FIG. 9. The amount and/or type of anti-fibrotic small molecule can be modified for different levels of immunomodulatory activity. Cell-adhesive peptides can be modified for improvement of endothelial cell adhesion to the vascular scaffolds of the hydrogel. The peptide in "MMP-sensitive PEG-diacrylate" can be chosen to allow other inducible degradation mechanisms: oxide-driven degradation, pH-driven degradation, ultrasound-driven degradation, heat-driven degradation.

### G. Advanced 3D Bioprinting of Biocompatible Hydrogels

Here, the inventors introduce a novel approach in the fabrication of biocompatible hydrogels: the automated fabrication of vascularized hydrogels with a low- cost stereolithography system the inventors have developed. The inventors demonstrate, for the first time, the ability to generate highly defined vascularized structures (50 µm x, y, z feature resolution) for the studies described in this proposal, typically with vascular channels on the order of 0.5-1 mm in diameter and the facile generation of tens of scaffolds per day. Here, the inventors seek to engineer living tissue and maintain control over the position of beta cells and the composition of the surrounding extracellular matrix (ECM). The inventors will utilize photopolymerizable synthetic hydrogels with more than 80% water content based on poly(ethylene glycol) diacrylate (PEGDA) and MMP sensitive peptides for the studies described here. PEGDA is a hydrophilic, biocompatible, FDA-approved material which has seen broad utility in the probing of fundamental cell-matrix interactions and exhibits ease of functionalization with acrylate-TMTD small molecule to modulate host immune responses (Burdick and Anseth, 2002; Lutolf *et al.,* 2003).

### H. Advanced Vascular Topologies to Support 3D Beta Cell Culture

To improve convection and diffusion to entrapped beta cells the inventors will further develop this hydrogel chamber with entrapped beta cells arranged precisely next to the vasculature. The inventors' 3D printing by stereolithography system enables the construction of empty cavities for seeding of a secondary alginate hydrogel with entrapped primary islets, and importantly allows for the fabrication of an underlying perfusable vasculature which the inventors hypothesize will boost nutrient and oxygen delivery to islets and thereby improve their function *in vivo.* Hydrogel chambers can also be fabricated with mixtures of PEGDA:PeptideMA that support the attachment of human endothelial cells to the vessel walls, with an optimal 10 wt% peptideMA/3 wt% PEGDA (3.4kDa) formulation (FIG. 8E) while maintaining vascular patency. Endothelial cells are injected into the vascular network at 25e6 cells/mL in media and the gels are rotated about the vascular axis in 180° increments every 15 minutes over 4 hours to allow endothelial cells to attach to all vascular surfaces. Gels will be cultured overnight and up to 7 days to allow for stable endothelial monolayers to form. The inventors will characterize the endothelial monolayer for healthy cobblestone morphology using confocal microscopy with stains for VE-cadherin, ZO-1, and CD31. Barrier function of the endothelium will be assessed with histamine and thrombin as previously described (Chrobak *et al.,* 2006). The inventors have previously shown that patterned endothelial networks can direct anastomosis with host vasculature and form hybrid human/mouse vessels. The inventors hypothesize the patterned vasculature will support host engraftment. Here the inventors will further characterize the degradation rate of chambers (with 0.4 mg/mL collagenase) made from mixtures of PEGDA:peptideMA which the inventors hypothesize will promote vascular development around the alginate-encapsulated primary islets.

### I. Methods for Integration of Islets Encapsulated within TMTD Alginate Gels into Vascularizing Devices

A typical cell encapsulation entails the following steps: syngeneic mouse islet clusters purchased from CellTrans Inc. Chicago, IL are centrifuged at 1,400 rpm for 1 min and washed with Ca-free Krebs-Henseleit buffer (4.7 mM KCl, 25 mM HEPES, 1.2 mM KH₂PO₄, 1.2 mM MgSO₄²⁻ × 7H₂O, 135 mM NaCl, pH≈7.4, ≈290 mOsm). After washing, islet cells are centrifuged again and all supernatant is aspirated. The islets pellet is then re-suspended in TMTD alginate solutions which will be synthesized as previously described (Vegas *et al.,* 2016a) at various cluster densities of 500 clusters per 0.5 mL alginate solution. The solution is poured into molds and gels are crosslinked using a BaCl₂ gelling solution. Immediately after crosslinking, the encapsulated islet clusters are washed 4 times with 50 mL of CMRL 1066 modified media and cultured overnight in a spinner flask at 37 °C, which would be just prior to transplantation. Due to an inevitable loss of islet clusters during the encapsulation process, the total number of encapsulated clusters is recounted post-encapsulation.

After encapsulation, cell viability and function will be characterized. Cell viability is measured using a fluorescence microscopy-based assay where cells are stained with a combination of fluorescein diacetate (live) and propidium iodide (dead) stains. The percentage of cell viability will be determined by sampling 100 islet clusters and analyzing the ratio of live vs. dead cells. The inventors will additionally perform *in vitro* Glucose stimulated insulin secretion assays (Vegas *et al.,* 2016a).

### VIII. Examples

The following examples are included to demonstrate preferred embodiments of the disclosure. It should be appreciated by those of skill in the art that the techniques disclosed in the examples which follow represent techniques discovered by the inventor to function well in the practice of the disclosure, and thus can be considered to constitute preferred modes for its practice..

### Prophetic Example 1

***In vivo* biocompatibility and vascular function assessment of vascularizing device.** The inventors have previously shown that patterned endothelial cells can anastomose with host vasculature and form hybrid human/mouse vessels after 14 days (Baranski *et al.,* 2013). The inventors will assess the biocompatibility and vascularization of hydrogel chambers described above using healthy non-diabetic C57/BL6 nu/nu mice T cell-deficient mice. They will also evaluate the biocompatibility of gels with immunomodulatory chemistries. For these studies the inventors will use non-diabetic C57/BL6 nude mice (Jackson Laboratories, Bar Harbor Maine). Using three iterations of devices - TMTD alginate alone, TMTD alginate in chambered gel with hollow vessel, and TMTD alginate chambered gel with endothelialized vessels - the inventors will test *in vivo* for two and 4 weeks.

The inventors will validate *in vivo* biocompatibility and vascular function assessment of hydrogels projection 3D printed hydrogel chambers with perfusable vessel networks will support high density 3D beta cell culture. Three iterations of devices will be tested *in vivo* using healthy C57/BL6 nu/nu mice, TMTD alginate alone (N=5 mice per time point) (left) TMTD alginate in chambered gel, with hollow vessels (N=5 mice per time point) (center), and TMTD alginate in chambered gel with endothelialized vessels (N=5 mice per time point) (right). Two retrieval time points - 2 weeks and 4 weeks - will be used for assessment.

**Testing empty (no islets) vascularizing device *in vivo* using healthy C57/BL6 nu/nu mice.** Device iterations outlined in FIGS. 8A-F will be implanted into intraperitoneal site of C57/BL6 nu/nu mice as previously described (Vegas *et al.,* 2016a). In this study, TMTD alginate alone is incorporated as control non-vascularization system and TMTD alginate in chambered gel, with hollow vessels is second control system that does not promote anastomosis with host endothelial cells. Each mouse will receive a single device implant, N=5 mice per device iteration and retrieval time point. Mice will be sacrificed at 2 and 4-week post implantation. Based on their prior work, the inventors anticipate that anastomosis of devices should occur in endothelialized devices by two weeks and after 4 weeks the inventors expect complete degradation of PEGDA:PeptideMA gel networks. At these defined 2 and 4-week time points, mice will be sacrificed and devices will be carefully explanted through microdissection to ensure that vascular network around remaining alginate gel blocks are not disrupted. The retrieved tissue and gels will be preserved with paraformaldehyde and further processed using standard histological processing techniques.

***Ex vivo* analysis of biocompatibility and vascular network integrity around empty (no islets) vascularizing device via histology.** The following assessments will then be performed on retrieved devices to assess vascularization and biocompatibility: 1) fibrotic overgrowth (FO) quantification via phase contrast microscopy and confocal fluorescent microscopy; 2) FACS analysis will assess the quantity of inflammatory cells (CD68 staining macrophages, Ly6Gr+ staining neutrophils, smooth muscle actin staining myofibroblasts, and CD31 endothelial cells) in single cell suspensions derived from: i) IP lavage of the retrieval solution; ii) dissociated cells from the surface of retrieved devices; and 3) histological analysis of devices to evaluate thickness of FO and state of inflammation of the tissue and vascularization. Tissue samples will be examined for evidence of fibrosis and rejection. Mason's trichrome staining will be used to monitor collagen deposition associated with fibrosis. Additionally, sections will be immunostained for fibroblasts, neutrophils, and macrophages, and endothelial cells using methods the inventors have previously developed (Vegas *et al.,* 2016a; 2016b; Veiseh *et al.,* 2015a).

Vascularization density around the device will be verified through histology and the inventors expect that >90% of printed channels surface area should be colonized by host endothelial cells. The process by which endothelial cells anastomosis with host vasculature takes 1-2 weeks and required the matrix PEGDA:PeptideMA to remain intact while anastomosis occurs. Based on prior experience the ratio of PEGDA:PeptideMA should last for two weeks; however, if the gel degrades too slowly then the inventors can simply alter the ratios of these two monomers to achieve the desired degradation rate. Increasing the ratio of PEGDA:PeptideMA will reduce the degradation rate and reducing the ratio enhances the degradation rate. For example, chambers created from PEGDA-alone would be non-degradable *in vivo.*

***In vivo* testing of islet cell loaded vascular networks in streptozotocin-induced (STZ-induced).** Using a syngeneic transplantation model, the inventors will evaluate the efficacy of islet loaded TMTD alginate gels into vascularizing device in STZ treated C57/BL6 nu/nu mice. Vascularization around the TMTD gels should facilitate improved cell viability and enhanced glucose stimulated insulin secretion kinetics.

**Seeding vascular scaffold with endothelial cells.** The vascular scaffold can be seeded with endothelial cells, which secrete VEGF, and can improve the *in vivo* responses of the device. Referring now to FIG. 7, endothelial cells can be used to line the vascular scaffold. Endothelial cells secrete VEGF to enhance vascularization of the device. Different sources of endothelial cells (off-the-shelf *versus* patient-derived) can be used, or no cells at all. This may result in different *in vivo* engraftment of the device. Since the device can be seeded with endothelial cells of any origin, patient-derived endothelial cells can be used for greatest safety.

Referring now to FIG. 10, two iterations of devices can be tested *in vivo*: a device with hollow vessels (left), and a device with endothelialized vessels (right). The iterations can be used to validate *in vivo* functionality of islet loaded TMTD alginate chambered with patterned vessel networks in diabetic nude mice. Two iterations of devices will be tested *in vivo* using STZ treated C57/BL6 nu/nu mice, syngeneic islets loaded TMTD alginate in chambered gel, with hollow vessels (N=5 mice) (left), and syngeneic islet loaded TMTD alginate in chambered gel with endothelialized vessels (N=5 mice per time point) (right).

The inventors will encapsulate syngeneic islet cells into TMTD alginate gels into vascularizing device (500 per device) and evaluate cell, morphology, viability, glucose responsive insulin secretion. The functionality and viability of the islets encapsulated within the gel assembly will be monitored using a combination of Live-Dead cell viability assays and test of insulin production in response to glucose challenges.

Transplant syngeneic islet will be loaded in vascular networks into STZ-B6 nu/nu mice and blood glucose levels monitored daily for up to 100 days. Six diabetic nude mice will be implanted IP with syngeneic islets loaded TMTD alginate in chambered gel, with hollow vessels and six diabetic nude mice will be implanted IP syngeneic islet loaded TMTD alginate in chambered gel with endothelialized vessels. The mice will be monitored for a period of 100 days. Blood glucose levels will be measured daily. At 14, 28, and 100 days post-transplantation an oral glucose tolerance test (OGTT) will be performed on the animals and the *in vivo* kinetics for both blood glucose correction and c-peptide secretion will be monitored. A cure will be determined as blood glucose levels less than 220 mg/dL one-week post implant and for the entirety of the 100-day monitoring period. Efficacy of the encapsulation will then be determined based on long-term viability and glucose responsive insulin secretion kinetics. Upon termination of *in vivo* normoglycemia experiments, transplants will the received and histopathological analysis will be performed. Potential causes for transplant failure will be determined, and islet viability (live/dead assays), functionality (*ex vivo* glucose challenge assays), and biocompatibility (immunostaining to characterize potential immune response and fibrosis) with be examined.

**Efficacy of the TMTD encapsulated islets in vascularizing devices will be determined in the diabetic nude mouse model.** Prior studies have shown that 500 islets per mouse is sufficient to achieve normoglycemic correction in STZ STZ-B6 nu/nu mice; however, if after 1-week diabetic mouse glucose correction is not achieved the inventors will transplant additional mice with devices that have been loaded with higher quantities of islets (1000 and 2000).

The inventors will develop and validate next-generation 3D culture of beta cells within 3D gels containing perfusable vasculature. The inventors hypothesize that perfusable vessel architectures will stabilize beta cell homeostasis and function in 3D gels, and that convective flow will permit high density beta cell culture to maximize the therapeutic potential of such engineered tissues. The hydrogel materials are synthesized in-house and fabricated using custom 3D bioprinting by stereolithography system, and the inventors can now generate a diversity of 3D vascular networks within *ex vivo* hydrogels. The inventors will screen a diversity of vascular topographies to identify lead formulations which support the highest density of cell viability and functionality *in vivo,* as shown in FIGS. 11A-F.

**Variations in vascular topology.** Vascular topology may have a significant effect for high density therapeutic cell culture. Referring now to FIG. 5, the 3D vasculature can be printed with many possible topologies. The topology of the 3D vasculature may have an important impact on the long-term viability and performance of encapsulated therapeutic cells and biosensors. The 3D printing approach disclosed herein allows for rapid synthesis of highly diverse and complex vascular structures. The 3D printing approach allows for development of highly complex vascular topologies, as shown in FIGS. 11A-F.

**Modifying alginate antifibrotic properties and stiffness.** Alginate used in the non-degradable portion can be modified in a variety of ways: antifibrotic small molecule of choice can be conjugated to the alginate with a dosage of choice. In addition, alginate stiffness can be modified by modifying the ratio of modified alginate and SLG100, or alteration of crosslinking buffer (use of Ca²⁺ or Sr²⁺). This can result in improved biomechanical response at different implant sites.

FIGS. 11A-F illustrate vascular designs to explore vascular efficiency in perfusable hydrogels. 3D vascular geometries will be computationally generated using space-filling algorithms with iterative complexity. FIG. 11A shows sample vascular topology growth algorithm in 2D form for clarity. FIG. 11B illustrates the inventors have also identified computational space filling algorithms as good candidates for building perfusable vascular topologies. In FIG. 11C, 3D vascular-inspired architectures that follow Murray's Law for daughter branch diameter scaling can be converted into topologies for 3D printing by Boolean subtraction from a bounding volume. FIGS. 11D-F present vascular topologies fabricated in perfusable hydrogel shown with macro photography or by µCT analysis, as labeled. The inventors further demonstrate that 3D vascular networks can allow for interpenetrating networks shown in (red, blue) in (FIGS. 11D-E).

### Example 2 - Materials and Methods for Example 3

**GelMA, PEGDA, and LAP Synthesis.** LAP was prepared as previously described using equimolar amounts of dimethyl phenylphosphinite and 2,3,6-trimethylbenzoyl chloride under argon overnight at room temperature (Fairbanks *et al.,* 2009). In the following step, poly(ethylene glycol) diacrylate (PEGDA), 3.4 kDa, 6 kDa, 10 kDa, 20 kDa, and 35 kDa, was synthesized by reaction between poly(ethylene glycol) acryloyl chloride (in presence of triethylamine overnight (FIG. 16A) (Miller *et al.,* 2010). Gelatin methacrylate (GelMA) was synthesized by the dropwise addition of methacrylic anhydride (0.1 mL per 1.0 g gelatin) to the mixture of gelatin (Type A, bloom 300) dissolved in 100 mM carbonate/bicarbonate buffer (pH = 9.5) at 50 °C for 4 hours (Shirahama *et al.,* 2016).

**Hydrogel fabrication with projection stereolithography.** Monolithic hydrogels were fabricated with projection stereolithography (pSLA) which involves sequential crosslinking of photo-sensitive polymers in a layer-by-layer fashion via the projection of photomasks to create a 3D volume (Arcaute,K. Ann. Biomed. Eng., 34: 1429-41, 2006). The inventors' lab has developed a custom pSLA 3D printer consisting of a commercial digital light processing (DLP) projector, a stepper motor and axis for the z-stage, a RepRap Arduino Mega Board (RAMBo), and 3D printed housing components which were fabricated using an Ultimaker 2 (Ultimaker, Netherlands) from consumer-grade poly(lactic acid) (PLA) plastic filament. Full set-up is described previously (Grigoryan, B. Science 364(6439): 458-464, 2019). The hydrogels are fabricated layer-by-layer by attaching to the glass slide surface on the z-stage which is immersed into a poly(dimtheylsiloxane) (PDMS) coated (6:1 based:catalyst) vat of prepolymer solution. The XY build size is 64x40 mm and has an XYZ resolution of 50 µm determined by the projector optics (XY) and photoabsorbers used (Z). The light source is 405 nm and is attached to a computer so that there is control over the motor, heating element, and projection of photomasks.

Pre-hydrogel mixtures were prepared containing 3.25 wt% 3.4 kDa PEGDA, 10 wt% GelMA, 17 mM LAP, 10% glycerol, and 2.255 mM tartrazine. Tartrazine (yellow food coloring FD&C Yellow 5, E102) is used as a photoabsorber which the inventors have previously established serves as a low toxic dye that washes out easily to result in a transparent hydrogel for facile imaging (Grigoryan, B. Science 2019). Should cells be encapsulated in the bulk of the hydrogel, the desired concentration and co-culture ratio of cells are included and resuspended in the pre-hydrogel solution (5 x 10⁶ endothelial cells to 1 x 10⁶ mesenchymal stem cells). The pre-hydrogel mixture is loaded into the PDMS coated dish where the build platform is lowered to the first layer position. Creation Workshop software (world-wide-web at envisionlabs.net/) enables control over the printer with GCode commands sent for vertical movement of the z-stage, projection of the photomask sequence, and controlled heating of the heating element. Because GelMA is sensitive to thermal gelation, the vat is maintained at 37 °C to maintain an aqueous pre-hydrogel mixture until crosslinked. Each layer height was set to 50 µm with exposure times adapted for each batch of GelMA (typically between 10-15 seconds per layer) at 19.5 mW/cm² power output. After the printed construct is complete, the 3D hydrogel is removed from the glass slide by applying a razor between the hydrogel and the slide and allowed to swell overnight in PBS with multiple washes.

**Cell Culture and endothelialization for long-term perfusion.** Human umbilical vein endothelial cells (HUVEC) were cultured in VascuLife Medium and supplements (LifeLine Technologies, Frederick, MD) at 37 °C and 5% CO₂. All HUVECs used in endothelialized channels were between passages 4-8. The hydrogel was placed inside a custom PLA gasket to serve as a perfusion chamber closely fitting the gel as well as the inlet and outlet 18-gauge needle tips. Before placing the hydrogel, the chamber was bonded to a glass slide and sterilized with ethanol for 15 minutes and dried thoroughly. Once the hydrogel was placed inside the chamber and the inlet and outlet were fitted with the 18-gauge needles, the top of the chamber was sealed with another glass slide. This set-up allowed for fluidically sealed perfusion through the lumenized architecture.

HUVECs were loaded into the lumens of the hydrogel's microchannels at a concentration of 30e6 cells/mL with a 1 mL Normject syringe connected to an 18-gauge flexible needle at the inlet of the channel. The outlet was likewise catheterized with an 18-gauge flexible needle to fluidically seal the channel. Following suspension injection, cells were allowed to adhere to the bottom cylindrical surface for 15 min. Every 15 min thereafter for 6 hours, the gel was rotated by 90 degrees to establish a cylindrical coating of adhered HUVECs. Any unattached cells were washed away with fresh media during the perfusion set-up.

Perfusion culture was maintained at 5 µl/min using the high precision, multi-channel peristaltic pump, ISMATEC (Cole Parmer). Tubing was connected with luer-lock fittings from an IV media bag through the peristaltic tubing to the inlet of the perfusion chamber. Two 0.22 µm filters were placed in line with the flow before reaching the hydrogel - one placed right before the inlet, the second placed at a y-junction stop-cock between the media bag and the peristaltic pump. During long-term perfusion culture, media was added to the bag through this junction as needed. All bubbles were evacuated by pre-wetting the tubing all the way through to the inlet before connecting to the perfusion chamber.

**Epifluorescence Imaging.** Stitched images were acquired using a Nikon Eclipse Ti inverted epifluorescent microscope (Nikon instruments Inc., Melville, NY) with a Zyla 4.2 sCMOS camera (Andor, South Windsor, CT). The degree of overlap is specified at 30%.

**Fat Pad Implantation of Vascularizing Gels.** All work involving animals was performed as described in protocols approved by the Rice University IACUC. Male SCID/bg mice (6-10 weeks old, Charles River, strain code 250), underwent fat pad implantation of vascularizing gels. Briefly, mice were anesthetized with 2-4% isoflurane, and abdomens were shaved and sterilized with betadine and isopropanol. Mice received 1 mg/kg buprenorphine SR lab (Zoopharm) and were administered 1 mL 0.9% saline to prevent dehydration. Scalpel and scissors were used to create an incision at the linea alba, and Adson forceps used to externalize the left and right gonadal fat pads. Vascularizing hydrogels were gently rinsed with PBS prior to implantation, and the gonadal fat pads were sutured to either side of the vascularizing hydrogel. Sterile 0.9% saline was used to gently irrigate the gel during implantation to maintain hydration. After suturing to the fat pads, the gel was introduced into the abdominal cavity, and muscle and skin were closed. Mice were monitored postoperatively and provided care and husbandry as needed.

**Dextran Injection.** For some samples, Texas Red-conjugated, fixable dextran, 70 kDa (D1864, ThermoFisher) was prepared at 25 mg/mL in PBS. For other samples, CF680-conjugated, fixable dextran, 70 kDa (Biotium, Cat# 80129) was prepared at 2.5 mg/mL in PBS. 100 µL of dextran solution was injected via tail vein 15 min prior to euthanasia. Gels were surgically explanted and photographed with a Sony A7R3 camera (Sony), and a Canon macro lens EF 100 mm 1:2.8 L IS USM (Canon). The gels were then submerged in icecold 4% PFA and fixed for 48 h at 4 °C. After fixation, gels were washed with PBS 3x, 20 min each wash, and maintained in PBS at 4 °C for imaging.

**Islet Isolation.** Female Sprague-Dawley rats (251-275g, Charles River, strain code 400), were utilized for islet isolation as described by protocols approved by the Rice University IACUC. Rats were first humanely euthanized. Then, the abdomen was opened via a V-shaped cut, the diaphragm was cut, and the liver was flipped to expose the common bile duct. 15-20 mL of islet isolation buffer (buffer formulation), containing 1.5 mg/mL of Collagenase XI (C7657, Millipore Sigma) was infused into the common bile duct, after which the pancreas was surgically removed and processed for enzymatic digestion of rat islets. Digestion included 14 min of incubation at 37 °C, several washes with 10% BSA (50-253-923, Fisher Scientific), and then gradient density separation (Products 99-690- CIS, 99-815-CIS, 99-691-CIS, 99-692-CIS, Corning). Islets were maintained in RPMI- 1640 (10-040-CM, Corning) with 10% FBS and 1% Penicillin/Streptomycin.

**Diabetes Induction.** Male SCID/bg mice (6-10 weeks old, Charles River, strain code 250), underwent diabetes induction as described by animal protocols approved by the Rice University IACUC. 70 mg/kg of streptozotocin (572201, Millipore Sigma), dissolved in citrate buffer, was administered to mice for 5 days consecutively.

**Glucose Monitoring.** Blood glucose strips and glucometers (BG1000, Clarity Diagnostics) were used to quantify blood glucose in a droplet of blood produced by tail prick. Mice were considered diabetic after 2 consecutive days of 1h-fasted blood glucose measurements greater than 400 mg/dL. Mice were considered normoglycemic after non-fasting blood glucose measurements less than or equal to 250 mg/dL.

**Islet Encapsulation in Alginate Blocks.** TMTD Alginate and SLG100 (Pronova, Sandikva, Norway) were dissolved at 5% and 3% in two separate solutions of 0.8% saline. These solutions were combined at an 80:20 v/v ratio of UPVLVG to SLG100. Alginate was sterile filtered with 0.2-micron syringe filter and used to resuspend freshly isolated rat islets. Casting was accomplished by pipetting alginate with islets into a sterile 20 wt.% PEGDA mold that was pre-soaked in crosslinking solution: 20 mM BaCl₂ (Sigma-Aldrich 342920), 5 wt.% mannitol (Thermo Fisher 33342). A thin polycarbonate membrane (Sterlitech 0.01 Micron, 62x22 mm, PCT00162X22100) was soaked in crosslinking solution and folded over the side of the mold so it could cover the top and extend under the bottom. The ends of the rectangular membrane were pinched with forceps to pull the membrane taut, and the mold was lifted and held in a 2-3 cm high bath of crosslinker (100 mm x 50 mm crystalizing dish, PYREX). This was held in crosslinker for 20 seconds before being brought out of the bath and laid on a clean, non-absorbent surface. The membrane was unwrapped, and the mold was gently stuck onto a glass slide (Thermo Fisher Cat. No. 12-544-7). The slide was then gently lowered back into the bath to pin the mold between the dish and the slide. A weight (VWR 50 mL conical tube filled with water) was then placed on top of the slide to depress the mold, and the crosslinking was allowed to continue for one hour. After the hour had passed, the mold was obtained from the bath, and the block was gently peeled out. The block was then washed in a 50 mL conical with 20 mL of HEPES buffer, where 20 mL was added, 15 mL was drained, and 15 mL of new buffer was added for each subsequent wash. The block was washed with complete media three times in the same fashion before being added to 10 mL of media for storage in an incubator.

**Islet Viability.** Live/dead staining solution was prepared by diluting Live/Dead Kit (L3224, Thermo Fisher) 1:200 v/v Calcein AM and 1:200 v/v Ethidium Homodimer-1 in complete RMPI-1640. This staining solution was added 1:1 v/v to media in wells containing encapsulated islets. The encapsulated islets were kept at 37 °C, 5% CO₂ for 30 min, and subsequently fixed with 4% PFA for 24 h at 4 °C. Stained and fixed islets were imaged using EVOS FL (Thermo Fisher) and Nikon Eclipse T*i* Epifluorescence Microscope (Nikon).

**Glucose Stimulated Insulin Release.** Krebs buffer (KRB) was prepared as described by (Veiseh et al., Nature Medicine 22: 306-311, 2016). KRB was prepared with 2 mM glucose (Sigma; G7528) (KR2) and 18 mM glucose (KR18). Encapsulated islets were incubated for 30 min in KR2 (wash), 1h in KR2 (low), then 1 hr in KR18 (high). Supernatants were then collected from the low (KR2) and high (KR20) wells, and the insulin concentrations were quantified by ultrasensitive rat insulin ELISA (ALPCO) in duplicate without sample dilution.

**Statistical Analysis.** Statistical analyses were performed using GraphPad Prism 8 software (GraphPad Software); *P < 0.05, **P < 0.01, ***P < 0.001 and ****P < 0.0001.

### Example 3 - Results

In order to develop an immunomodulatory small molecule-modified, 3D printable, and degradable copolymer gel substrate, the inventors synthesized PEGDA, GelMA and Met-RZA15 using established methods. LAP was prepared as previously described using equimolar amounts of dimethyl phenylphosphinite and 2,3,6-trimethylbenzoyl chloride under argon overnight at room temperature (FIG. 16A) (Fairbanks *et al.,* 2009). In the following step, poly(ethylene glycol) diacrylate (PEGDA), 3.4 kDa, 6 kDa, 10 kDa, 20 kDa, and 35 kDa, was synthesized by reaction between poly(ethylene glycol) acryloyl chloride (in presence of triethylamine overnight (FIG. 16A) (Miller *et al.,* 2010). Gelatin methacrylate (GelMA) was synthesized by the dropwise addition of methacrylic anhydride (0.1 mL per 1.0 g gelatin) to the mixture of gelatin (Type A, bloom 300) dissolved in 100 mM carbonate/bicarbonate buffer (pH = 9.5) at 50 C for 4 hours (FIG. 16B) (Shirahama *et al.,* 2016). Immunomodulatory small molecule Met-RZA15 was synthesized in a two-step method (FIG. 16C). In the first step, RZA15 was synthesized by a click chemistry reaction in presence of copper iodide as a catalyst (Vegas *et al.,* 2016b). In the following step, methacryloyl modified RZA15 (Met-RZA15) was synthesized by the reaction between RZA15 and methacryloyl chloride in presence of triethyl amine under argon (FIG. 16C). Intermediates and products were characterized by ESI and/or NMR (¹H and ¹³C) analysis to determine the mass and purity. Small molecule modified gels were produced by photopolymerization of 0.5 M aqueous Met-RZA15 to PEGDA (3.25 wt%), GelMA (10 wt%), and 17 mM of LAP (FIG. 16D). The batch sizes for these reagents are sufficiently large to meet the large material quantities required for these studies.

**Table A**

| **Polymer** | **Acrylation (%) (in-house)** | **Acrylation (%) (range in literature)** | **Citation(s)** |
|---|---|---|---|
| PEGDA (3.4 kDa) | 88.7 ± 1.3 | >95 | (5), (6) |
| PEGDA (6 kDa) | 87.1 ± 5.7 | >90 | (6), (7) |
| GelMA | 53.4 ± 8.3 | >50 | (8) |

**Degrees of polymer acrylation are consistent and comparable to those found throughout the literature.** For bi-functional polymers such a PEGDA, where functional groups are only present on the termini of each linear chain, a high degree of acrylation is required to fully incorporate each molecule within the hydrogel mesh. With >85% acrylation, our in-house synthesis is comparable to other expert chemists in the field, supporting 3D printing of high-quality gels. For multi-functional polymers such as GelMA, where functional groups are present as amino acid side chains throughout the backbone, the desired degree of acrylation is application-dependent. Progressively higher acrylation increases gel stiffness for improved handling, but decreases pore size, inhibiting cell spreading and leading to slower collagenase degradation rates. For this reason, all studies were performed with GelMA having moderate acrylation (53.4 ± 8.3%). Acrylation was confirmed with 1H NMR by measuring the percentage of gelatin's lysine residues that had primary amine substitutions. With optimal acrylation degrees for large batches of biocompatible polymer (350 g PEGDA, 45 g GelMA), our synthesis pipeline supports 3D printing of hundreds of vascularization devices for parallel studies *in vitro* and *in vivo.* This scale is previously unreported in the field, reinforcing our group's potential for success in pursuing vascular integration of implanted islet structures.

**Table B**

| **Polymer** | **Storage Modulus (Pa)** |
|---|---|
| 3.4 kDa PEGDA (20% w/v) | 64,962 ± 4,091 |
| 6 kDa PEGDA (20% w/v) | 36,663 ± 5,759 |
| GelMA (5% w/v) | 688 ± 179 |

**Analysis of polymer mechanics demonstrates low batch-to-batch variability.** Beyond NMR confirmation of reproducible acrylation, we further demonstrate reproducibility by quantifying the ability of different polymer batches to behave consistently under shear deformation (storage modulus). This metric was obtained using an AR-G2 parallel plate rheometer. For each replicate, a 45 µL sample of polymer solution (containing 0.05 wt% Irgacure 2959) was added to the bottom plate and the top plate was lowered to obtain a gap size of 50 µm. Using an angular frequency of 5 rad/s and 10% strain, the polymer solution was pre-conditioned for 30 s and then exposed to 10 mW/cm² of light from a 365 nm lamp. The evolution of the storage modulus was monitored as the photocrosslinking reaction proceeded, ultimately yielding a plateau value that can be compared to other samples.

With clinical targets in mind, implantable gel structures must be able to degrade within the body. But if these gels completely degrade before anastomosis occurs, exogenous islets will be unable to interact with the native circulation. A similar balance exists with respect to gel mechanics, where gels must be stiff enough to surgically manipulate. Here the inventors find that small (150 µL) 10% GelMA gels rapidly degrade in the presence of 0.2 mg/mL collagenase, and that degradation kinetics can be tuned by incorporating PEGDA. By adding relatively small amounts of PEGDA to the gel formulation, the collagenase degradation takes 4-fold longer and produces a 3-fold increase in shear stiffness as measured using a parallel plate rheometer during UV exposure. These data suggest that the current formulation of 10% GelMA, 3.25% 3.4 kDa PEGDA is able to satisfy enzymatic and mechanical requirements: moderate degradation rates to support anastomosis and sufficient rigidity to support surgical manipulation. Furthermore, the ability to tune these properties presents a unique opportunity to optimize vascularization *in vivo* without the need for exogenous stromal cells or growth factors. See FIGS. 17A-B.

The inventors incorporate the anti-fibrotic molecule Met-RZA to prevent fibrosis of the 3D printed gel. To verify the surface presence of the Met-RZA on the gels, TOF-SIMS analysis was used to characterize the gel surfaces. Negative high mass resolution depth profile was obtained using a TOF-SIMS NCS instrument, which combines a TOF-SIMS instrument (ION-TOF GmbH, Munster, Germany) and an *in-situ* Scanning Probe Microscope (NanoScan, Switzerland). A bunched 30 keV Bi³⁺ ions (with a measured current of 0.2pA) was used as primary probe for analysis (scanned area 100 × 10 µm²) with a raster of 64 x 64 pixels. An adjustment of the charge effects has been operated using a surface potential of -10 V. The cycle time was fixed to 200 µs (corresponding to m/z = 0 - 3649 a.m.u mass range). The TOF-SIMS analysis analyzed the gel surface for the presence of small molecule (Met-RZA and RZA15) (FIGS. 18B-D). The negative polarity TOF-SIMS data shows higher level of Met-RZA (mass = 459.26, 1.6-fold) and fragmented RZA15 (mass=390.98, 5.6-fold) in the gels printed with the Met-RZA small molecule compared to those printed without. This demonstrates the presence of immunoprotective small molecule on the surface of 3D printed gels, which will reduce fibrosis of implanted gels.

The inventors demonstrate their ability to not only print patent vascular channels, but also to control circularity, which is critical for endothelial coverage, proper hemodynamics, and leakproof catheterization. This capability enables realization of hydrogel vascular networks with complex 3D architectures, including various topologies of vascular networks. See FIGS. 19A-D.

With the inventors' demonstrated channel patency fabricated as designed, the inventors show they can successfully create perfusable vessel networks to support convective flow within a biocompatible vascularization device. This also implies this 3D bioprinting technique can accommodate arbitrary vascular architectures should the inventors deem a varied vascular design better suits long-term viability of the islets within the vascularization device.

From the velocity profiles of both hydrogel architectures, it appears that the no-slip boundary condition is a valid assumption because the velocity at either edge of perfusable volume is approximately zero. The bestfit curves represent the data well, as indicated by high r2 values. Although minor variations exist in the flow dynamics of these gel architectures, flow generally appears to be free of obstructions. It is therefore possible for these hydrogels to accommodate blood flow if anastomosis occurs *in vivo,* which would assist vascular integration to support islet cells. See FIGS. 21A-G.

As the inventors expect, their vascularizing devices to fully integrate with the host's circulatory system, it is critical that the vessels do not burst during the pressure changes of the cardiac cycle. Furthermore, cyclic expansion of blood vessels supports healthy hemodynamics and provides mechanical cues to the underlying cell populations (e.g., endothelium, muscle, stroma) that promote homeostasis.

The inventors demonstrate that their vascular networks can be fully endothelialized while retaining their lumens (FIG. 23C, inset cross section). Moreover, they have evidence that the HUVECs can actively degrade and invade the gel. They have also observed migrating HUVECs in the parenchymal gel space indicative of single cell migration (FIG. 23A, inset, arrows indicate single cell migration) and of angiogenic sprouting (FIG. 23D). The endothelial cells align in the direction of flow (FIG. 23B) and assume a classic cobblestone morphology (FIG. 23C, inset). Over multiple days, HUVECs spread to form a confluent monolayer within the cylindrical lumen. These endothelialized gels can be stably maintained under long-term perfusion. believe this endothelial monolayer has reached quiescence and can persist until ready for *in vivo* implantation. The inventors believe that the incorporation of endothelialized networks within the vascularization device will support pancreatic islet viability *in vivo* while promoting host vasculature integration for long-term viability and functionality.

. High endothelial cell viability after multiple days of perfusion suggests that the endothelialized vascular network remains robust long-term further suggesting that the endothelial monolayer has matured. The endothelial monolayers can be maintained at this quiescent state for multiple days, without loss to viability. This allows grafts to be prepared ahead of time, and seeded as donor islets become available.

After successfully seeding the 3D printed gels with endothelial cells, islets were introduced to the gels (FIG. 25A) by alginate casting. The casting method was developed to ensure crosslinked alginate surrounds the anchors (FIG. 25B) and holds the alginate and islets in place. In a sterile environment, two types of alginate, UP VLVG modified with RZA15 and SLG100 (NovoMatrix, Sandvika, Norway), were mixed in a 60:40 ratio and filtered, yielding TMTD alginate. 60 µl of TMTD alginate was pipetted into the gel chamber, and crosslinked with 100 mM CaCl₂ and 1 mM BaCl₂ solution for 30 min. The process was repeated to fill the chamber with alginate and islets. Successful crosslinking was verified by vibratome cross-section, showing solid alginate throughout the block. By following the above procedure, gels cast with TMTD can be consistently produced. This alginate casting procedure leads to a construct that can be handled firmly without falling apart. Successful alginate casting permits islet encapsulation.

TMTD alginate was prepared and mixed with human pancreatic islets. Gels with alginate and islets were incubated with Live/Dead stain (1:200 Calcein AM, 1:200 Ethidium Homodimer in complete CMRL-1066) for 30 min. Islet-containing gels were then fixed and imaged on a Nikon T*i* Eclipse epifluoresence microscope.

For GSIS, 3D-printed vascularizing devices were cast with TMTD alginate and human pancreatic islets. 3D-printed vascularizing devices were incubated in 2 mM Krebs buffer for 30 min (pre-wash), 60 min (wash), and 60 min (Low glucose). They were then incubated in 18 mM Krebs buffer for 60 min (High glucose). The supernatants of Low and High glucose were analyzed in duplicate by ultrasensitive human ELISA (Alpco, Salem, NH). Stimulation index was calculated as the ratio of insulin produced during High glucose divided by that produced during Low glucose.

Demonstrating >90% viability and stimulation index >2 indicates that the inventors' device and methods of islet encapsulation are compatible with fragile islet biology, enhancing translational value of this approach for allogenic and xenogenic islet transplantation.

In order to assess *in vivo* anastomosis of gels with patterned vasculature, the inventors implanted gels endothelialized with GFP-HUVECs (FIG. 26A). Gels were seeded with GFP-HUVECs and maintained for 6 days in perfusion culture (FIG. 26B). To promote vascular anastomosis, the highly vascularized gonadal fat pads were wrapped around and sutured to the gel (FIG. 26C). Prior to euthanasia, mice received human-specific lectin and mouse-specific lectin via tail vein injection. Gels were explanted at 3 weeks, fixed, and maintained at 4C for analysis. 3D-printed channels retain serpentine topology and remain patent during *in vivo* implantation, indicating that uniquely patterned vasculature *in vitro* translates to similar patterns *in vivo.* Visualization of blood in the gel channels (FIG. 26D) is a promising sign of vascular integration with the gel. Closely associated blood vessels (FIG. 26D) indicate the gel is degradable, and capable of vascular integration with the host. These findings encourage the potential of this approach to yield patterned vasculature *in vivo* for encapsulated islet transplantation.

### Example 4

Development of patterned, multi-layer hydrogels with various biomaterial characteristics. Patterned hydrogels be developed as follows: multiple hydrogel formulations can be utilized to create a multi-layered patterned hydrogel with various biomaterial characteristics, including degradability, stiffness, cellular components, cell-adhesion properties.

A tri-layered hydrogel, with a degradable bottom and top layer, with a non-degradable middle layer can be developed as follows: hyaluronic acid modified with norbornene functional groups (norHA), combined with photoinitiator (*e*.*g*., LAP), and degradable peptide crosslinker (*e*.*g*., CGPQGIWGQGCR (SEQ ID NO: 1)), results in a patterned, fully synthetic hydrogel formulation with degradable peptide crosslinks, allowing for specifying the degradation profile.

After the non-degradable portion is printed, for example, using a stereolithography approach, the print solution can be exchanged for one that is non-degradable, for example 6.0 kDa PEG-diacrylate. A patterned component of non-degradable PEG-diacrylate can be printed in the same device.

Finally, the non-degradable gel formulation can be exchanged for a degradable gel formulation, *e*.*g*., norHA + LAP + CGPQGIWGQGCR (SEQ ID NO: 1) peptide crosslinks. This would allow for creation of multi-layered biomaterials with different biomaterial properties.

Hyaluronic acid was functionalized with norbornene to yield norbornene-modified hyaluronic acid (NorHA) by mixing norbornene and hyaluronic acid in the presence of Boc2O, DMAP, and DMSO, stirring at 45C for 20 hours. Hyaluronic acid gel was printed (exposure to blue 405 nm light) with pendant cell-adhesive peptides, with degradable peptide crosslinks by addition of lithium acrylphosphinate (LAP), along with cell-adhesive, thiol-terminated peptide (CGRGDS (SEQ ID NO: 2)), and MMP sensitive dithiol crosslinking peptide (CGPQGIWGQGCR (SEQ ID NO: 1)). Peptide sequences CGPQGIWGQGCR (SEQ ID NO: 1) (highly degradable, HD, 1261.42 g/mol) and CGRGDS (SEQ ID NO: 2) (thiol-terminated cell-adhesive peptide) were custom synthesized by Aapptec (Louisville, KY). Peptide was supplied as a trifluoroacetate salt at >95% purity. Peptides were evacuated of air and stored under argon (to minimize disulfide formation) at -80 °C until needed (Miller et al., Biomaterials, 31(13: 3736-43, 2010).

Gels were prinited as follows: NorHA and 6 kDa PEG-diacrylate were dissolved in PBS at 4 wt% each. Thiol-terminated degradable crosslinking peptide CGPQGIWGQGCR (SEQ ID NO: 1) was added at 10 mM. LAP was added at 34 mM. Photoabsorber tartrazine was added at 2.25 mM.

### IX. References

The following references, to the extent that they provide exemplary procedural or other details supplementary to those set forth herein.
Veiseh O, Tang BC, Whitehead KA, Anderson DG, Langer R. Managing diabetes with nanomedicine: challenges and opportunities. Nat Rev Drug Discov. 2015a;14(1):45-57. doi: 10.1038/nrd4477. PubMed PMID: 25430866; PMCID: 4751590.
Pickup JC. Insulin-pump therapy for type 1 diabetes mellitus. The New England journal of medicine. 2012;366(17):1616-24. doi: 10.1056/NEJMct1113948. PubMed PMID: 22533577.
Robertson RP. Islet transplantation as a treatment for diabetes - a work in progress. The pNew England journal of medicine. 2004;350(7):694-705. doi: 10.1056/NEJMra032425. PubMed PMID: 14960745.
Hirshberg B. Lessons learned from the international trial of the Edmonton protocol for islet transplantation. Current diabetes reports. 2007;7(4):301-3. PubMed PMID: 17686407.
Shapiro AM, Ricordi C, Hering BJ, Auchincloss H, Lindblad R, Robertson RP, Secchi A, Brendel MD, Berney T, Brennan DC, Cagliero E, Alejandro R, Ryan EA, DiMercurio B, Morel P, Polonsky KS, Reems JA, Bretzel RG, Bertuzzi F, Froud T, Kandaswamy R, Sutherland DE, Eisenbarth G, Segal M, Preiksaitis J, Korbutt GS, Barton FB, Viviano L, Seyfert-Margolis V, Bluestone J, Lakey JR. International trial of the Edmonton protocol for islet transplantation. The New England journal of medicine. 2006;355(13):1318-30. doi: 10.1056/NEJMoa061267. PubMed PMID: 17005949.
Pagliuca FW, Millman JR, Gurtler M, Segel M, Van Dervort A, Ryu JH, Peterson QP, Greiner D, Melton DA. Generation of functional human pancreatic beta cells in vitro. Cell. 2014;159(2):428-39. doi: 10.1016/j.cell.2014.09.040. PubMed PMID: 25303535; PMCID: 4617632.
Vegas AJ, Veiseh O, Gurtler M, Millman JR, Pagliuca FW, Bader AR, Doloff JC, Li J, Chen M, Olejnik K, Tam HH, Jhunjhunwala S, Langan E, Aresta-Dasilva S, Gandham S, McGarrigle JJ, Bochenek MA, Hollister-Lock J, Oberholzer J, Greiner DL, Weir GC, Melton DA, Langer R, Anderson DG. Long-term glycemic control using polymer-encapsulated human stem cell-derived beta cells in immune-competent mice. Nat Med. 2016a;22(3):306-11. doi: 10.1038/nm.4030. PubMed PMID: 26808346; PMCID: PMC4825868.
Shapiro AM. Islet transplantation in type 1 diabetes: ongoing challenges, refined procedures, and long-term outcome. The review of diabetic studies: RDS. 2012;9(4):385-406. doi: 10.1900/RDS.2012.9.385. PubMed PMID: 23804275; PMCID: 3740705.
Vogel G. Biomedicine. Stem cell recipe offers diabetes hope. Science. 2014;346(6206):148. doi: 10.1126/science.346.6206.148. PubMed PMID: 25301592.
Dolgin E. Encapsulate this. Nat Med. 2014;20(1):9-11. doi: 10.1038/nm0114-9. PubMed PMID: 24398953.
Jacobs-Tulleneers-Thevissen D, Chintinne M, Ling Z, Gillard P, Schoonjans L, Delvaux G, Strand BL, Gorus F, Keymeulen B, Pipeleers D, Beta Cell Therapy Consortium E-F. Sustained function of alginate-encapsulated human islet cell implants in the peritoneal cavity of mice leading to a pilot study in a type 1 diabetic patient. Diabetologia. 2013;56(7):1605-14. doi: 10.1007/s00125-013-2906-0. PubMed PMID: 23620058.
Harding JL, Reynolds MM. Combating medical device fouling. Trends in biotechnology. 2014;32(3):140-6. doi: 10.1016/j.tibtech.2013.12.004. PubMed PMID: 24438709.
Langer R. Perspectives and challenges in tissue engineering and regenerative medicine. Advanced materials. 2009;21(32-33):3235-6. doi: 10.1002/adma.200902589. PubMed PMID: 20882493.
Williams DF. On the mechanisms of biocompatibility. Biomaterials. 2008;29(20):2941-53. doi: 10.1016/j.biomaterials.2008.04.023. PubMed PMID: 18440630.
Ratner BD. Reducing capsular thickness and enhancing angiogenesis around implant drug release systems. Journal of controlled release : official journal of the Controlled Release Society. 2002;78(1-3):211-8. PubMed PMID: 11772462.
Ward WK. A Review of the Foreign-body Response to Subcutaneously-implanted Devices: The Role of Macrophages and Cytokines in Biofouling and Fibrosis. J Diabetes Sci Technol. 2008;2:768-77; PMCID: 2769792.
Zhang L, Cao Z, Bai T, Carr L, Ella-Menye JR, Irvin C, Ratner BD, Jiang S. Zwitterionic hydrogels implanted in mice resist the foreign-body reaction. Nature biotechnology. 2013;31(6):553-6. doi: 10.1038/nbt.2580. PubMed PMID: 23666011.
Lum ZP, Tai IT, Krestow M, Norton J, Vacek I, Sun AM. Prolonged reversal of diabetic state in NOD mice by xenografts of microencapsulated rat islets. Diabetes. 1991;40(11):1511-6. PubMed PMID: 1936609.
Scharp DW, Marchetti P. Encapsulated islets for diabetes therapy: history, current progress, and critical issues requiring solution. Advanced drug delivery reviews. 2014;67-68:35-73. doi: 10.1016/j.addr.2013.07.018. PubMed PMID: 23916992.
Schneider S, Feilen PJ, Brunnenmeier F, Minnemann T, Zimmermann H, Zimmermann U, Weber MM. Long-term graft function of adult rat and human islets encapsulated in novel alginate-based microcapsules after transplantation in immunocompetent diabetic mice. Diabetes. 2005;54(3):687-93. PubMed PMID: 15734844.
Kearney CJ, Mooney DJ. Macroscale delivery systems for molecular and cellular payloads. Nature materials. 2013;12(11):1004-17. doi: 10.1038/nmat3758. PubMed PMID: 24150418.
Lee KY, Mooney DJ. Alginate: properties and biomedical applications. Progress in polymer science. 2012;37(1):106-26. doi: 10.1016/j.progpolymsci.2011.06.003. PubMed PMID: 22125349; PMCID: 3223967.
de Vos P, Faas MM, Strand B, Calafiore R. Alginate-based microcapsules for immunoisolation of pancreatic islets. Biomaterials. 2006;27(32):5603-17. doi: 10.1016/j.biomaterials.2006.07.010. PubMed PMID: 16879864.
Robitaille R, Dusseault J, Henley N, Desbiens K, Labrecque N, Halle JP. Inflammatory response to peritoneal implantation of alginate-poly-L-lysine microcapsules. Biomaterials. 2005;26(19):4119-27. doi: 10.1016/j.biomaterials.2004.10.028. PubMed PMID: 15664639.
Weir GC. Islet encapsulation: advances and obstacles. Diabetologia. 2013;56(7):1458-61. doi: 10.1007/s00125-013-2921-1. PubMed PMID: 23636639.
King A, Sandler S, Andersson A. The effect of host factors and capsule composition on the cellular overgrowth on implanted alginate capsules. Journal of biomedical materials research. 2001;57(3):374-83. PubMed PMID: 11523032.
Sato Y, Endo H, Okuyama H, Takeda T, Iwahashi H, Imagawa A, Yamagata K, Shimomura I, Inoue M. Cellular hypoxia of pancreatic beta-cells due to high levels of oxygen consumption for insulin secretion in vitro. J Biol Chem. 2011;286(14):12524-32. Epub 2011/02/08. doi: 10.1074/jbc.M110.194738. PubMed PMID: 21296882; PMCID: PMC3069454.
Miller JS. The billion cell construct: will three-dimensional printing get us there? PLoS Biol. 2014;12(6):e1001882. Epub 2014/06/18. doi: 10.1371/journal.pbio.1001882. PubMed PMID: 24937565; PMCID: PMC4061004.
Baranski JD, Chaturvedi RR, Stevens KR, Eyckmans J, Carvalho B, Solorzano RD, Yang MT, Miller JS, Bhatia SN, Chen CS. Geometric control of vascular networks to enhance engineered tissue integration and function. Proc Natl Acad Sci U S A. 2013;110(19):7586-91. Epub 2013/04/24. doi: 10.1073/pnas.1217796110. PubMed PMID: 23610423; PMCID: PMC3651499.
O'Sullivan ES, Vegas A, Anderson DG, Weir GC. Islets transplanted in immunoisolation devices: a review of the progress and the challenges that remain. Endocrine reviews. 2011;32(6):827-44. Epub 2011/09/29. doi: 10.1210/er.2010-0026. PubMed PMID: 21951347; PMCID: 3591674.
de Vos P, Lazarjani HA, Poncelet D, Faas MM. Polymers in cell encapsulation from an enveloped cell perspective. Advanced drug delivery reviews. 2013. Epub 2013/11/26. doi: 10.1016/j.addr.2013.11.005. PubMed PMID: 24270009.
Lacik I. Current status on immunoprotection of transplanted islets: focus on islet microencapsulation. Micro and Nanosystems. 2013;5(3):168-76.
Rokstad AM, Lacik I, de Vos P, Strand BL. Advances in biocompatibility and physicochemical characterization of microspheres for cell encapsulation. Advanced drug delivery reviews. 2013. Epub 2013/07/24. doi: 10.1016/j.addr.2013.07.010. PubMed PMID: 23876549.
Finkenzeller G, Torio-Padron N, Momeni A, Mehlhorn AT, Stark GB. In vitro angiogenesis properties of endothelial progenitor cells: a promising tool for vascularization of ex vivo engineered tissues. Tissue Eng. 2007;13(7):1413-20. doi: 10.1089/ten.2006.0369. PubMed PMID: 17550338.
Lovett M, Lee K, Edwards A, Kaplan DL. Vascularization strategies for tissue engineering. Tissue Eng Part B Rev. 2009;15(3):353-70. doi: 10.1089/ten.TEB.2009.0085. PubMed PMID: 19496677; PMCID: PMC2817665.
Wenger A, Kowalewski N, Stahl A, Mehlhorn AT, Schmal H, Stark GB, Finkenzeller G. Development and characterization of a spheroidal coculture model of endothelial cells and fibroblasts for improving angiogenesis in tissue engineering. Cells Tissues Organs. 2005;181(2):80-8. doi: 10.1159/000091097. PubMed PMID: 16534202.
Andrae J, Gallini R, Betsholtz C. Role of platelet-derived growth factors in physiology and medicine. Genes Dev. 2008;22(10):1276-312. doi: 10.1101/gad.1653708. PubMed PMID: 18483217; PMCID: PMC2732412.
Bauer SM, Bauer RJ, Liu ZJ, Chen H, Goldstein L, Velazquez OC. Vascular endothelial growth factor-C promotes vasculogenesis, angiogenesis, and collagen constriction in three-dimensional collagen gels. J Vasc Surg. 2005;41(4):699-707. doi: 10.1016/j.jvs.2005.01.015. PubMed PMID: 15874936.
Burdick JA, Anseth KS. Photoencapsulation of osteoblasts in injectable RGD-modified PEG hydrogels for bone tissue engineering. Biomaterials. 2002;23(22):4315-23. PubMed PMID: 12219821.
Lutolf MP, Lauer-Fields JL, Schmoekel HG, Metters AT, Weber FE, Fields GB, Hubbell JA. Synthetic matrix metalloproteinase-sensitive hydrogels for the conduction of tissue regeneration: engineering cell-invasion characteristics. Proc Natl Acad Sci U S A. 2003;100(9):5413-8. doi: 10.1073/pnas.0737381100. PubMed PMID: 12686696; PMCID: PMC154359.
Chrobak KM, Potter DR, Tien J. Formation of perfused, functional microvascular tubes in vitro. Microvasc Res. 2006;71(3):185-96. Epub 2006/04/08. doi: 10.1016/j.mvr.2006.02.005. PubMed PMID: 16600313.
Vegas AJ, Veiseh O, Doloff JC, Ma M, Tam HH, Bratlie K, Li J, Bader AR, Langan E, Olejnik K, Fenton P, Kang JW, Hollister-Locke J, Bochenek MA, Chiu A, Siebert S, Tang K, Jhunjhunwala S, Aresta-Dasilva S, Dholakia N, Thakrar R, Vietti T, Chen M, Cohen J, Siniakowicz K, Qi M, McGarrigle J, Lyle S, Harlan DM, Greiner DL, Oberholzer J, Weir GC, Langer R, Anderson DG. Combinatorial hydrogel library enables identification of materials that mitigate the foreign body response in primates. Nature biotechnology. 2016b;34(3):345-52. doi: 10.1038/nbt.3462. PubMed PMID: 26807527.
Veiseh O, Doloff JC, Ma M, Vegas AJ, Tam HH, Bader AR, Li J, Langan E, Wyckoff J, Loo WS, Jhunjhunwala S, Chiu A, Siebert S, Tang K, Hollister-Lock J, Aresta-Dasilva S, Bochenek M, Mendoza-Elias J, Wang Y, Qi M, Lavin DM, Chen M, Dholakia N, Thakrar R, Lacik I, Weir GC, Oberholzer J, Greiner DL, Langer R, Anderson DG. Size- and shape-dependent foreign body immune response to materials implanted in rodents and non-human primates. Nature materials. 2015b;14(6):643-51. doi: 10.1038/nmat4290. PubMed PMID: 25985456; PMCID: 4477281.
Sooppan et al. 2016. Tissue Engineering. Part C, Methods, 22(1), 1-7. doi.org/10.1089/ten.TEC.2015.0239
Baranski et al. 2013. Geometric control of vascular networks to enhance engineered tissue integration and function. PNAS, 110(19), 7586-7591. doi.org/10.1073/pnas.1217796110
Miller, J. S., Shen, C. J., Legant, W. R., Baranski, J. D., Blakely, B. L., & Chen, C. S. (2010). Bioactive hydrogels made from step-growth derived PEG-peptide macromers. Biomaterials, 31(13), 3736-3743. doi.org/10.1016/j.biomaterials.2010.01.058
Stevens, K. R., Miller, J. S., Blakely, B. L., Chen, C. S., & Bhatia, S. N. (2015). Degradable hydrogels derived from PEG-diacrylamide for hepatic tissue engineering. Journal of Biomedical Materials Research. Part A, 103(10), 3331-3338. doi.org/10.1002/jbm.a.35478
Stevens KR, Miller JS, Blakely BL, Chen CS, Bhatia SN. Degradable hydrogels derived from PEG-diacrylamide for hepatic tissue engineering. J Biomed Mater Res A. 2015;103(10):3331-8. doi: 10.1002/jbm.a.35478. PubMed PMID: 25851120; PMCID: PMC4890565.
Fujita H, Mochizuki A. The origin of the diversity of leaf venation pattern. Dev Dyn. 2006;235(10):2710-21. doi: 10.1002/dvdy.20908. PubMed PMID: 16894601.
Fairbanks BD, Schwartz MP, Bowman CN, Anseth KS. Photoinitiated polymerization of PEGdiacrylate with lithium phenyl-2,4,6-trimethylbenzoylphosphinate: polymerization rate and cytocompatibility. Biomaterials. 2009;30(35):6702-7. Epub 2009/09/29. doi: 10.1016/j.biomaterials.2009.08.055. PubMed PMID: 19783300; PMCID: PMC2896013.
Shirahama H, Lee BH, Tan LP, Cho NJ. Precise Tuning of Facile One-Pot Gelatin Methacryloyl (GelMA) Synthesis. Sci Rep. 2016;6:31036. Epub 2016/08/10. doi: 10.1038/srep31036. PubMed PMID: 27503340; PMCID: PMC4977492.
Peter M, Tayalia P. An alternative technique for patterning cells on poly(ethylene glycol) diacrylate hydrogels. Rsc Adv. 2016;6(47):40878-85. doi: 10.1039/c6ra08852j. PubMed PMID:WOS:000375270600032.
Zhang X, Xu B, Puperi DS, Yonezawa AL, Wu Y, Tseng H, Cuchiara ML, West JL, Grande-Allen KJ. Integrating valve-inspired design features into poly(ethylene glycol) hydrogel scaffolds for heart valve tissue engineering. Acta Biomater. 2015;14:11-21. Epub 2014/11/30. doi: 10.1016/j.actbio.2014.11.042. PubMed PMID: 25433168; PMCID: PMC4334908.
Son KH, Lee JW. Synthesis and Characterization of Poly(Ethylene Glycol) Based Thermo-Responsive Hydrogels for Cell Sheet Engineering. Materials (Basel). 2016;9(10). Epub 2017/08/05. doi: 10.3390/ma9100854. PubMed PMID: 28773974; PMCID: PMC5456593.
Zhou L, Tan G, Tan Y, Wang H, Liao J, Ning CJRA. Biomimetic mineralization of anionic gelatin hydrogels: effect of degree of methacrylation2014;4(42):21997-2008.
Thielicke W, Stamhuis EJ. PIVlab - Towards User-friendly, Affordable and Accurate Digital Particle Image Velocimetry in MATLAB. Journal of Open Research Software. 2014;2(1):e30. doi: 10.5334/jors.bl.
MillerLab Pneumatic System 2016. Available from: github.com/MillerLabFTW/OpenSourcePneumaticSystem.
L'Heureux N, Dusserre N, Konig G, Victor B, Keire P, Wight TN, Chronos NA, Kyles AE, Gregory CR, Hoyt G, Robbins RC, McAllister TN. Human tissue-engineered blood vessels for adult arterial revascularization. Nat Med. 2006;12(3):361-5. Epub 2006/02/24. doi: 10.1038/nm1364. PubMed PMID: 16491087; PMCID: PMC1513140.
U.S. Pat. Pub. 2011/0270412
U.S. Pat. Pub. 2016/0030360
U.S. Pat. Pub. 2017/0239397
U.S. Pat. Pub. 2017/0355799
U.S. Pat. Pub. 2018/0002658
U.S. Patent 9,452,239
U.S. Patent 9,555,007
PCT Pat. Pub. WO2016187225

## Claims

1. An implantable device comprising a degradable outer housing comprising a degradable polymer, and an inner non-degradable portion comprising a non-degradable polymer, wherein the degradable outer housing is patterned, and the patterned portion comprises blood vessel geometries.

2. The implantable device of claim 1, wherein the non-degradable portion comprises a natural or synthetic hydrogel.

3. The implantable device of claim 2:
a) wherein the synthetic hydrogel is poly(ethylene glycol) diacrylate (PEDGA);
b) wherein the implantable device further comprises gelatin methacrylate (GelMA); and/or
c) wherein the natural or synthetic hydrogel comprises more than 80% water; optionally
wherein the natural or synthetic hydrogel comprises about 10% GelMA and about 3-4% PEDGA having a molecular weight of about 3.4 kDa.

4. The implantable device of claim 2, wherein:
a) said natural or synthetic hydrogel comprises monomers of acrylate-(PEG-protein)ₙ-PEG-acrylate, optionally wherein PEG is 2-35 kDa, protein is greater than 100 Da, and total MW is at least 500 kDa or n = 2-500 repeat units; or
b) the natural or synthetic hydrogel comprises acrylamide, vinyl-sulfone, norbornene replace the acrylate groups, alginate, silk, dextran, chondroitin sulfate, hyaluronic acid, cellulose, heparin or poly(caprolactone).

5. The implantable device of any of claims 1-4, wherein the inner non-degradable portion is loaded with cells, drugs, *i.e.,* small molecule therapeutics or biological therapeutics including peptides or antibodies, or crystals, optionally
wherein:
a) the cells comprise cells from xenotissue, cells from a cadaver, stem cells, cells derived from stem cells, cells from a cell line, primary cells, reprogrammed cells, reprogrammed stem cells, cells derived from reprogrammed stem cells, genetically engineered cells, or combinations thereof;
b) the cells are human cells;
c) the cells are insulin-producing cells; or
d) the cells are pancreatic islet cells.

6. The implantable device of any of claims 1-5, wherein:
a) the degradable outer housing or inner non-degradable portion is loaded with oxygen-releasing compounds, such as CaO₂; and/or
b) the degradable outer housing or inner non-degradable portion is loaded with drugs, *i.e.,* small molecule therapeutics or biological therapeutics including peptides or antibodies, crystals, with non-crystallized, crystallized, and/or encapsulated pro-angiogenic factors, such as vascular endothelial growth factor (VEGF), cells, such as pro-angiogenic cells, such as endothelial cells, mesenchymal stem cells, endothelial progenitors, proangiogenic macrophages.

7. The implantable device of any of claims 1-4, wherein the inner non-degradable portion is loaded with a biosensor, optionally
wherein the biosensor can measure blood oxygen, blood glucose, tumor antigens, cholesterol, creatinine, hemoglobin A1C, insulin, glucagon, hormone levels, levels of small molecule drugs, DNA, RNA, cytokines or circulating tumor cells.

8. The implantable device of any of claims 1-7, wherein (i) the inner non-degradable portion is patterned to permit vascularization once implanted, optionally
wherein the patterned portion comprises blood vessel geometries, such as a plurality of branched vessels ranging in size from tens to hundreds of micrometers in diameter and having substantially smooth inner walls to minimize frictional drag and turbulence during fluid flow; and/or
(ii) the blood vessel geometries of the degradable outer housing comprise a plurality of branched vessels ranging in size from tens to hundreds of micrometers in diameter and having substantially smooth inner walls to minimize frictional drag and turbulence during fluid flow.

9. The implantable device of any of claims 1-8, wherein the device comprises multiple layers of hydrogels.

10. The implantable device of any of claims 1-9, suitable for use in a method of monitoring a state or condition in a subject, the method comprising implanting the device into said subject, wherein said device comprises a sensor or biosensor.

11. The implantable device of any of claims 1-9, suitable for use in a method of treating a medical condition or disease in a subject, the method comprising implanting the device into said subject, wherein the device comprises a therapeutic agent.

12. The implantable device of any of claims 1-9, suitable for use in a method of creating a vascularized device in a subject, the method comprising implanting the device into said subject.

13. The implantable device of any of claims 1-9, suitable for use in a method of creating a vascularized tissue around an implant or prosthetic in a subject, the method comprising implanting the device into said subject, such as wherein said implant or prosthetic is a neural interface device.

14. The implantable device of any of claims 1-9, suitable for use in a method of creating a volumized tissue around an implant or prosthetic in a subject, the method comprising implanting the device into said subject, such as wherein said implant or prosthetic is a breast implant or tissue filler.

## Patentansprüche

1. Implantierbare Vorrichtung, umfassend ein abbaubares Außengehäuse, das ein abbaubares Polymer umfasst, und einen inneren, nicht abbaubaren Abschnitt, der ein nicht abbaubares Polymer umfasst, wobei das abbaubare Außengehäuse strukturiert ist und der strukturierte Abschnitt Blutgefäßgeometrien umfasst.

2. Implantierbare Vorrichtung nach Anspruch 1, wobei der nicht abbaubare Abschnitt ein natürliches oder synthetisches Hydrogel umfasst.

3. Implantierbare Vorrichtung nach Anspruch 2:
a) wobei das synthetische Hydrogel Poly(ethylenglykol)diacrylat (PEDGA) ist;
b) wobei die implantierbare Vorrichtung außerdem Gelatinemethacrylat (GelMA) umfasst; und/oder
c) wobei das natürliche oder synthetische Hydrogel mehr als 80 % Wasser umfasst; wobei
das natürliche oder synthetische Hydrogel gegebenenfalls etwa 10 % GelMA und etwa 3-4 % PEDGA mit einem Molekulargewicht von etwa 3,4 kDa umfasst.

4. Implantierbare Vorrichtung nach Anspruch 2, wobei:
a) das natürliche oder synthetisches Hydrogel Monomere aus Acrylat-(PET-Protein)ₙ-PEG-Acrylat umfasst, wobei PEG gegebenenfalls 2-35 kDa aufweist, das Protein mehr als 100 Da aufweist und das Gesamt-MG zumindest 500 kDa oder n = 2-500 Grundeinheiten ist; oder
b) das natürliche oder synthetische Hydrogel Acrylamid, Vinylsulfon, Norbornen ersetzt die Acrylatgruppen, Alginat, Seide, Dextran, Chondroitinsulfat, Hyaluronsäure, Cellulose, Heparin oder Poly(caprolacton) umfasst.

5. Implantierbare Vorrichtung nach einem der Ansprüche 1-4, wobei der innere, nicht abbaubare Abschnitt mit Zellen, Arzneimitteln, d.h. kleinmolekularen Therapeutika oder biologischen Therapeutika, einschließlich Peptiden oder Antikörpern, oder Kristallen beladen ist
wobei gegebenenfalls:
a) die Zellen Zellen aus Xenogewebe, Zellen von einer Leiche, Stammzellen, von Stammzellen abgeleitete Zellen, Zellen von einer Zelllinie, Primärzellen, reprogrammierte Zellen, reprogrammierte Stammzellen, von reprogrammierten Stammzellen abgeleitete Zellen, gentechnisch veränderte Zellen oder Kombinationen davon umfassen;
b) die Zellen menschliche Zellen sind;
c) die Zellen Insulin produzierende Zellen sind; oder
d) die Zellen Langerhans-Insel-Zellen sind.

6. Implantierbare Vorrichtung nach einem der Ansprüche 1-5, wobei:
a) das abbaubare Außengehäuse oder der innere, nicht abbaubare Abschnitt mit Sauerstoff freisetzenden Verbindungen, wie z. B. CaO₂, beladen ist; und/oder
b) das abbaubare Außengehäuse oder der innere, nicht abbaubare Abschnitt mit Arzneimitteln, d**.** h. kleinmolekularen Therapeutika oder biologischen Therapeutika, einschließlich Peptiden oder Antibiotika, Kristallen, mit nicht kristallisierten, kristallisierten und/oder verkapselten proangiogenen Faktoren, wie z. B. dem Gefäßendothelwachstumsfaktor (VEGF), Zellen, wie etwa proangiogenen Zellen, z. B. Endothelzellen, mesenchymalen Stammzellen, Endothelvorläufern, proangiogenen Makrophagen beladen sind.

7. Implantierbare Vorrichtung nach einem der Ansprüche 1-4, wobei der innere, nicht abbaubare Anteil mit einem Biosensor beladen ist
wobei der Biosensor gegebenenfalls Blutsauerstoff, Blutglucose, Tumorantigene, Cholesterin, Kreatinin, Hämoglobin A1C, Insulin, Glucagon, Hormonspiegel, Spiegel an kleinmolekularen Arzneimitteln, DNA, RNA, Cytokine oder zirkulierende Tumorzellen messen kann.

8. Implantierbare Vorrichtung nach einem der Ansprüche 1-7, wobei (i) der innere, nicht abbaubare Abschnitt strukturiert ist, um nach der Implantation eine Vaskularisation zu ermöglichen
wobei der strukturierte Abschnitt gegebenenfalls Blutgefäßgeometrien umfasst, wie z. B. eine Vielzahl an verzweigten Gefäßen, deren Größe von einigen Dutzend bis einigen Hundert Mikrometern im Durchmesser reicht und die eine Wesentlichen glatte Innenwände aufweisen, um den Reibungswiderstand und Turbulenzen in der Fluidströmung zu minimieren; und/oder
(ii) die Blutgefäßgeometrien des abbaubaren Außengehäuses eine Vielzahl von verzweigten Gefäßen umfassen, deren Größe von einigen Dutzend bis einigen Hundert Mikrometern im Durchmesser reicht und die im Wesentlichen glatte Innenwände aufweisen, um den Reibungswiderstand und Turbulenzen in der Fluidströmung zu minimieren.

9. Implantierbare Vorrichtung nach einem der Ansprüche 1-8, wobei die Vorrichtung mehreren Schichten aus Hydrogelen umfasst.

10. Implantierbare Vorrichtung nach einem der Ansprüche 1-9, die zur Verwendung in einem Verfahren zur Überwachung eines Zustands oder eines Leidens bei einem Individuum geeignet ist, wobei das Verfahren das Implantieren der Vorrichtung in das Individuum umfasst, wobei die Vorrichtung einen Sensor oder Biosensor umfasst.

11. Implantierbare Vorrichtung nach einem der Ansprüche 1- bis 9, die zur Verwendung in einem Verfahren zur Behandlung eines medizinischen Leidens oder einer Erkrankung bei einem Individuum geeignet ist, wobei das Verfahren das Implantieren der Vorrichtung in das Individuum umfasst, wobei die Vorrichtung ein Therapeutikum umfasst.

12. Implantierbare Vorrichtung nach einem der Ansprüche 1 bis 9, die zur Verwendung in einem Verfahren zur Schaffung einer vaskularisierten Vorrichtung in einem Individuum geeignet ist, wobei das Verfahren das Implantieren der Vorrichtung in das Individuum umfasst.

13. Implantierbare Vorrichtung nach einem der Ansprüche 1 bis 9, die zur Verwendung in einem Verfahren zur Schaffung eines vaskularisierten Gewebes um ein Implantat oder eine Prothese in einem Individuum geeignet ist, wobei das Verfahren das Implantieren der Vorrichtung in das Individuum umfasst, wobei das Implantat oder die Prothese beispielsweise eine neuronale Schnittstellenvorrichtung ist.

14. Implantierbare Vorrichtung nach einem der Ansprüche 1-9, die zur Verwendung in einem Verfahren zur Schaffung eines voluminisierten Gewebes um ein Implantat oder eine Prothese in einem Individuum geeignet ist, wobei das Verfahren das Implantieren in das Individuum umfasst, wobei das Implantat oder die Prothese beispielsweise ein Brustimplantat oder ein Gewebefüller ist.

## Revendications

1. Dispositif implantable comprenant un boîtier externe dégradable comprenant un polymère dégradable et une partie interne non dégradable comprenant un polymère non dégradable, dans lequel le boîtier externe dégradable présente des motifs et la partie à motifs comprend des géométries de vaisseau sanguin.

2. Dispositif implantable selon la revendication 1, dans lequel la partie non dégradable comprend un hydrogel naturel ou synthétique.

3. Dispositif implantable selon la revendication 2 :
a) dans lequel l'hydrogel synthétique est du diacrylate de poly(éthylène glycol) (PEDGA) ;
b) dans lequel le dispositif implantable comprend en outre du méthacrylate de gélatine (GelMA) ; et/ou
c) dans lequel l'hydrogel naturel ou synthétique comprend plus de 80 % d'eau ; éventuellement
dans lequel l'hydrogel naturel ou synthétique comprend environ 10 % de GelMA et environ 3-4 % de PEDGA présentant un poids moléculaire d'environ 3,4 kDa.

4. Dispositif implantable selon la revendication 2, dans lequel :
a) ledit hydrogel naturel ou synthétique comprend des monomères d'acrylate-(PEG-protéine)ₙ-PEG-acrylate, éventuellement dans lequel le PEG est de 2-35 kDa, la protéine est supérieure à 100 Da et le MW total est d'au moins 500 kDa ou n = 2-500 motifs de répétitions ; ou
b) l'hydrogel naturel ou synthétique comprend de l'acrylamide, de la vinyl-sulfone et du norbornène qui remplacent les groupes acrylate, l'alginate, la soie, le dextrane, le sulfate de chondroïtine, l'acide hyaluronique, la cellulose, l'héparine ou la poly(caprolactone).

5. Dispositif implantable selon l'une quelconque des revendications 1 à 4, dans lequel la partie interne non dégradable est chargée de cellules, de médicaments, c'est-à-dire, des agents thérapeutiques à petites molécules ou des agents thérapeutiques biologiques comportant des peptides ou des anticorps, ou des cristaux, éventuellement
dans lequel :
a) les cellules comprennent des cellules provenant de xénotissus, des cellules provenant d'un cadavre, des cellules souches, des cellules dérivées de cellules souches, des cellules dérivées d'une lignée cellulaire, des cellules primaires, des cellules reprogrammées, des cellules souches reprogrammées, des cellules dérivées de cellules souches reprogrammées, des cellules génétiquement modifiées, ou des combinaisons de celles-ci ;
b) les cellules sont des cellules humaines ;
c) les cellules sont des cellules productrices d'insuline ; ou
d) les cellules sont des cellules d'îlots pancréatiques.

6. Dispositif implantable selon l'une quelconque des revendications 1 à 5, dans lequel :
a) le boîtier externe dégradable ou la partie interne non dégradable est chargé(e) de composés de libération d'oxygène, tels que du CaO₂ ; et/ou
b) le boîtier externe dégradable ou la partie interne non dégradable est chargé(e) de médicaments, c'est-à-dire, des agents thérapeutiques à petites cellules ou des agents thérapeutiques biologiques comportant des peptides ou des anticorps, des cristaux, avec des facteurs proangiogéniques non cristallisés, cristallisés et/ou encapsulés, tels que le facteur de croissance de l'endothélium vasculaire (VEGF), des cellules, telles que des cellules proangiogéniques, telles que des cellules endothéliales, des cellules souches mésenchymateuses, des progéniteurs endothéliaux, des macrophages proangiogéniques.

7. Dispositif implantable selon l'une quelconque des revendications 1 à 4, dans lequel la partie interne non dégradable est chargée d'un biocapteur, éventuellement dans lequel le biocapteur peut mesurer l'oxygène sanguin, la glycémie, les antigènes tumoraux, le cholestérol, la créatinine, l'hémoglobine A1C, l'insuline, le glucagon, les niveaux d'hormones, les niveaux de médicaments à petites cellules, l'ADN, l'ARN, les cytokines ou des cellules tumorales circulantes.

8. Dispositif implantable selon l'une quelconque des revendications 1 à 7, dans lequel (i) la partie interne non dégradable présente des motifs pour permettre une vascularisation une fois implantée, éventuellement
dans lequel la partie à motifs comprend des géométries de vaisseau sanguin, telles qu'une pluralité de vaisseaux ramifiés dont la taille varie de dizaines à des centaines de micromètres de diamètre et présentant des parois internes sensiblement lisses pour réduire au minimum la résistance au frottement et la turbulence pendant un écoulement de fluide ; et/ou
(ii) les géométries de vaisseau sanguin du boîtier externe dégradable comprennent une pluralité de vaisseaux ramifiés dont la taille varie de dizaines à des centaines de micromètres de diamètre et présentant des parois internes sensiblement lisses pour réduire au minimum la résistance au frottement et la turbulence pendant un écoulement de fluide.

9. Dispositif implantable selon l'une quelconque des revendications 1 à 8, dans lequel le dispositif comprend de multiples couches d'hydrogels.

10. Dispositif implantable selon l'une quelconque des revendications 1 à 9, approprié pour une utilisation dans un procédé de surveillance d'un état ou d'une affection d'un sujet, le procédé comprenant l'implantation du dispositif dans ledit sujet, dans lequel ledit dispositif comprend un capteur ou un biocapteur.

11. Dispositif implantable selon l'une quelconque des revendications 1 à 9, approprié pour une utilisation dans un procédé de traitement d'une affection médicale ou d'une maladie chez un sujet, le procédé comprenant l'implantation du dispositif dans ledit sujet, dans lequel le dispositif comprend un agent thérapeutique.

12. Dispositif implantable selon l'une quelconque des revendications 1 à 9, approprié pour une utilisation dans un procédé de création d'un dispositif vascularisé chez un sujet, le procédé comprenant l'implantation du dispositif dans ledit sujet.

13. Dispositif implantable selon l'une quelconque des revendications 1 à 9, approprié pour une utilisation dans un procédé de création d'un tissu vascularisé autour d'un implant ou d'une prothèse chez un sujet, le procédé comprenant l'implantation du dispositif dans ledit sujet, par exemple dans lequel ledit implant ou ladite prothèse est un dispositif d'interface neuronale.

14. Dispositif implantable selon l'une quelconque des revendications 1 à 9, approprié pour une utilisation dans un procédé de création d'un tissu volumisé autour d'un implant ou d'une prothèse chez un sujet, le procédé comprenant l'implantation du dispositif dans ledit sujet, par exemple dans lequel ledit implant ou ladite prothèse est un implant mammaire ou un agent de remplissage tissulaire.
